# EUROPEAN PATENT APPLICATION

(11) **EP 1 435 353 A1**
(43) Date of publication of application: **07.07.2004**
(21) Application number: 02800750.8
(22) Date of filing: 02.10.2002
(51) Int. Cl.: C07D 233/30, C07D 263/16, C07D 263/24, C07D 277/14, C07D 413/06, C07D 413/12, C07D 417/06, A61K 31/4166, A61K 31/421, A61K 31/422, A61K 31/426, A61K 31/427, A61K 31/4439, A61P 1/00

(54) **NOVEL HETEROCYCLIC COMPOUND AND ANTI-INFLAMMATORY AGENT**

(30) Priority: 03.10.2001 JP 2001307301
(71) Applicant: NIPPON SODA CO., LTD., Chiyoda-ku, Tokyo 100-8165 (JP)
(72) Inventor: TAKAGI, M.; c/o Odawara Res. Center, Odawara-shi, Kanagawa 250-0280 (JP); ISHIMITSU, Keiichi; c/o Odawara Res. Center, Odawara-shi, Kanagawa 250-0280 (JP); NISHIBE, Tadayuki; c/o Odawara Res. Center, Odawara-shi, Kanagawa 250-0280 (JP)
(74) Representative: Wibbelmann, Jobst, Dr., Dipl.-Chem.
(86) International application number: PCT/JP2002/010291
(87) International publication number: WO 2003/031414

(57) **Abstract**

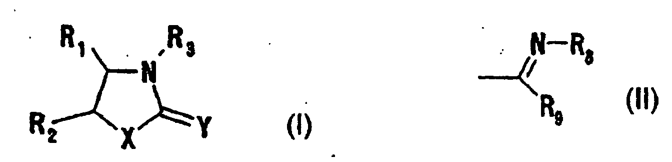

A compound represented by the formula [I]: [I] (wherein X represents oxygen, sulfur, NR₄, or CHR₅; Y represents oxygen or sulfur; R₁ represents C₁₋₆ alkyl, etc.; R₂ represents optionally substituted phenyl, etc.; and R₃ represents optionally substituted C₁₋₆ alkyl, a group represented by any of the following formulae, etc.) [II] or a pharmaceutically acceptable composite of the compound; a medicinal composition containing any of these as an active ingredient; and a use thereof, etc.

## Description

### Field of Invention:

The present invention relates to novel compounds and medicinal compositions containing them as active ingredients. In more detail, it relates to drugs containing, as active ingredients, compounds with inhibitory activities on phospholipase A (2) [hereinafter referred to as PLA (2)] or pharmaceutically acceptable composites thereof.

### Background Art:

Inflammation is a series of defensive response processes caused in tissues, induced by injurious events (inflammatory stimuli) applied on any part of a human body. When tissues are damaged by inflammatory stimuli that are caused by bacterial infections, immunological responses or physiochemical injuries, they respond to the stimuli (acute inflammation) and remove them so as to repair the damages. Alternatively, if it is difficult to remove the stimuli, the damages progress to induce continuous proliferate tissue swelling (chronic inflammation). These inflammations are well known to be associated with a large number of diseases. Various types of mediators participate in the activations and interactions of various cells involved in each of the steps.

PLA (2) is the general term for enzymes that preferentially catalyze the hydrolysis of the fatty acid bound at the *sn-2* position of the glycerol skeleton of a glycerophospholipid, which is a major component of cell membrane. It is also known that these enzymes are involved in the neogenesis metabolisms of biomembrane lipids and that the hydrolysates and their metabolites are lipid mediators with strong, diverse physiological activities. One of the products, arachidonic acid, itself works as a mediator, and is further metabolized in respective inflammation-associated cells to prostaglandins, thromboxanes, lipoxins, leukotrienes and others, to induce characteristic physiological responses [Irvine, R., *Biochemical Journal* **204**: 3-16 (1982)]. The other product, lysophosphatidylcholine, not only plays a role as a mediator but also is utilized as a precursor of the platelet activating factor (hereinafter referred to as PAF). These lipid mediators play an essential role to maintain the homeostasis of living organisms. They are however produced excessively in the conditions associated with inflammation and involved in the exacerbation of symptoms of the diseases. In fact, steroidal anti-inflammatory drugs and various non-steroidal anti-inflammatory drugs (hereinafter referred to as NSAIDs) have been widely used in clinical therapies as drugs to interfere with the arachidonic acid cascade. PLA (2) positions at the upstream of the arachidonic acid cascade and is enzymes involved in the rate-determining step of the production of these lipid mediators. Thus PLA (2) has been expected to be a promising target for the development of anti-inflammatory drugs [Glaser, K.B., *Advances in Pharmacology* **32**: 31-66 (1995)]

Recently, new isozymes of PLA (2) have been identified one after another, being more than 15. These consist of a big superfamily as a whole that is subdivided into four families on the basis of the protein structures and the characteristics in the enzyme activities [Dennis, E.A., *Trends in Biochemical Science* **22**: 1-2 (1997); Balsinde, J., et al., *Annual Review of Pharmacology and Toxicology* **39**: 175-189 (1999) and others]. Among them, only specific isozymes have high specificity against phospholipids with arachidonic acid bound at the *sn-2* position of the glycerol structure, or are recognized to have enhanced activities in inflammatory diseases Examples of such inflammation-specific PLA (2) include group IV-cytosolic PLA (2) [hereinafter referred to as IV-cPLA (2); molecular weight 85 kDa] and subtypes IIA, IID, V and X of secretory PLA (2) [hereinafter referred to as sPLA (2), molecular weight 14kDa]. Among them, IV-cPLA (2) is considered to be a major isozyme responsible for controlling the production of the lipid mediators in the inflammatory diseases. This is supported by findings from tests with knockout mice [Uozumi, N., et al., *Nature* **390**: 619-622 (1997); Bonventre, J.V., et al., *Nature* **390**: 622-625 (1997); and Nagase, T., et al., *Nature Immunology* **1**: 42-46 (2000)]. Therefore, inhibition of the activity of the isozyme can suppress the increased production of the lipid mediators due to the disease, which may lead to treatment and/or prevention of inflammatory diseases. Examples of such diseases include anaphylaxis, septic shock, fever and pain that are induced by various inflammatory stimuli; respiratory diseases such as bronchitis, pneumonia and adult respiratory distress syndrome; digestive diseases such as inflammatory intestine disorder, Crohn's disease, ulcerative colitis, hepatitis and nephritis; cardiovascular diseases such as vasculitis and arteriosclerosis; allergic inflammatory diseases such as rhinitis, asthma and atopic syndromes; autoimmune diseases such as rheumatism; and ischemic or ischemic-reperfusion injuries such as cerebral infarction and myocardial infarction; as well as nerve degenerative diseases, solar keratosis and psoriasis.

However, no compounds have been developed that have effects useful in clinical therapies by inhibiting the enzyme activity. It is therefore desired to develop new drugs that specifically and comprehensively control the production of lipid mediators in inflammatory diseases, with excellent therapeutic and/or preventive effects.

The compounds represented by the following formula are disclosed in World Open WO 97/05135 as oxa(thia)zolidine compounds having an activity of inhibiting the PLA (2) activity.

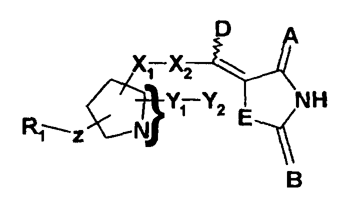

Furthermore, the compounds represented by the following formula are disclosed to have pharmacological activities, such as anti-inflammatory activity, by leukotnene D₄ antagonistic effects in Musser, J H., et al., *Journal of Medicinal Chemistry* **30**: 2087-2093 (1987) and GB 2183641

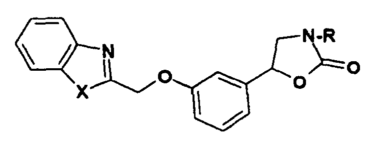

Oxa(thia)zolidine derivatives similar to the compounds of the present invention are disclosed to have insecticidal and acaricidal activities in USP 4,442,116, USP 4,431,814 and others. Compounds having glutamic-acid blocking activities are disclosed in EP 250241 and those with bactericidal activities in Japanese Patent Laid-open No. Hei 8-291155.

Oxazolidine derivatives are disclosed as starting materials or intermediates for stereoselective syntheses in *Tetrahedron Letters* 1997, 38 (51), 8807-8810; *J. Amer. Chem. Soc.* 2001, 123 (3), 398-408 and others.

As for imidazolidine derivatives similar to the compounds of the present invention, World Open WO 2000/37473 discloses the compounds represented by the following formulae as intermediates for producing drugs (Compounds 97 and 98).

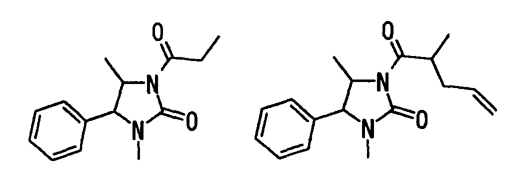

Compounds represented by the formula shown below are descnbed in WO 96/03386.

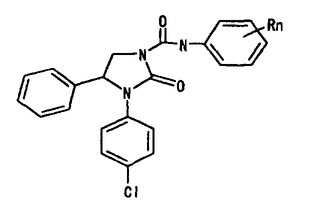

In addition, *Angew. Chem*. 1991, 103 (1), 76-8; *Tetrahedron* 1993, 49 (35), 7787-92 and others describe 3-phenylsulfonylimidazolidinones.

As for pyrrolidine derivatives similar to the compounds of the present invention, N-acyl-4-phenylpyrrolidin-2-one compounds are disclosed in EP 439766.

In *Tetrahedron Asymmetry* 1999, 10 (23), 4553-4561, N-phenylsulfonyl-2-methyl-3-benzyl-2-one compounds are described

However, these compounds are not known to have PLA (2) inhibitory or anti-inflammatory activities.

### Disclosure of the Invention:

As described above, it is understood that the enhanced PLA (2) activity plays an important role in the progresses of various inflammatory diseases. It is therefore an object of the present invention to provide novel drugs that are effective to ease symptoms of inflammatory diseases and to treat or prevent relevant diseases.

The inventors of the present invention studied in earnest to achieve the above object. As a result, they have found heterocyclic compounds represented by Formula [I] to be inhibitors of the PLA (2) activity.

The present invention consists of the following (1) through (6).
(1) A compound represented by Formula [I] 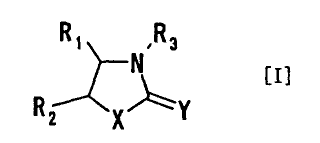 [wherein, X represents oxygen, sulfur, NR₄ or CHR₅;
   Y represents oxygen or sulfur,
   R₁ represents C₁₋₆ alkyl or C₁₋₆ haloalkyl;
   R₂ represents phenyl, naphthyl, indanyl, tetrahydronaphthyl, thienyl, phenyl C₁₋₆ alkyl, phenyl C₂₋₆ alkenyl, thienyl C₂₋₆ alkenyl or naphthyl C₂₋₆ alkenyl, and the rings of these groups are optionally substituted with one or more, same or different, groups represented by A₁;
   R₃ represents C₁₋₆ alkyl optionally substituted with A₂, C₂₋₆ alkenyl optionally substituted with A₂ or one of the groups represented by the following formulae

   CZR₆, S(O)mR₇,

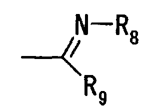

   R₄ represents hydrogen, C₁₋₆ alkyl, C₃₋₇ cycloalkyl, C₁₋₆ alkylcarbonyl, C₁₋₆ haloalkylcarbonyl, C₁₋₆ alkenylcarbonyl, C₁₋₆ alkoxycarbonyl, C₁₋₆ haloalkoxycarbonyl, or mono- or di-C₁₋₆ alkylcarbamoyl;
   R₅ represents hydrogen or C₁₋₆ alkoxycarbonyl;
   R₆ represents C₁₋₆ alkyl optionally substituted with A₂, C₁₋₆ alkoxy optionally substituted with A₂, C₂₋₆ alkenyl optionally substituted with A₂, C₃₋₇ cycloalkyl optionally substituted with A₃, C₃₋₇ cycloalkoxy optionally substituted with A₃, C₃₋₇ cycloalkenyl optionally substituted with A₃, C₃₋₇ cycloalkenyloxy optionally substituted with A₃, phenyl optionally substituted with A₄, a saturated or unsaturated 5- or 6-membered heterocyclic group having at least a hetero atom selected from oxygen, sulfur and nitrogen and optionally substituted with A₃, or a group represented by Formula NR₁₀R₁₁;
   R₇ represents C₁₋₆ alkyl optionally substituted with A₂, C₂₋₆ alkenyl optionally substituted with A₂, C₃₋₇ cycloalkyl optionally substituted with A₃, a saturated or unsaturated 5- or 6-membered heterocyclic group having at least a hetero atom selected from oxygen, sulfur and nitrogen and optionally substituted with A₃, or a group represented by Formula NR₁₂R₁₃;
   R₈ represents cyano, nitro, C₁₋₆ alkyl or C₃₋₇ cycloalkyl,
   R₉ represents C₁₋₆ alkyl, C₁₋₆ alkylthio, C₁₋₆ alkylamino, C₃₋₇ cycloalkylamino or morpholino;
   R₁₀ and R₁₂ represent hydrogen or C₁₋₆ alkyl;
   R₁₁ and R₁₃ represent C₁₋₆ alkyl optionally substituted with A₂, C₂₋₆ alkenyl optionally substituted with A₂, C₂₋₆ alkynyl optionally substituted with A₂, C₁₋₆ alkoxy optionally substituted with A₂, C₂₋₆ alkenyloxy optionally substituted with A₂, C₂₋₆ alkynyloxy optionally substituted with A₂, C₁₋₆ alkylcarbonyl, phenylcarbonyl optionally substituted with A₄, C₁₋₆ alkylsulfonyl, phenylsulfonyl optionally substituted with A₄, mono- or di-C₁₋₆ alkylamino optionally substituted with A₂, C₃₋₇ cycloalkyl optionally substituted with A₃, C₅₋₇ cycloalkenyl optionally substituted with A₃, phenyl optionally substituted with A₄, phenoxy optionally substituted with A₄, anilino optionally substituted with A₄, or a saturated or unsaturated 5- or 6-membered heterocyclic group having at least a hetero atom selected from oxygen, sulfur and nitrogen and optionally substituted with A₃;
   Z represents oxygen or sulfur, m is 0, 1 or 2,
   A₁ represents halogen, nitro, cyano, C₁₋₆ alkyl, C₂₋₆ alkenyl, C₁₋₆ haloalkyl, C₃₋₇ cycloalkyl, phenyl optionally substituted with halogen, C₁₋₆ alkyl or C₁₋₆ haloalkyl; pyridyl, thienyl, C₁₋₆ alkoxy, methylenedioxy, C₁₋₆ alkylthio, C₁₋₆ alkylsulfenyl, C₁₋₆ alkylsulfonyl, C₁₋₆ haloalkoxy, optionally substituted benzyl, optionally substituted phenethyl, optionally substituted phenoxy, optionally substituted phenylthio, optionally substituted benzoyl, C₁₋₆ alkylcarbonyl, C₁₋₆ alkoxycarbonyl, C₁₋₆ haloalkylcarbonyl, C₁₋₆ haloalkoxycarbonyl or C₁₋₆ alkylcarbonyloxy;
   A₂ represents halogen, cyano, phenyl optionally substituted with A₄, a saturated or unsaturated 5- or 6-membered heterocyclic group having at least a hetero atom selected from oxygen, sulfur and nitrogen and optionally substituted with A₃, amino substituted with one or two groups of 'C₁₋₆ alkyl, C₃₋₇ cycloalkyl or C₁₋₆ alkylcarbonyl', C₁₋₆ alkylcarbonyl, C₁₋₆ alkoxycarbonyl, or one of the groups represented by the following formulae

   OR₁₄, S(O)mR₁₅

   (wherein, R₁₄ represents hydrogen, C₁₋₆ alkyl, C₂₋₆ alkenyl, C₂₋₆ alkynyl, C₁₋₆ haloalkyl, C₁₋₆ alkylcarbonyl, mono- or di-C₁₋₆ alkylcarbamoyl, C₃₋₇ cycloalkyl, phenyl optionally substituted with A₄, or a saturated or unsaturated 5- or 6-membered heterocyclic group having at least a hetero atom selected from oxygen, sulfur and nitrogen and optionally substituted with A₃; and
   R₁₅ represents as defined for R₇);
   A₃ represents halogen, hydroxyl, oxo, C₁₋₆ alkyl, C₁₋₆ alkoxy, C₁₋₆ alkoxy C₁₋₆ alkoxy, amino, mono- or di-C₁₋₆ alkylamino, C₁₋₆ alkylcarbonyloxy, C₁₋₆ alkoxycarbonyl mono- or di-C₁₋₆ alkylcarbamoyl, or mono- or di-C₁₋₆ alkylcarbonylamino;
   A₄ represents halogen, nitro, cyano, hydroxyl, C₁₋₆ alkyl, C₁₋₆ haloalkyl, C₁₋₆ alkoxy, C₁₋₆ alkylthio, C₁₋₆ alkylsulfenyl, C₁₋₆ alkylsulfonyl, C₁₋₆ alkoxy C₁₋₆ alkoxy, C₁₋₆ haloalkoxy, C₁₋₆ alkylcarbonyl, C₁₋₆ alkoxycarbonyl, or C₁₋₆ alkylcarbonyloxy;
   If a group is substituted with two or more substituents, such as A₁, A₂, A₃ and A₄, the substituents may be the same or different from each other;
   R₃ represents not a group represented by Formula CZNR₁₀R₁₁, when X is oxygen or sulfur and R₂ is phenyl optionally substituted with A₁, naphthyl optionally substituted with A₁, indanyl optionally substituted with A₁, tetrahydronaphthyl optionally substituted with A₁, or thienyl optionally substituted with A₁; and
   R₃ represents not alkylcarbonyl or alkenylcarbonyl, when X is NR₄] and a pharmaceutically acceptable composite thereof.
(2) A medicinal composition characterized by containing at least a component selected from the group consisting of the heterocyclic compounds of Formula [I] or pharmaceutically acceptable composites thereof as an active ingredient, and in particular the said composition is an inhibitor of the phospholipase A (2) activity.
(3) A use of a medicinal composition characterized by containing at least a component selected from the group consisting of the heterocyclic compounds of Formula [I] or pharmaceutically acceptable composites thereof as an active ingredient, for a mammal that needs a treatment of inflammatory disease or disorder.
(4) A method for treating or relieving an inflammatory disease or disorder by means of controlling and/or preventing the progress of the symptoms of the disease so as to, in which an effective dose of a composition containing at least a component selected from the group consisting of the heterocyclic compounds of Formula [I] or pharmaceutically acceptable composites thereof is administered to a mammal that needs a treatment of an inflammatory disease or disorder. In particular, a method according to the above, in which the inflammatory disease or disorder is anaphylaxis, allergic inflammation, asthma, rhinitis, bronchitis, pneumonia, adult respiratory distress syndrome, inflammatory intestine disorder, Crohn's disease, ulcerative colitis, ischemia-reperfusion injuries, vasculitis, arteriosclerosis, hepatitis, nephritis, nerve degenerative diseases, arthritis, dermatitis, solar keratosis, psoriasis, septic shock or fever, a method according to the above, in which the symptoms of the inflammatory disease or disorder progress with the enhanced phospholipase A (2) activity; a method according to the above, in which the inflammatory disease or disorder is mediated by arachidonic acid and its metabolites, lysophosphatidylcholine and/or the platelet activating factor (PAF), which are inflammatory lipid mediators; and a method according to the above, in which the inflammatory lipid mediators are suppressed by inhibitors of phospholipase A (2) activity.
(5) A use of the heterocyclic compounds of Formula [I] for producing drugs to use for easing inflammatory and allergic conditions and conditions associated with immunity, and/or treating such diseases.
(6) A use, as a medicine, of a composition containing at least a component selected from the group consisting of the heterocyclic compounds of Formula [I] or pharmaceutically acceptable composites thereof as an active ingredient; and particularly, a use of the composition as an anti-inflammatory agent, anti-allergic agent and/or immune controlling agent.

The compounds of the present invention, represented by Formula [I], consist of compounds represented by Formulae [I-1] to [I-4] shown below:

### Compounds represented by Formula [I-1]

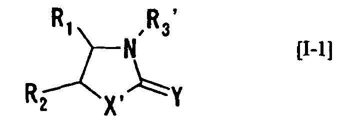

(wherein, X' represents oxygen or sulfur;
R₁, R₂ and Y represent as defined above;
R₃' represents C₁₋₆ alkyl substituted with A₂, C₂₋₆ alkenyl substituted with A₂, or one of the groups represented by the following formulae

CZR₆', S(O)mR₇,

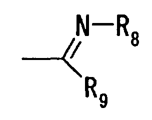

R₆' represents C₁₋₆ alkyl optionally substituted with A₂, C₁₋₆ alkoxy optionally substituted with A₂, C₂₋₆ alkenyl optionally substituted with A₂, C₃₋₇ cycloalkyl optionally substituted with A₃, C₃₋₇ cycloalkoxy optionally substituted with A₃, C₃₋₇ cycloalkenyl optionally substituted with A₃, C₃₋₇ cycloalkenyloxy optionally substituted with A₃, phenyl optionally substituted with A₄, or a saturated or unsaturated 5- or 6-membered heterocyclic group having at least a hetero atom selected from oxygen, sulfur and nitrogen and optionally substituted with A₃; and R₇, R₈ and R₉ are as defined above], or pharmaceutically acceptable composites thereof.

### Compounds represented by Formula [I-2]

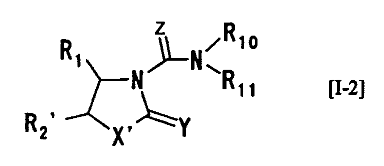

(wherein, X' represents oxygen or sulfur,
R₁, Y, Z, R₁₀ and R₁₁ represent as defmed above; and
R₂' represents phenyl C₁₋₆ alkyl optionally substituted with A₁, phenyl C₂₋₆ alkenyl optionally substituted with A₁, thienyl C₂₋₆ alkenyl optionally substituted with A₁ or naphthyl C₂₋₆ alkenyl optionally substituted with A₁) and pharmaceutically acceptable composites thereof.

### Compounds represented by Formula [I-3]

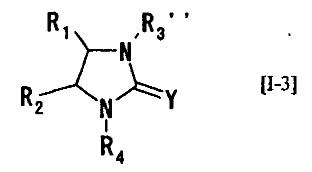

(wherein, R₁, R₂, R₄ and Y are as defined above,
R₃'' represents C₁₋₆ alkyl optionally substituted with A₂, C₂₋₆ alkenyl optionally substituted with A₂, or one of the groups represented by the following formulae

CZR₆'', S(O)mR₇,

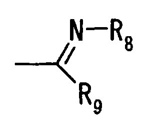

R₆" represents C₁₋₆ alkyl substituted with A₂, C₁₋₆ alkoxy optionally substituted with A₂, C₂₋₆ alkenyl substituted with A₂, C₃₋₇ cycloalkyl optionally substituted with A₃, C₃₋₇ cycloalkoxy optionally substituted with A₃, C₃₋₇ cycloalkenyl optionally substituted with A₃, C₃₋₇ cycloalkenyloxy optionally substituted with A₃, phenyl optionally substituted with A₄, or a saturated or unsaturated 5- or 6-membered heterocyclic group having at least a hetero atom selected from oxygen, sulfur and nitrogen and optionally substituted with A₃; or a group represented by Formula NR₁₀R₁₁; and
R₇, R₈, R₉, R₁₀, R₁₁, A₂, A₃ and A₄ are as defined above), or pharmaceutically acceptable composites thereof.

### Compounds represented by Formula [1-4]

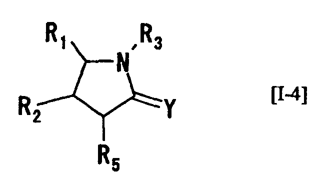

(wherein, R₁, R₂, R₃, R₅ and Y are as defined above) and pharmaceutically acceptable composites thereof.

### Forms to Implement the Invention:

The compounds of the present invention, represented by Formula [I] are described in more detail.

In the above definitions, R₁ represents C₁₋₆ alkyl such as methyl, ethyl, propyl, isopropyl, butyl, isobutyl, s-butyl or t-butyl; or C₁₋₆ haloalkyl such as chloromethyl, fluoromethyl, bromomethyl, 2-chloroethyl, 2-fluoroethyl, 1,2-dichloroethyl, 1,2-difluoroethyl or trifluoromethyl.

R₂ represents phenyl; naphthyl; indanyl; tetrahydronaphthyl; thienyl; phenyl C₁₋₆ alkyl such as benzyl, phenethyl, α-methylbenzyl or phenylpentyl; phenyl C₂₋₆ alkenyl such as styryl, 3-phenyl-1-propenyl or 3-phenyl-2-buten-2-yl; thienylvinyl or naphthylvinyl. The rings of these groups are optionally substituted with one or more groups represented by A, shown below. When they are substituted with two or more groups, the substituents may be the same or different from each other.

Substituent A₁ represents halogen such as fluorine, chlorine or bromine; nitro; cyano; C₁₋₆ alkyl such as methyl, ethyl, propyl, isopropyl, butyl or t-butyl; C₂₋₆ alkenyl such as vinyl, 1-propenyl or allyl; C₁₋₆ haloalkyl such as chloromethyl, chlroroethyl or trifluoromethyl; C₃₋₇ cycloalkyl such as cyclopropyl, cyclopentyl or cyclohexyl, phenyl optionally substituted with fluorine, chlorine, bromine, methyl, ethyl or trifluoromethyl; pyridyl; thienyl; C₁₋₆ alkoxy such as methoxy, ethoxy or propoxy; methylenedioxy; C₁₋₆ alkylthio such as methylthio; C₁₋₆ alkylsulfenyl such as methylsulfenyl; C₁₋₆ alkylsulfonyl such as methanesulfonyl; C₁₋₆ haloalkoxy such as chloromethoxy, trifluoromethoxy or 2-chloroethoxy; optionally substituted benzyl; optionally substituted phenethyl; optionally substituted phenoxy; optionally substituted phenylthio; optionally substituted benzoyl; C₁₋₆ alkylcarbonyl such as acetyl or ethylcarbonyl; C₁₋₆ alkoxycarbonyl such as methoxycarbonyl or ethoxycarbonyl; C₁₋₆ haloalkylcarbonyl such as chloroacetyl or trifluoroacetyl; C₁₋₆ haloalkoxycarbonyl such as chloromethoxycarbonyl or trifluoromethoxycarbonyl; or C₁₋₆ alkylcarbonyloxy such as methoxycarbonyloxy or ethoxycarbonyloxy.

R₃ represents C₁₋₆ alkyl optionally substituted with A₂ such as methyl, ethyl, propyl, isopropyl, butyl, isobutyl, s-butyl or t-butyl; C₂₋₆ alkenyl optionally substituted with A₂ such as vinyl, 1-propenyl, isopropenyl, allyl or 2-butenyl; or one of the groups represented by the following formulae

CZR₆, S(O)mR₇,

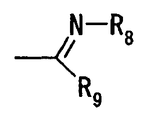

wherein, Z represents oxygen or sulfur; and m is 0, 1 or 2.

R₆ represents C₁₋₆ alkyl optionally substituted with A₂ such as methyl, ethyl, propyl, isopropyl, butyl, sec-butyl, isobutyl, t-butyl, pentyl or its isomer, or hexyl or its isomer, C₁₋₆ alkoxy optionally substituted with A₂ such as methoxy, ethoxy, propoxy, isopropoxy, butoxy, sec-butoxy, isobutoxy or t-butoxy; C₂₋₆ alkenyl optionally substituted with A₂ such as vinyl, 1-propenyl, 2-propenyl, 1-butenyl, 2-butenyl, 3-butenyl, 1-methyl-2-propenyl, 2-methyl-2-propenyl, 1-pentenyl, 2-pentenyl, 3-pentenyl, 4-pentenyl, 1-methyl-2-butenyl, 2-methyl-2-butenyl or 1-hexenyl; C₃₋₇ cycloalkyl optionally substituted with A₃ such as cyclopropyl, cyclopentyl, cyclohexyl or cycloheptyl; C₃₋₇ cycloalkoxy optionally substituted with A₃ such as cyclopropyloxy, cyclopentyloxy, cyclohexyloxy or cycloheptyloxy; C₃₋₇ cycloalkenyl optionally substituted with A₃ such as cyclopentenyl, cyclohexenyl or cycloheptenyl; C₃₋₇ cycloalkenyloxy optionally substituted with A₃ such as cyclopentenyloxy, cyclohexenyloxy or cycloheptecryloxy; phenyl optionally substituted with A₄; a saturated or unsaturated 5- or 6-membered heterocyclic group having at least a hetero atom selected from oxygen, sulfur and nitrogen and optionally substituted with A₃ such as tetrahydrofuryl, tetrahydrothienyl, pyrrolidinyl, pyrazolidinyl, imidazolidinyl, tetrahydropyranyl, tetrahydrothiapyranyl, piperidinyl, pyrimidinyl, pyridyl or morpholino; or a group represented by Formula NR₁₀R₁₁.

R₇ represents C₁₋₆ alkyl optionally substituted with A₂ such as methyl, ethyl, propyl, isopropyl, butyl, sec-butyl, isobutyl, t-butyl, pentyl or its isomer, or hexyl or its isomer, C₂₋₆ alkenyl optionally substituted with A₂ such as vinyl, 1-propenyl, allyl, 1-butenyl, 2-butenyl or 3-butenyl; C₃₋₇ cycloalkyl optionally substituted with A₃ such as cyclopropyl, cyclopentyl or cyclohexyl; a saturated or unsaturated 5- or 6-membered heterocyclic group having at least a hetero atom selected from oxygen, sulfur and nitrogen and optionally substituted with A₃ such as tetrahydrofuryl, tetrahydrothienyl, pyrrolidinyl, pyrazolidinyl, imidazolidinyl, tetrahydropyranyl, tetrahydrothiapyranyl, piperidinyl, pyrimidinyl, pyridyl or morpholino; or a group represented by Formula NR₁₂R₁₃.

R₈ represents cyano; nitro; C₁₋₆ alkyl such as methyl or ethyl; or C₃₋₇ cycloalkyl such as cyclohexyl.

R₉ represents C₁₋₆ alkyl such as methyl or ethyl; C₁₋₆ alkylthio such as methylthio or ethylthio, C₁₋₆ alkylamino such as methylamino or ethylamino; C₃₋₇ cycloalkylamino such as cyclohexylamino; or morpholino.

R₁₀ and R₁₂ represent hydrogen or C₁₋₆ alkyl such as methyl or ethyl.

R₁₁ and R₁₃ represent C₁₋₆ alkyl optionally substituted with A₂ such as methyl, ethyl, propyl, isopropyl, butyl, sec-butyl, isobutyl, t-butyl, pentyl or its isomer, or hexyl or its isomer, C₂₋₆ alkenyl optionally substituted with A₂ such as vinyl, 1-propenyl or allyl; C₂₋₆ alkynyl optionally substituted with A₂ such as ethynyl or 2-propynyl; C₁₋₆ alkoxy optionally substituted with A₂ such as methoxy or ethoxy; C₂₋₆ alkenyloxy optionally substituted with A₂ such as allyloxy or 2-propenyloxy; C₂₋₆ alkynyloxy optionally substituted with A₂ such as 2-propynyloxy; C₁₋₆ alkylcarbonyl such as acetyl, ethylcarbonyl, propylcarbonyl or butylcarbonyl; phenylcarbonyl optionally substituted with A₄; C₁₋₆ alkylsulfonyl such as methylsulfonyl or ethylsulfonyl; phenylsulfonyl optionally substituted with A₄; mono- or di-C₁₋₆ alkylamino optionally substituted with A₂ such as methylamino, dimethylamino or ethylamino; C₃₋₇ cycloalkyl optionally substituted with A₃ such as cyclopentyl or cyclohexyl; C₅₋₇ cycloalkenyl optionally substituted with A₃ such as cyclopentenyl or cyclohexenyl; phenyl optionally substituted with A₄; phenoxy optionally substituted with A₄; anilino optionally substituted with A₄; or a heterocyclic group optionally substituted with A₃ such as tetrahydropyranyl, tetrahydrothiapyranyl or piperidinyl.

Substituent A₂ represents halogen such as fluorine or chlorine; cyano; phenyl optionally substituted with A₄; a saturated or unsaturated 5- or 6-membered heterocyclic group having at least a hetero atom selected from oxygen, sulfur and nitrogen and optionally substituted with A₃ such as tetrahydrofuryl, tetrahydrothienyl, pyrrolidinyl, pyrazolidinyl, imidazolidinyl, tetrahydropyranyl, tetrahydrothiapyranyl, piperidinyl, pyrimidinyl, pyridyl or morpholino; amino substituted with one or two groups of C₁₋₆ alkyl such as methyl or ethyl, C₃₋₇ cycloalkyl such as cyclopropyl, cyclopentyl or cyclohexyl or C₁₋₆ alkylcarbonyl such as acetyl, C₁₋₆ alkylcarbonyl such as acetyl, ethylcarbonyl or propylcarbonyl; C₁₋₆ alkoxycarbonyl such as methoxycarbonyl or ethoxycarbonyl; or one of the groups represented by the following formulae

OR₁₄, S(O)mR₁₅

(wherein, R₁₄ represents hydrogen, C₁₋₆ alkyl such as methyl or ethyl; C₂₋₆ alkenyl such as vinyl, 1-propenyl or 2-propenyl; C₂₋₆ alkynyl such as propynyl, C₁₋₆ haloalkyl such as chloromethyl, chloroethyl or trifluoromethyl; C₁₋₆ alkylcarbonyl such as acetyl or ethylcarbonyl; mono- or di-C₁₋₆ alkylcarbamoyl such as methylcarbamoyl or ethylcarbamoyl; C₃₋₇ cycloalkyl such as cyclopropyl, cyclopentyl or cyclohexyl; phenyl optionally substituted with A₄; or a saturated or unsaturated 5- or 6-membered heterocyclic group having at least a hetero atom selected from oxygen, sulfur and nitrogen and optionally substituted with A₃ such as tetrahydrofuryl, tetrahydrotluenyl, pyrrolidinyl, pyrazolidinyl, imidazolidinyl, tetrahydropyranyl, tetrahydrothiapyranyl, piperidinyl, pyrimidinyl, pyndyl or morpholino.

R₁₅ is as defined for R₇.

R₁₆ represents hydrogen or C₁₋₆ alkyl. R₁₇ is as defined for R₈, and R₁₈ as R₉.)

Substituent A₃ represents halogen such as fluorine or chlorine; hydroxyl; oxo, C₁₋₆ alkyl such as methyl, ethyl or propyl; C₁₋₆ alkoxy such as methoxy, ethoxy or propoxy, C₁₋₆ alkoxy C₁₋₆ alkoxy such as methoxymethoxy or methoxyethoxy; amino; mono- or di-C₁₋₆ alkylamino such as methylamino, ethylamino, dimethylamino or methylethylamino; C₁₋₆ alkylcarbonyloxy such as acetyloxy or ethoxycarbonyloxy; C₁₋₆ alkoxycarbonyl such as methoxycarbonyl or ethoxycarbonyl; mono- or di-C₁₋₆ alkylcarbamoyl such as methylcarbamoyl, dimethylcarbamoyl or ethylcarbamoyl; or mono- or di-C₁₋₆ alkylcarbonylamino such as acetylamino, ethylcarbonylamino or diacetylamino.

A₄ represents halogen such as fluorine or chlorine; nitro; cyano; hydroxyl; C₁₋₆ alkyl such as methyl, ethyl or propyl; C₁₋₆ haloalkyl such as chloromethyl or trifluoromethyl; C₁₋₆ alkoxy such as methoxy, ethoxy or propoxy; C₁₋₆ alkylthio such as methylthio or ethylthio; C₁₋₆ alkylsulfenyl such as methylsulfenyl or ethylsulfenyl; C₁₋₆ alkylsulfonyl such as methanesulfonyl or ethanesulfonyl; C₁₋₆ alkoxy C₁₋₆ alkoxy such as methoxymethoxy or methoxyethoxy; C₁₋₆ haloalkoxy such as chloromethoxy, chloroethoxy or trifluoromethoxy; C₁₋₆ alkylcarbonyl such as acetyl or ethylcarbonyl; C₁₋₆ alkoxycarbonyl such as methoxycarbonyl or ethoxycarbonyl; or C₁₋₆ alkylcarbonyloxy such as acetyloxy or ethylcarbonyloxy.

When X is NR₄, R₄ represents hydrogen, C₁₋₆ alkyl such as methyl, ethyl, propyl, isopropyl or butyl; C₃₋₇ cycloalkyl such as cyclopentyl or cyclohexyl; C₁₋₆ alkylcarbonyl such as acetyl, ethylcarbonyl or i-propylcarbonyl; C₁₋₆ haloalkylcarbonyl such as fluoroacetyl, chloroacetyl or trifluoroacetyl; C₂₋₆ alkenylcarbonyl such as allylcarbonyl; C₁₋₆ alkoxycarbonyl such as methoxycarbonyl, ethoxycarbonyl, propoxycarbonyl or hexyloxycarbonyl; C₁₋₆ haloalkoxycarbonyl such as fluoromethoxycarbonyl, chloromethoxycarbonyl or trifluoromethoxycarbonyl; or mono- or di-C₁₋₆ alkylcarbamoyl such as methylcarbamoyl, ethylcarbamoyl, propylcarbamoyl, i-propylcarbamoyl, hexylcarbamoyl or dimethylcarbamoyl.

When X is CHR₅, R₅ represents hydrogen or C₁₋₆ alkoxycarbonyl such as methoxycarbonyl, ethoxycarbonyl, n-propoxycarbonyl, i-propoxycarbonyl, n-butoxycarbonyl, t-butoxycarbonyl or hexyloxycarbonyl.

"A pharmaceutically acceptable composite" refers to a composite comprising a compound mentioned above and an atoxic, low-molecular-weight compound that interacts with the compound through ionic, hydrogen or coordinate bonds at a specific ratio. The composite liberates the said compound in an aqueous solution. Its actual examples include salts such as hydrochlorides, organic acid salts and amino acid salts; and solvates such as hydrates.

The compounds of Formula [I] have at least structural isomers with regard to Substituents R, and R₂ on the rings. Each isomer has optical isomers. The present invention is not restricted to specific isomers and covers all possible isomers and racemic compounds including those mentioned above. The compounds of the present invention include prodrugs of the compounds and their metabolites as the case may be.

Processes for the preparation of compounds relevant in the present invention are described.

The compounds of the present invention can be produced according to, for example, the reaction scheme shown below: 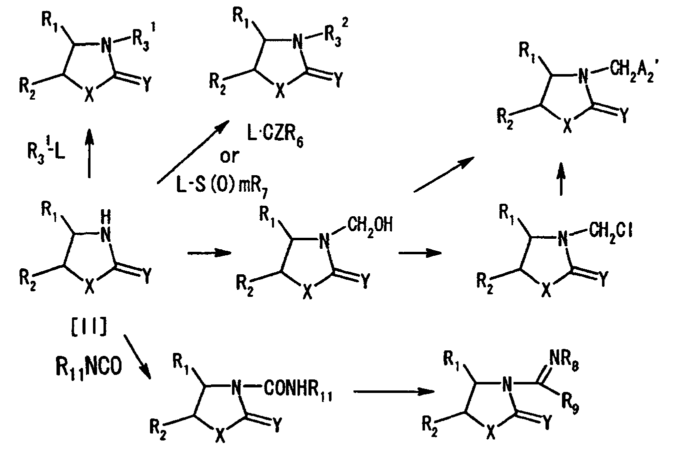 (wherein, L is halogen; R₁, R₂, R₆, R₇, R₈, R₉, R₁₁, Z and m are as defined above; R₃¹ is optionally substituted C₁₋₆ alkyl or optionally substituted C₂₋₆ alkenyl; R₃² is a group represented by Formula CZR₆ or S(O)mR₇; A₂' is a group represented by Formula OR₁₄ or S(O)mR₁₅).

Among the compounds of Formula [II], compounds where X is oxygen or sulfur can be prepared according to the methods disclosed in US 4,431,814, etc.

Compounds where X is carbon can be prepared according to, for example, the following reaction scheme: 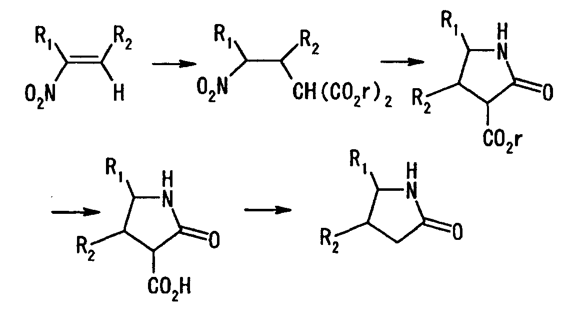 (wherein, R₁ and R₂ are as defined above).

When X is nitrogen, compounds, for example where R₃ is a group represented by Formula CZR₆ mentioned above, can be prepared according to the following reaction scheme. 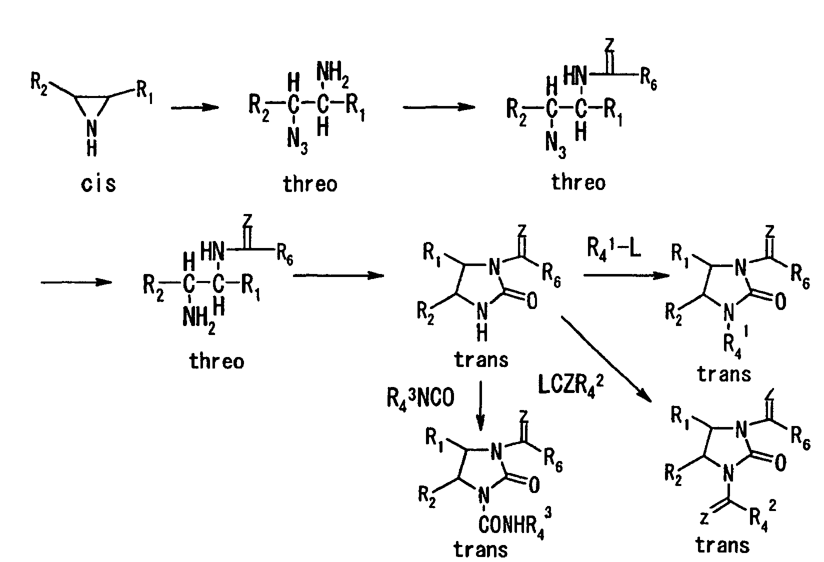 (wherein, R₁, R₂ and R₆ are as defined above; R₄¹ is C₁₋₆ alkyl or C₃₋₇ cycloalkyl; R₄² is C₁₋₆ alkyl, C₁₋₆ haloalkyl, C₁₋₆ alkenyl, C₁₋₆ alkoxy or C₁₋₆ haloalkoxy; and R₄³ is C₁₋₆ alkyl).

The structures of the compounds of the present invention were determined by MASS, NMR and other means.

### <Anti-inflammatory Agents>

A compound of Formula [I] or a pharmaceutically acceptable composite thereof may be administered, as it is or together with common carriers for pharmaceutical formulations, to a human and an animal. It can be administered by any route Either a systemic administration or local application, i.e. non-systemic application, is selected to use as required. Examples of forms for the administration include pharmaceutical formulations for oral administration such as tablets, capsules, granules, powders, oral solutions and troches; and parenteral solutions or suspensions such as those for intravenous, intramuscular and subcutaneous injections. In addition, other administration routes may be utilized, including administration of suppository through the rectum and an aerosol or powder inhalant through the respiratory tract (inhalation through a nose or through a mouth). A pharmaceutical formulation suitable for a topical application is in a form that the active ingredient can penetrate into the inflammatory region through the skin or mucosa. Its examples include solutions, liniments, creams, emulsions, ointments and pastes, as well as drops suitable for the treatment of eyes, ears and noses Any amount of an active ingredient can be administered. A dose may be determined at discretion over a wide range, depending on an administration route, a compound selected, and a human or an animal to be administered. To attain the intended effect, it is preferable to administer a compound of the present invention at a daily dose of 0.01 to 100 mg per kg body weight once or a few times a day. A pharmaceutical formulation preferably contains 0.1 to 1,000 mg of an active ingredient.

In the present invention, pharmaceutical formulations for oral administration, such as tablets, capsules, granules and oral solutions, are produced according to conventional methods: A tablet is produced by that a compound of Formula [I] or a pharmaceutically acceptable composite thereof is mixed with pharmaceutical excipients, such as starch, lactose, gelatin, magnesium stearate, talc and gum Arabic, and made tablets. A method for producing a capsule is that a compound of Formula [I] or a pharmaceutically acceptable composite thereof is mixed with an inactive pharmaceutical filler or diluent, and filled in hard gelatin capsules, soft capsules or the like. A medicated syrup or elixir of oral solution is prepared by that a compound of Formula [I] or a pharmaceutically acceptable composite thereof is mixed with a sweetener such as sucrose, an antiseptic such as methylparaben or propylparaben, a coloring agent, a flavor and others. A parenteral pharmaceutical formulation is produced according to a conventional process: For example, a compound of Formula [I] or a pharmaceutically acceptable composite thereof is dissolved in a sterilized liquid carrier. A preferred carrier is water or a saline solution. A liquid formulation with a desired transparency, stability and congeniality for parenteral use is produced by dissolving about 0.1 to 1,000 mg of an active ingredient in water and an organic solvent, and further in polyethylene glycol having a molecular weight of 200 to 5,000. Such a solution favorably contains a lubricant such as polyvinyl pyrrolidone, polyvinyl alcohol, sodium carboxymethyl cellulose or methyl cellulose. In addition, the said solution may contain a bactericide and fungicide such as benzyl alcohol, phenol or thimerosal, and, if necessary, an isotonic fluid such as sucrose or sodium chloride, a local anesthetic, a stabilizer, a buffer agent and others. To improve the stability furthermore, a pharmaceutical formulation for parenteral use is frozen after filling, and water is removed according to a freeze-drying technique known by those in the art. Accordingly, a solution can be prepared from the freeze-dried powder immediately before use.

A compound of Formula [I] or a pharmaceutically acceptable composite thereof has a strong inhibitory activity on the liberation of arachidonic acid in an inflammatory response. Only an extremely weak inhibitory activity is however recognized on the enzymatic hydrolysis by the secretary group IB-PLA (2) (hereinafter referred to as IB-sPLA (2), molecular weight 14 kDa) of the porcine pancreas, when a phospholipid whose carbon in the oleic acid substituent at Position 2 is labeled with a radioactive isotope is used as a substrate. Thanks to the above fact, the compound or composite is expected to be highly safe. Some of the compounds of the present invention have herbicidal, insecticidal, acaricidal and/or antifungal activities as well, being useful as agrochemicals. In particular, compounds with inhibitory activities against fungi that cause infectious diseases to mammals are expected to have excellent efficacy against diseases accompanied with opportunistic fungal infections, such as pneumonia.

### Best Form to Implement the Invention:

The present invention is described in more detail in reference to Examples. The scope of the present invention is not limited by the examples as they are only shown as examples.

Example 1

Preparation of trans-5-(4-chlorophenyl)-4-methyl-3-(4-methoxymethoxy-3-nitrobenzyl)-2-thiazolidinone and tans-5-(4-chlorophenyl)-4-methyl-3-(4-hydroxy-3-nitrobenzyl)-2-thiazolidinone

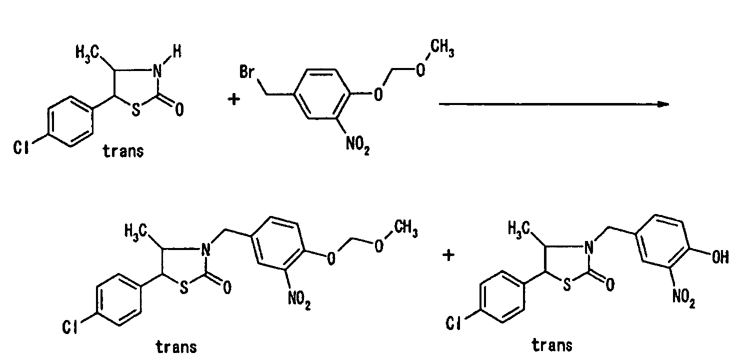

To a mixed solution of 0.2g of 60% sodium hydride and 30 ml of N,N-dimethylformamide was added 1.0g of trans-5-(4-chlorophenyl)-4-methyl-2-thiazolidinone at room temperature, and further 1.2g of 3-nitro-4-methoxymethoxybenzyl bromide was added at room temperature. The resulting solution was reacted at room temperature for 15 hours, poured into ice-water, and extracted with ethyl acetate. The extract was washed with water and dried over magnesium sulfate. The solvent was dried up under reduced pressure. The obtained residue was purified through column chromatography (silica gel, benzene/ethyl acetate = 9/1) to give 0.6g of trans-5-(4-chlorophenyl)-4-mehyl-3-(4-methoxymethoxy-3-nitrobenzyl)-2-thiazolidinone as an oily product (refractive index n_{D} (25.5°C): 1.5750) and 0.2g of trans-5-(4-chlorophenyl)-4-methyl-3-(4-hydroxy-3-nitrobenzyl)-2-thiazolidinone as crystals (mp. 138 to 141°C).

Example 2

Preparation of trans-5-(4-chlorophenyl)-4-methyl-3-(4-hydroxy-3-nitrobenzyl)-2-thiazolidinone

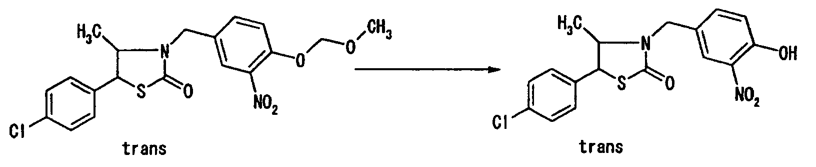

A mixed solution of 1.0g of trans-5-(4-chlorophenyl)-4-methyl-3-(4-methoxymethoxy-3-nitrobenryl)-2-thiazolidinone, 0.05g of concentrated sulfuric acid and 20 ml of acetic acid was refluxed for 2 hours. The reaction solution was poured into ice-water and extracted with ethyl acetate. The extract was washed with water and dried over magnesium sulfate. The solvent was dried up under reduced pressure. The obtained residue was washed with n-hexane to give 0.8g of the title compound as crystals. m.p. 138 to 141°C

Example 3

Preparation of trans-4-methyl-5-phenyl-3-cyclohexylcarbamoyhmidazolidin-2-one

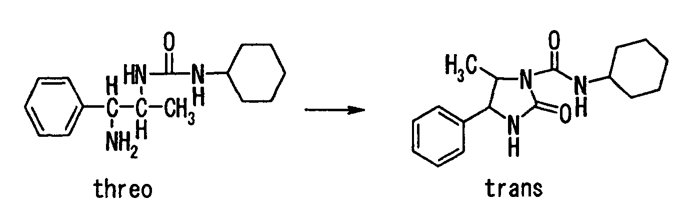

To 30 ml of chloroform were added 1.1g of threo-2-cyclohexylcarbamoylamino-1-amino-1-phenylpropane and 1.0g of N,N-dimethylaniline, and, while cooling with ice, 0.5g of trichloroformate was dropped. The resulting solution was reacted for about 5 hours. Upon the completion of the reaction, the reaction solution was treated with a 0.1N aqueous solution of hydrogen chloride and a 5% aqueous solution of sodium bicarbonate, washed with water, dried over magnesium sulfate, and concentrated under reduced pressure. The obtained crystals were washed with hexane to give 1.0g of the title compound.

Example 4

Preparation of trans-4-methyl-5-phenyl-3-cyclohexylcarbamoyl-1-methylimidazolidin-2-one

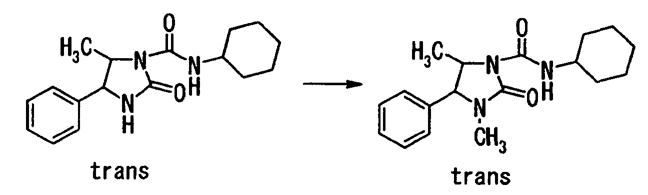

0.55g of trans-4-methyl-5-phenyl-3-cyclohexylcarbamoylimidazolidin-2-one was dissolved in 15 ml of DMF, and, while cooling with ice, 0.08g of 60% sodium hydride was added and further 0.3g of methyl iodide was dropped. The resulting solution was reacted for an hour, poured into ice-water and extracted with ethyl acetate. The extract was washed with water, dried over magnesium sulfate, and concentrated under reduced pressure. The obtained oily product was separated and punfied through column chromatography to give 0.5g of the title compound. m.p. 107.5 to 108.5°C

Example 5

Preparation of trans-5-methyl-4-(4-chlorophenyl)-1-(2-tetrahydropyranylcarbamoyl)-2-pyrrolidinone

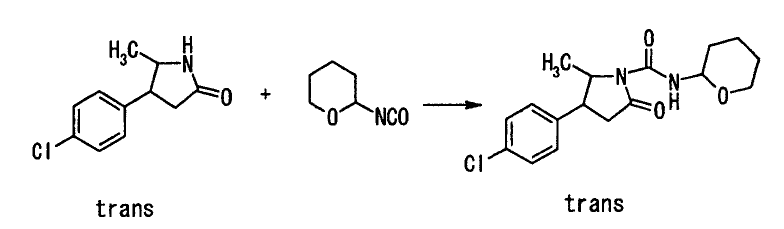

0.8g of trans-5-methyl-4-(4-chlorophenyl)-2-pyrrolidinone was dissolved in 4 ml of DMSO, and 3 drops of DBU and 0.5g of 2-tetrahydropyranylisocyanate were dropped into in this order. The resulting solution was reacted for 4 hours at room temperature, poured into ice-water and extracted with chloroform. The extract was washed with water, dried over magnesium sulfate, and concentrated under reduced pressure. The obtained oily product was separated and purified through column chromatography to give 0.9g of the title compound. m.p. 136 to 143°C

Representative examples of the compounds of the present invention that are produced according to the processes of the present invention, including those described in the above examples, are shown in Tables 1 to 4. The symbols and abbreviations used in the tables stand for the following.

Me: methyl, Et: ethyl, Pr: propyl, Bu: butyl, Pent: pentyl, Hex: hexyl, Hep: heptyl, Ac: acetyl, Naph: naphthyl, THNaph: 1,2,3,4-tetrahydronaphthyl, Ph: phenyl, Bn: benzyl, Bz: benzoyl, Pyr: pyridyl, THF: tetrahydrofuranyl, THP: tetrahydropyranyl, Dxn: dioxanyl, Morph: morpholino, n: normal, i: iso, s: secondary, t: tertiary, and c: cyclo.

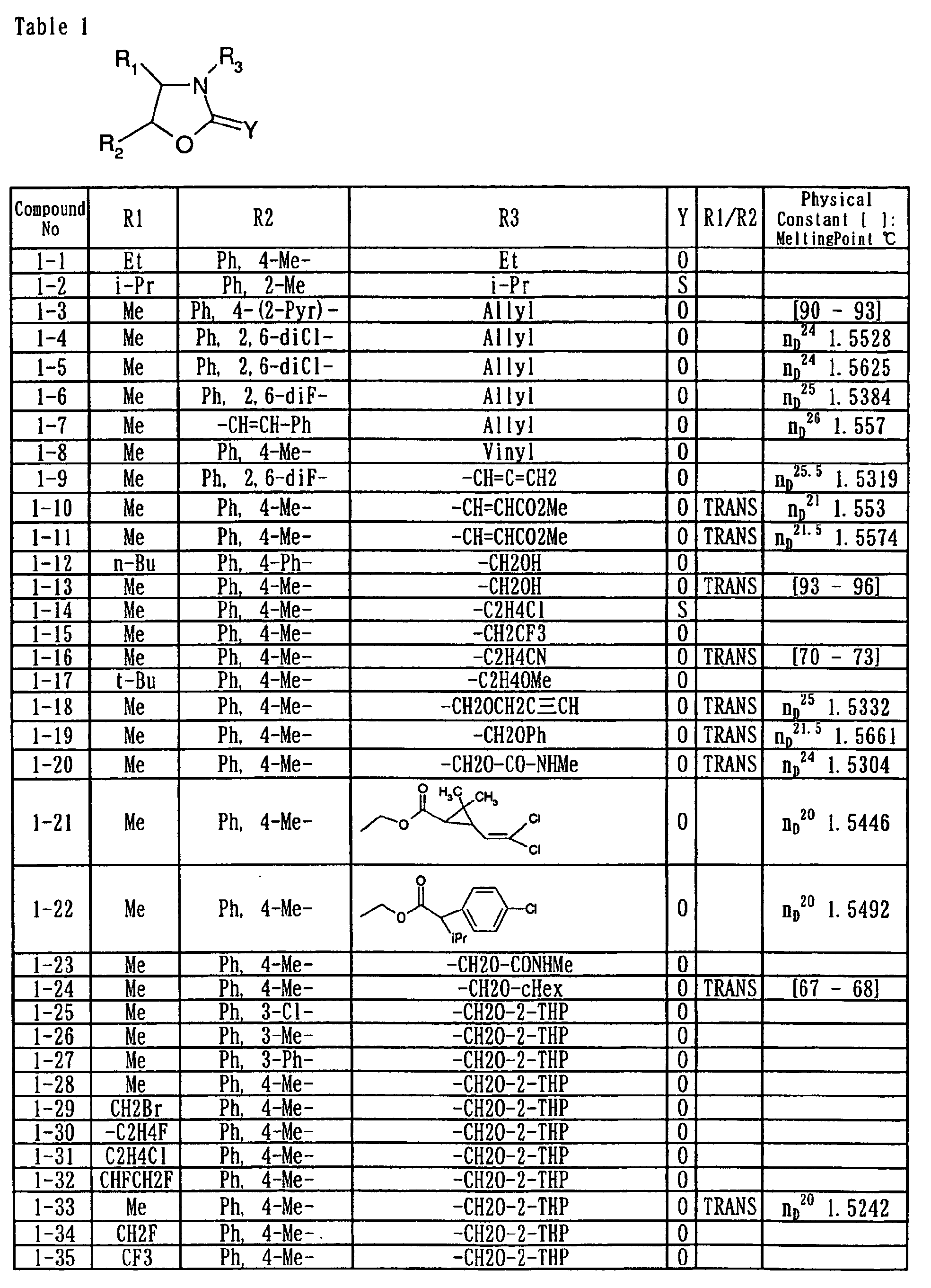

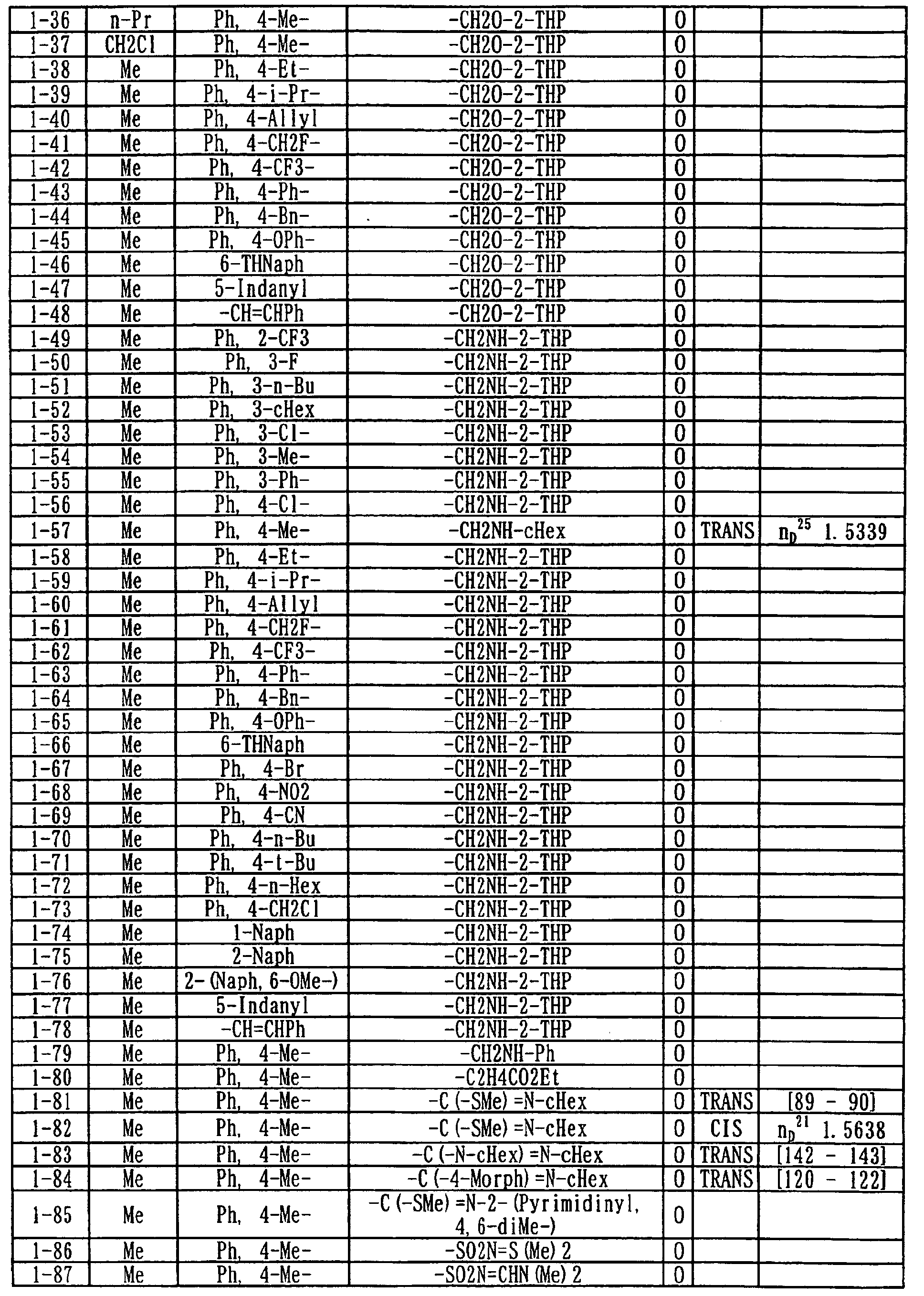

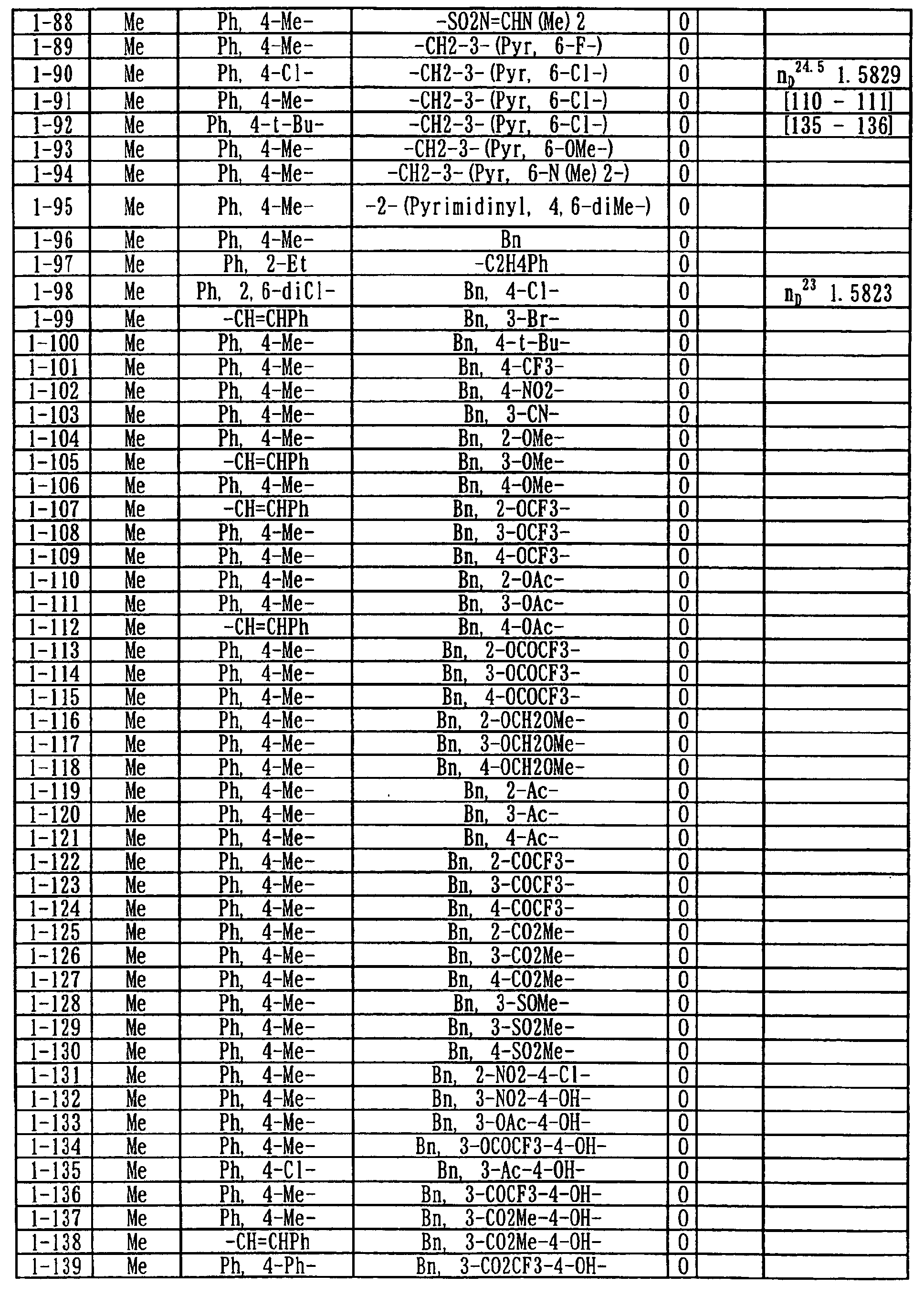

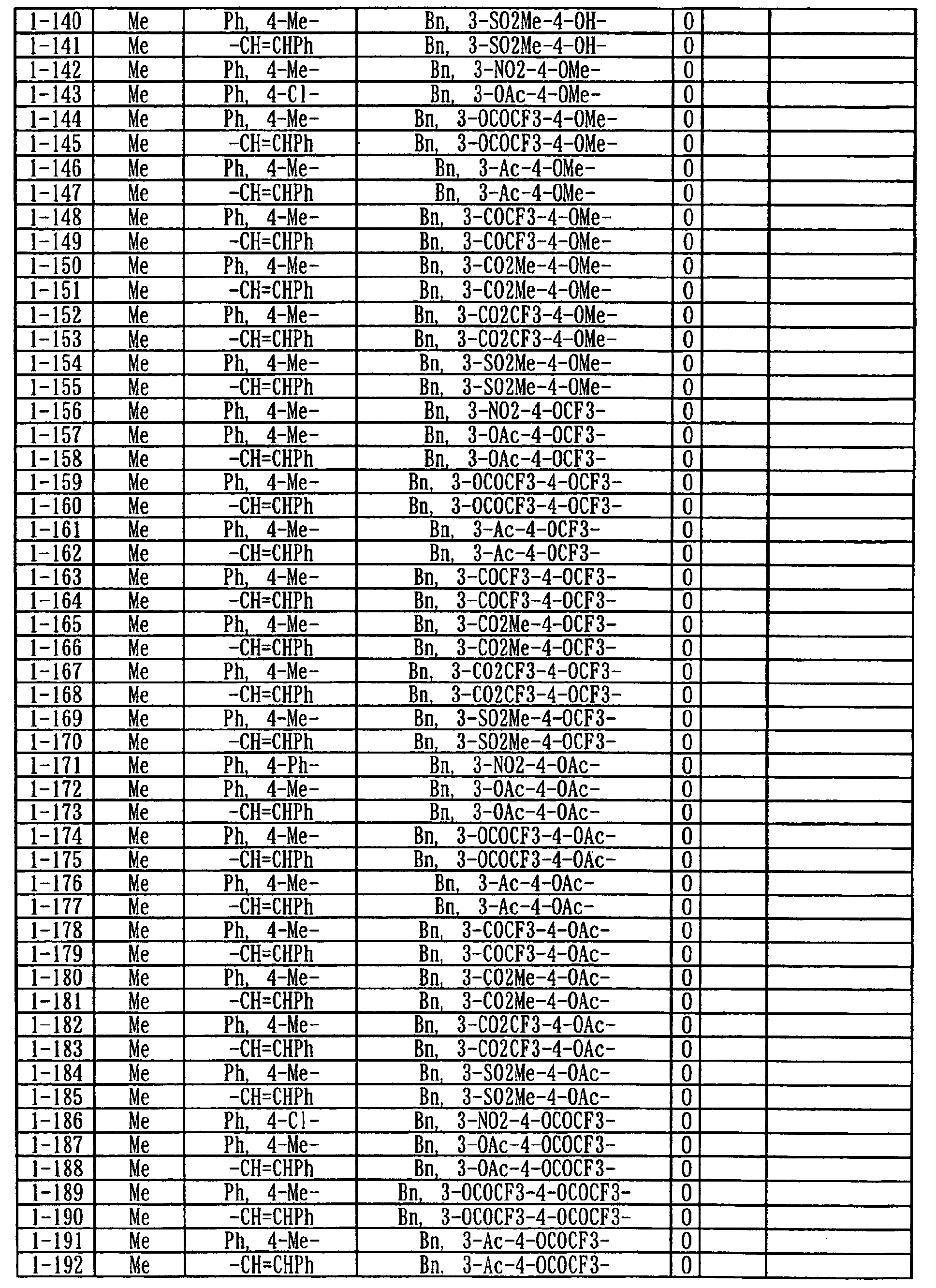

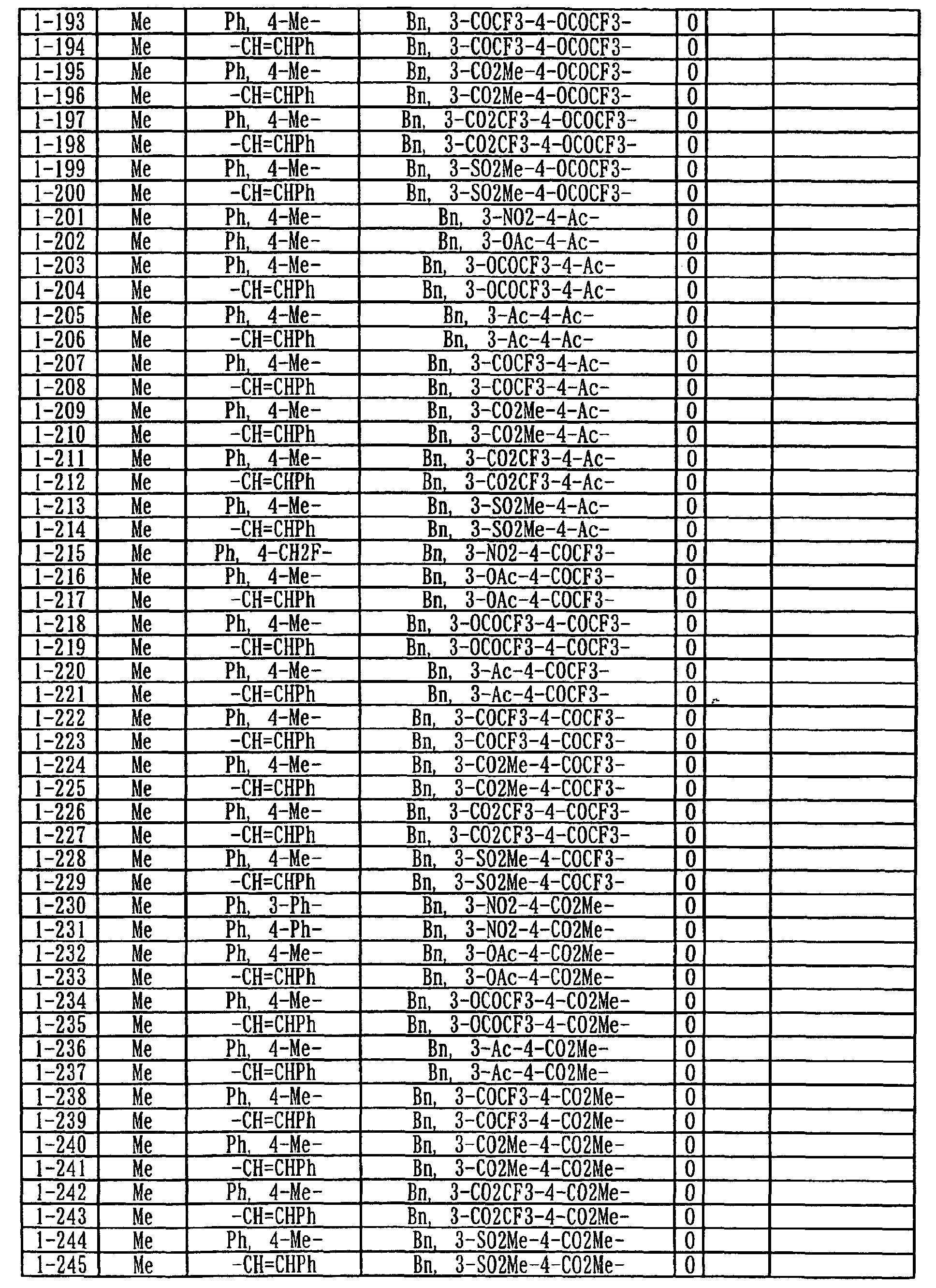

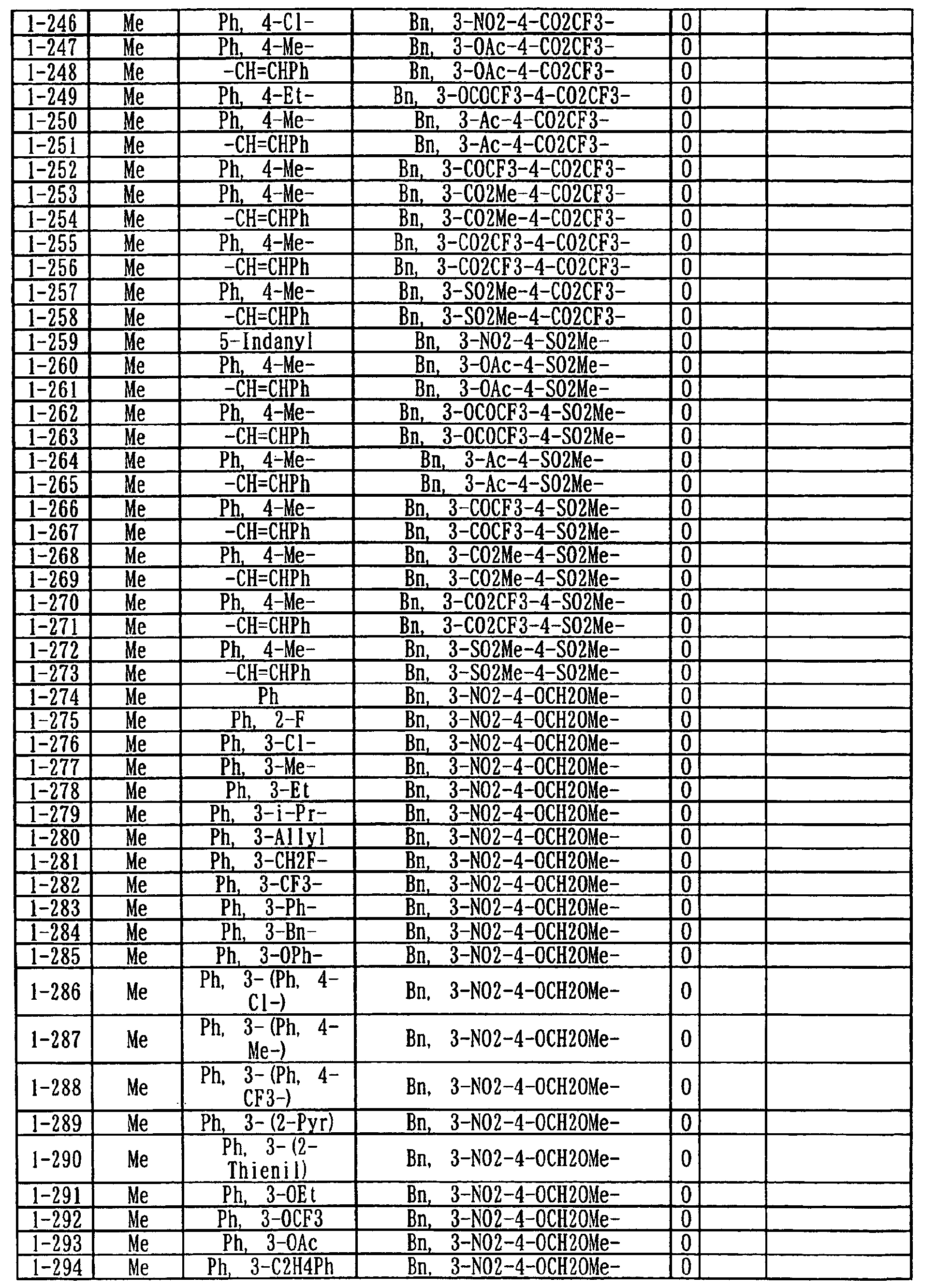

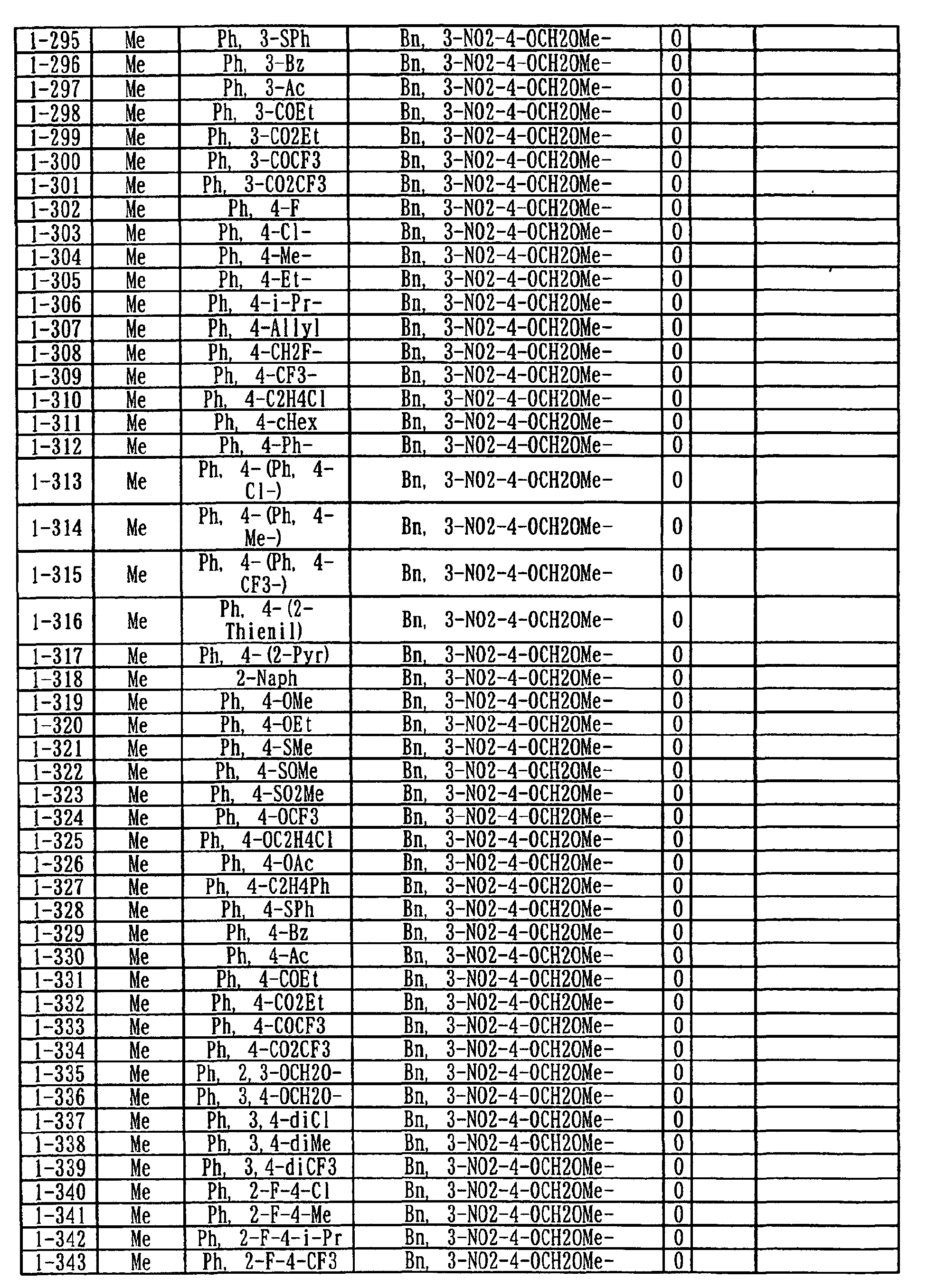

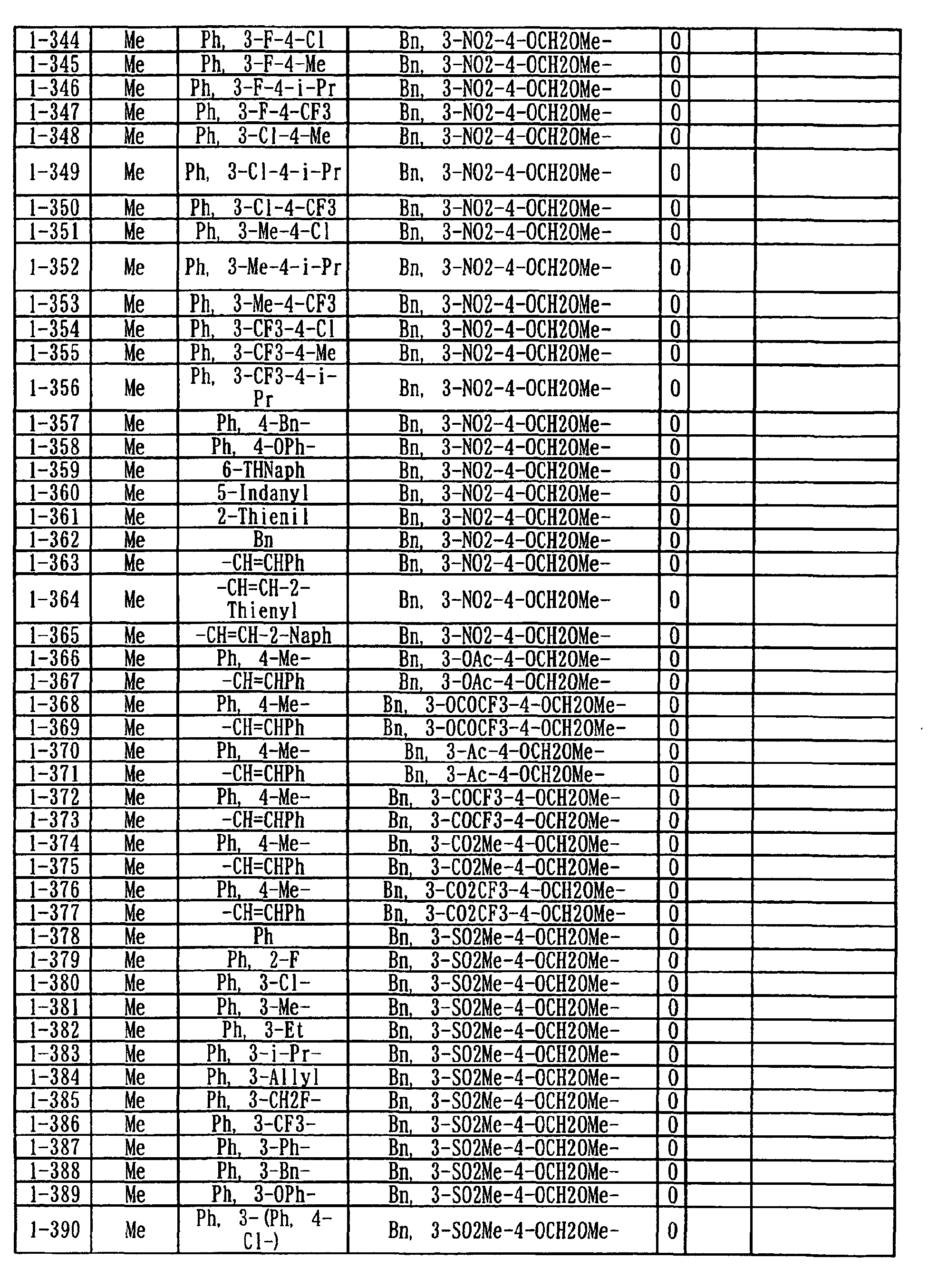

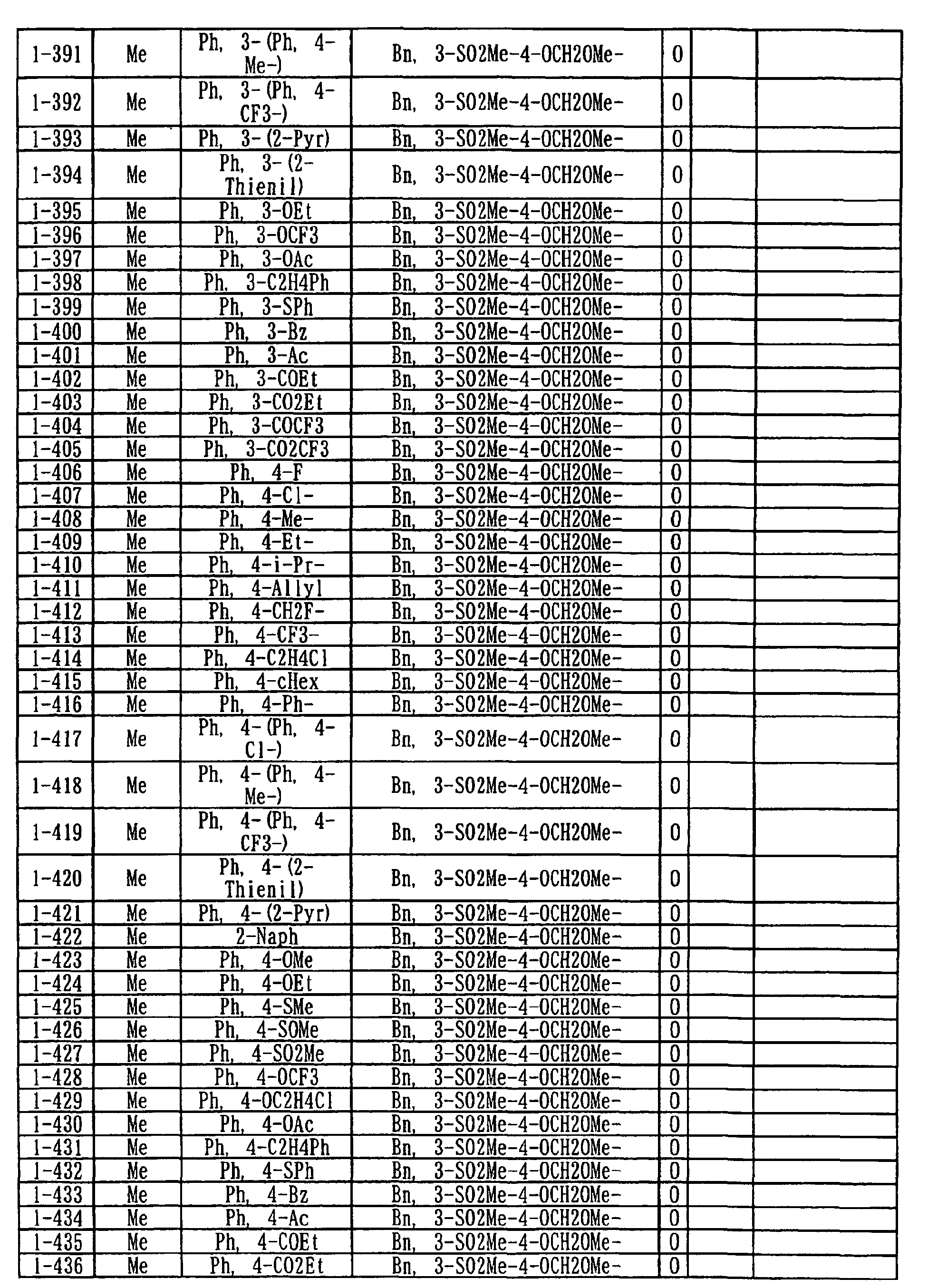

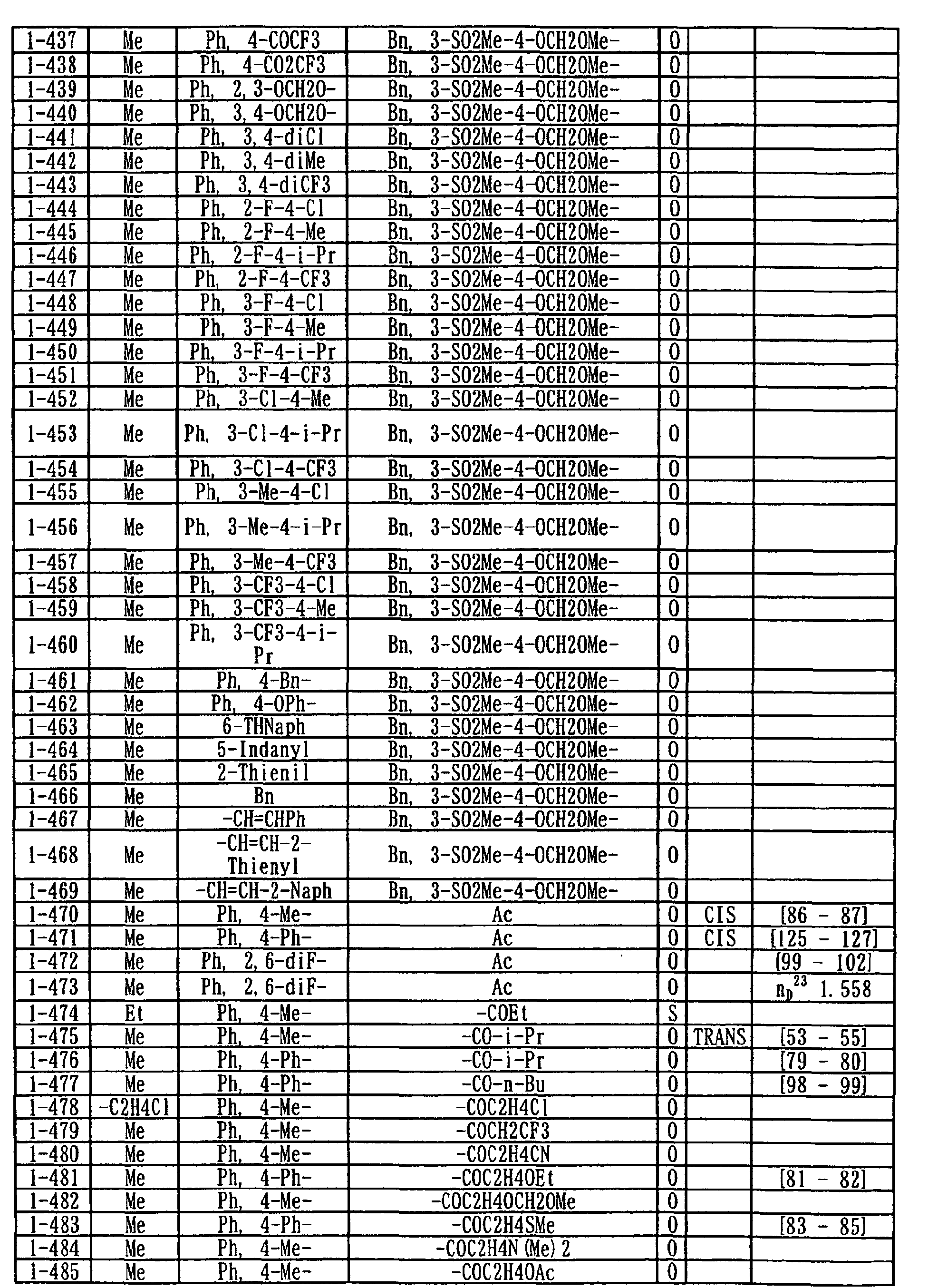

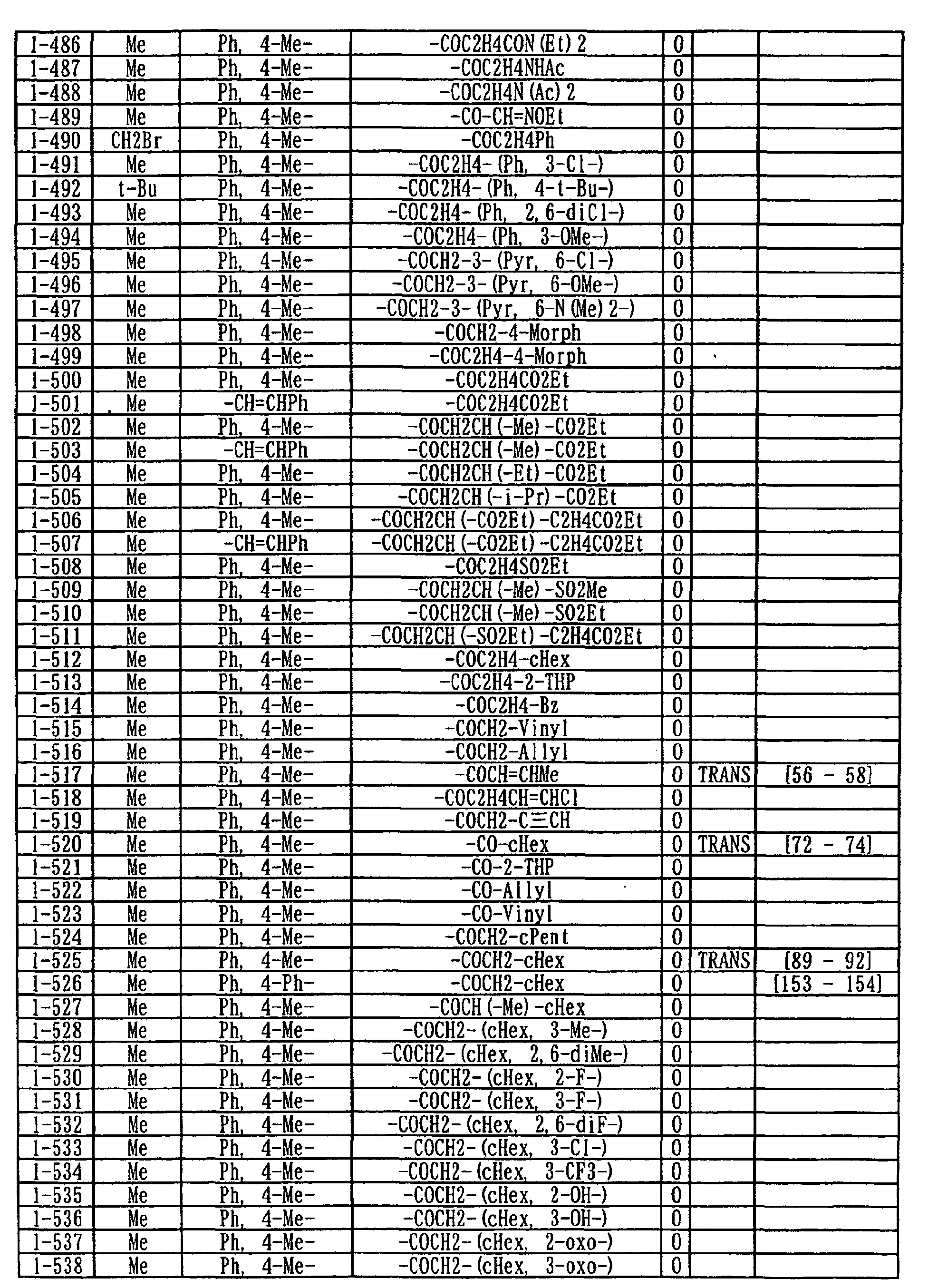

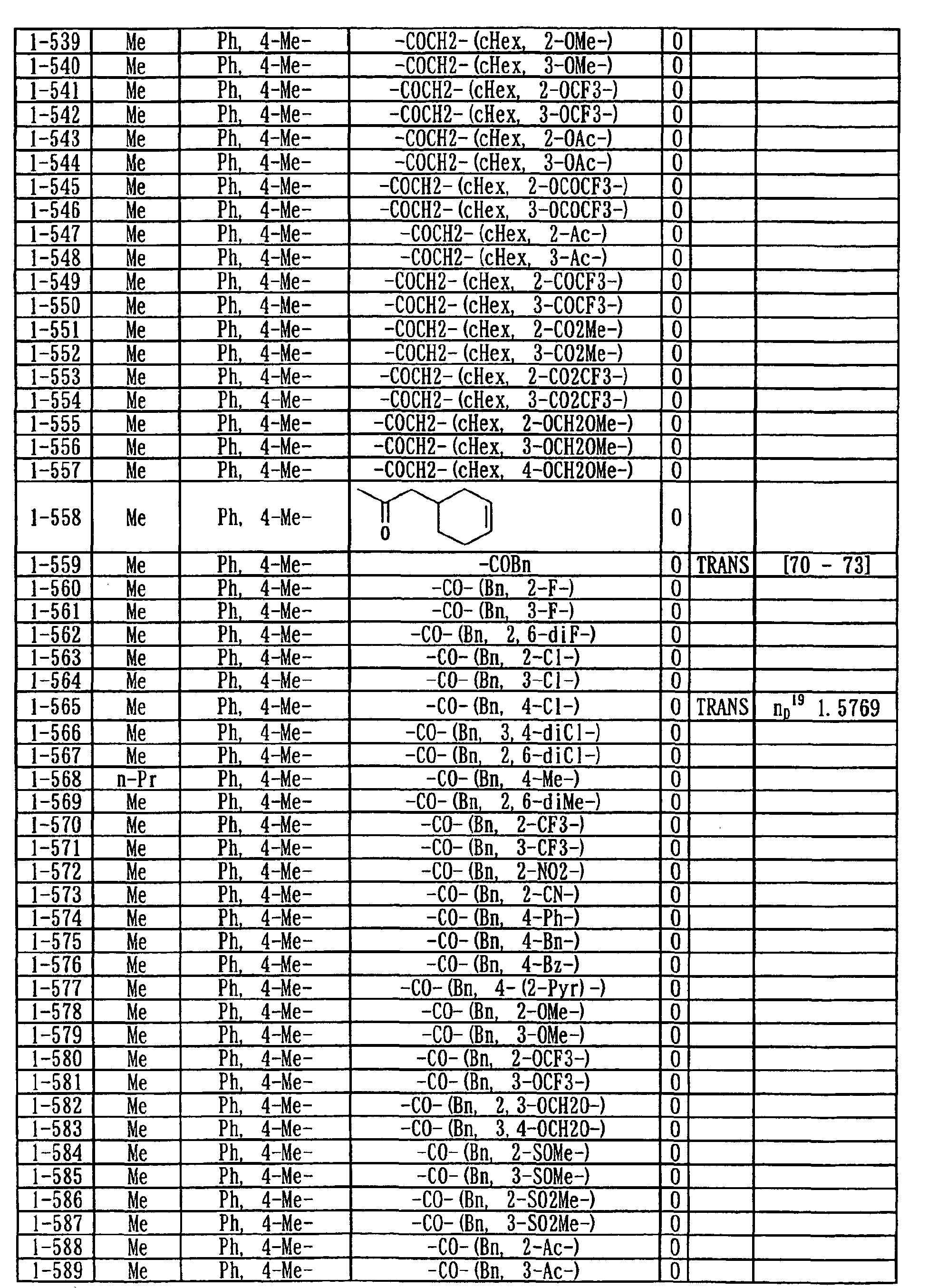

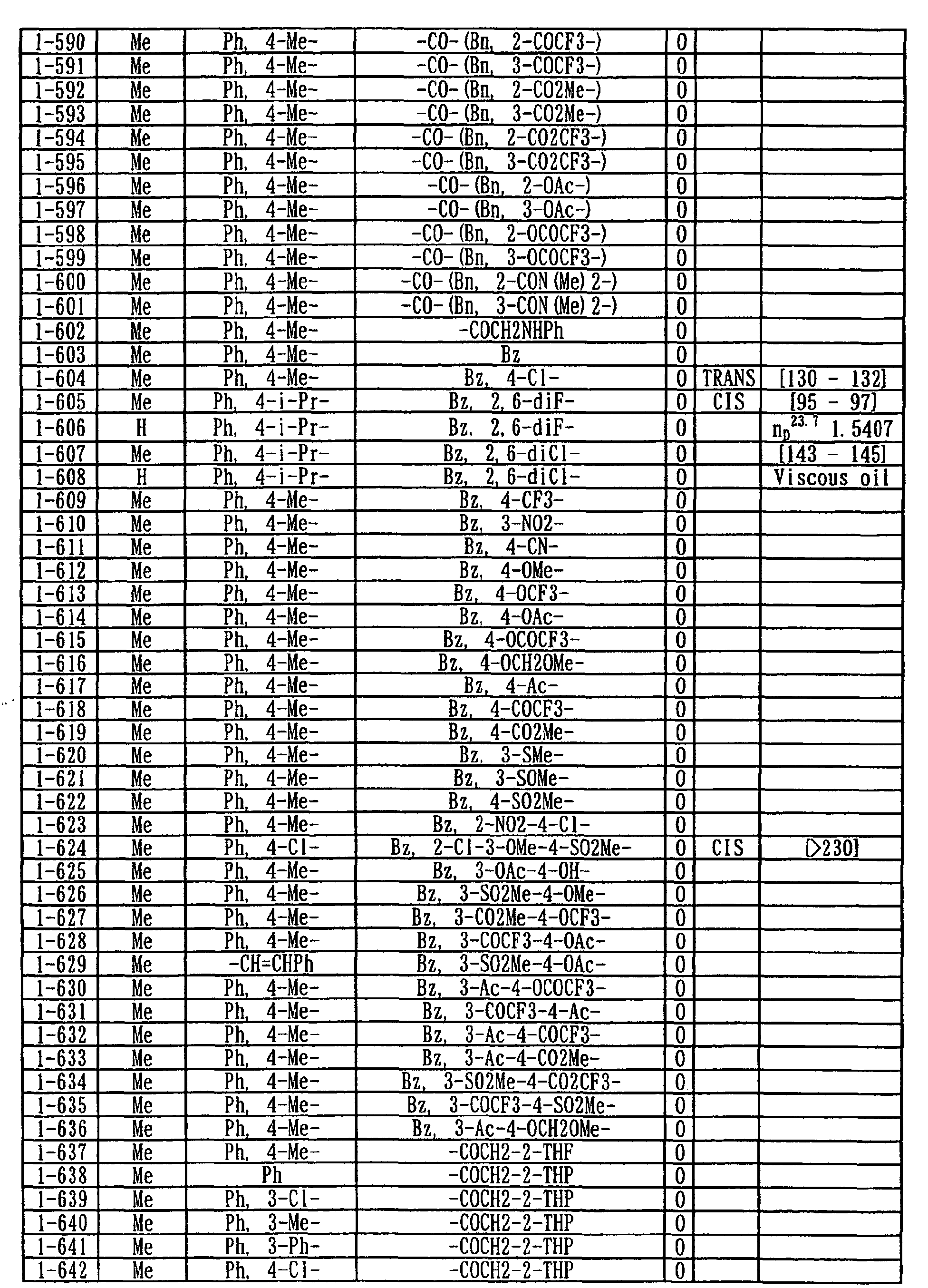

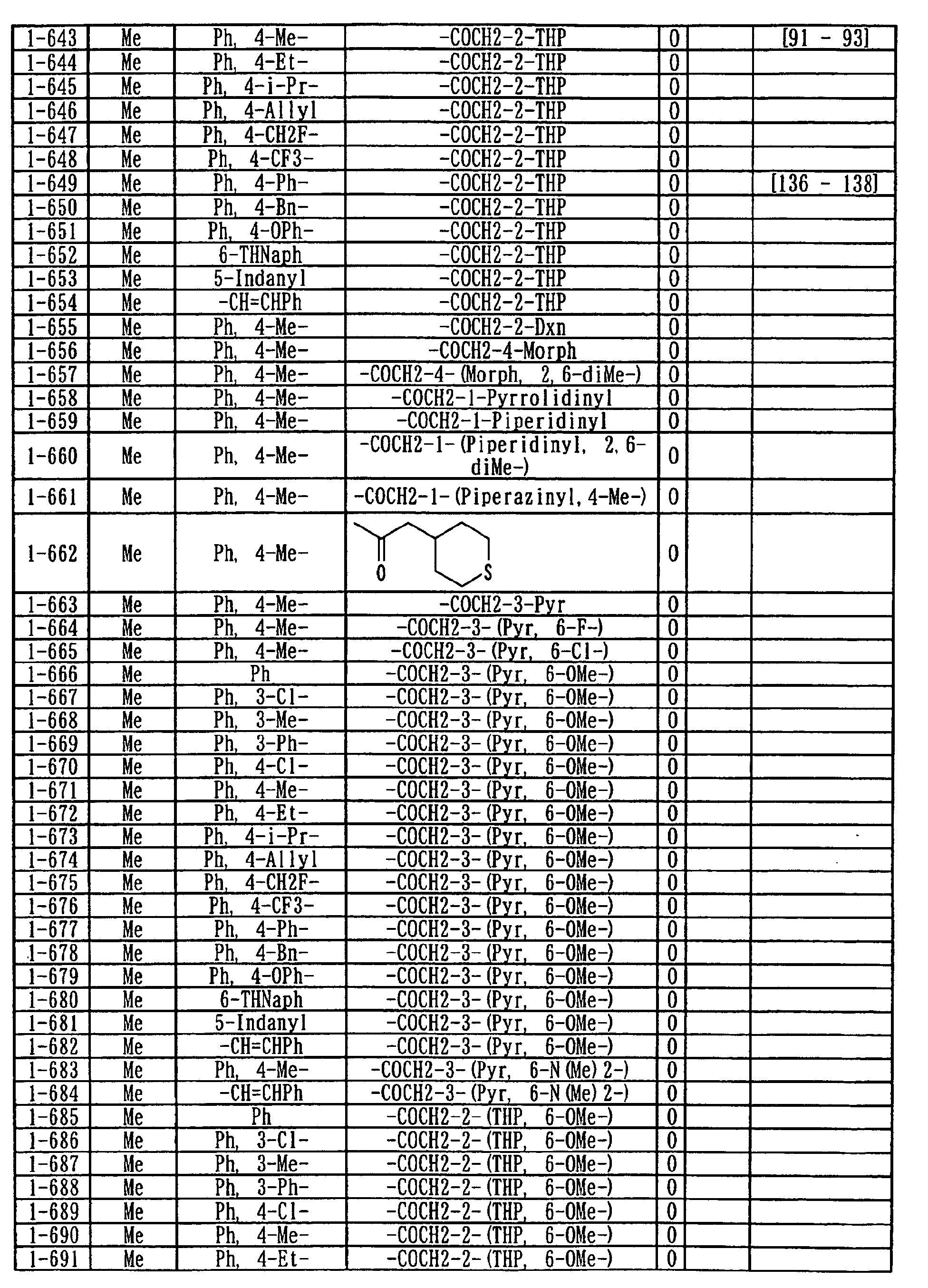

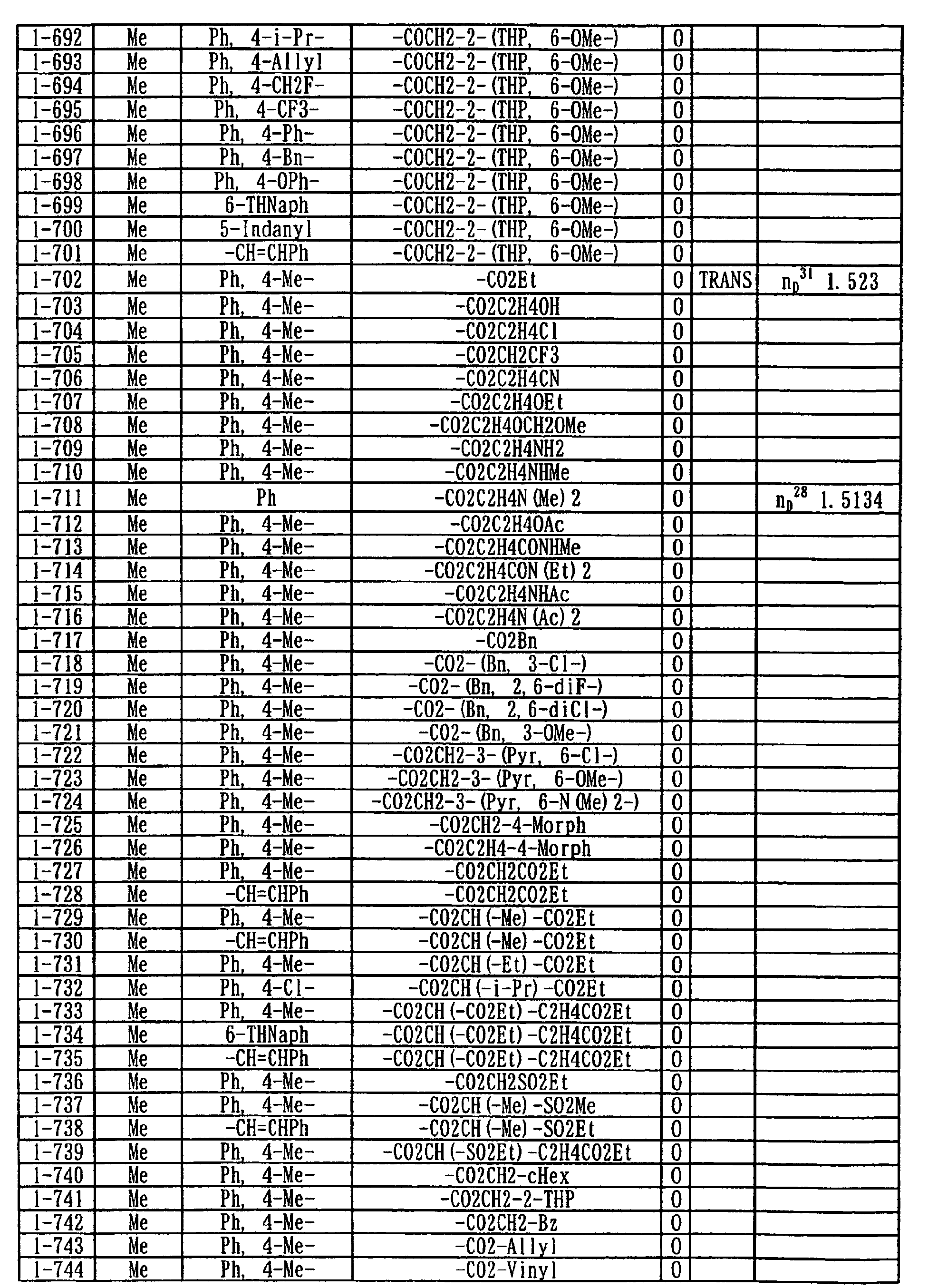

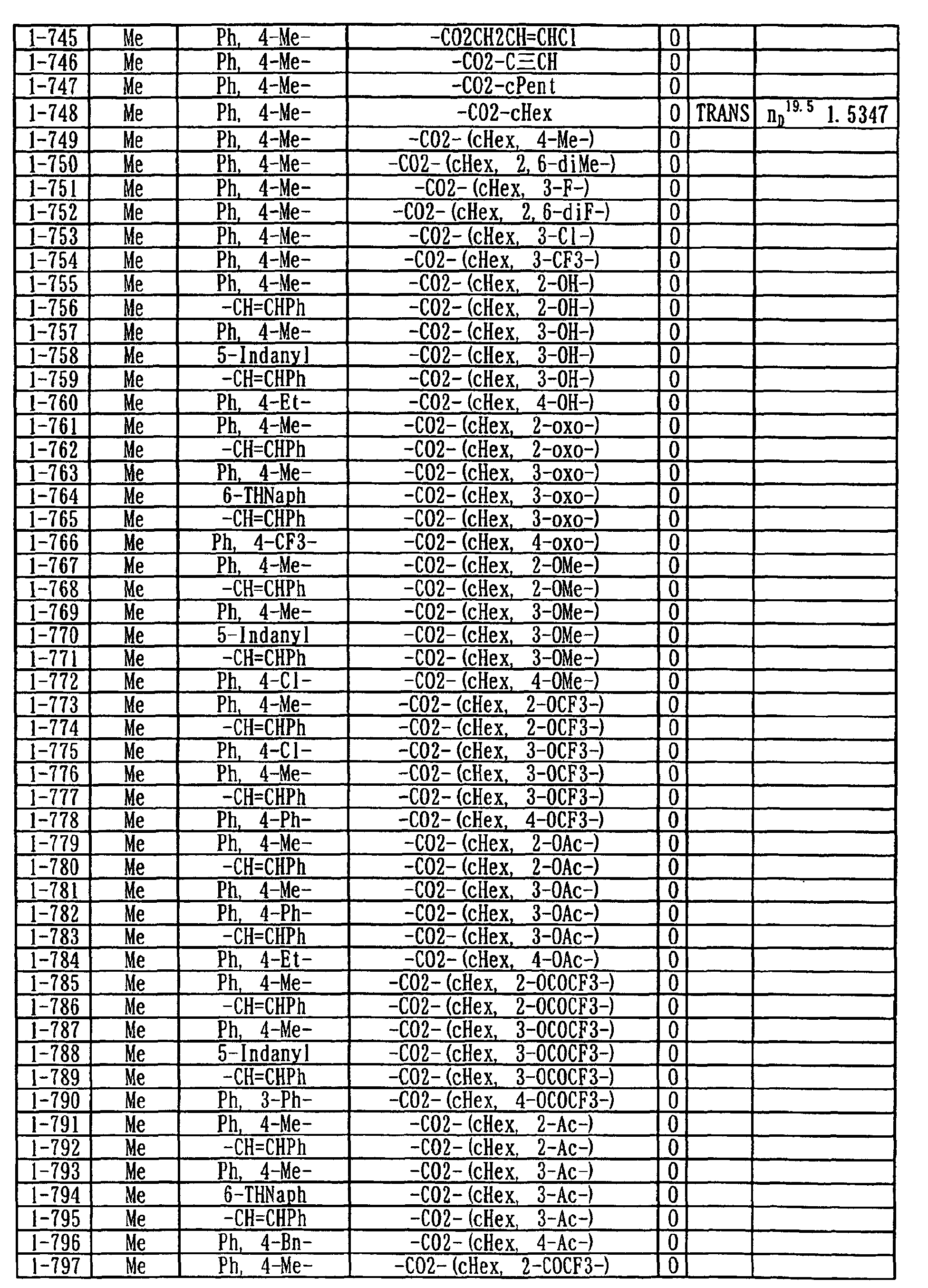

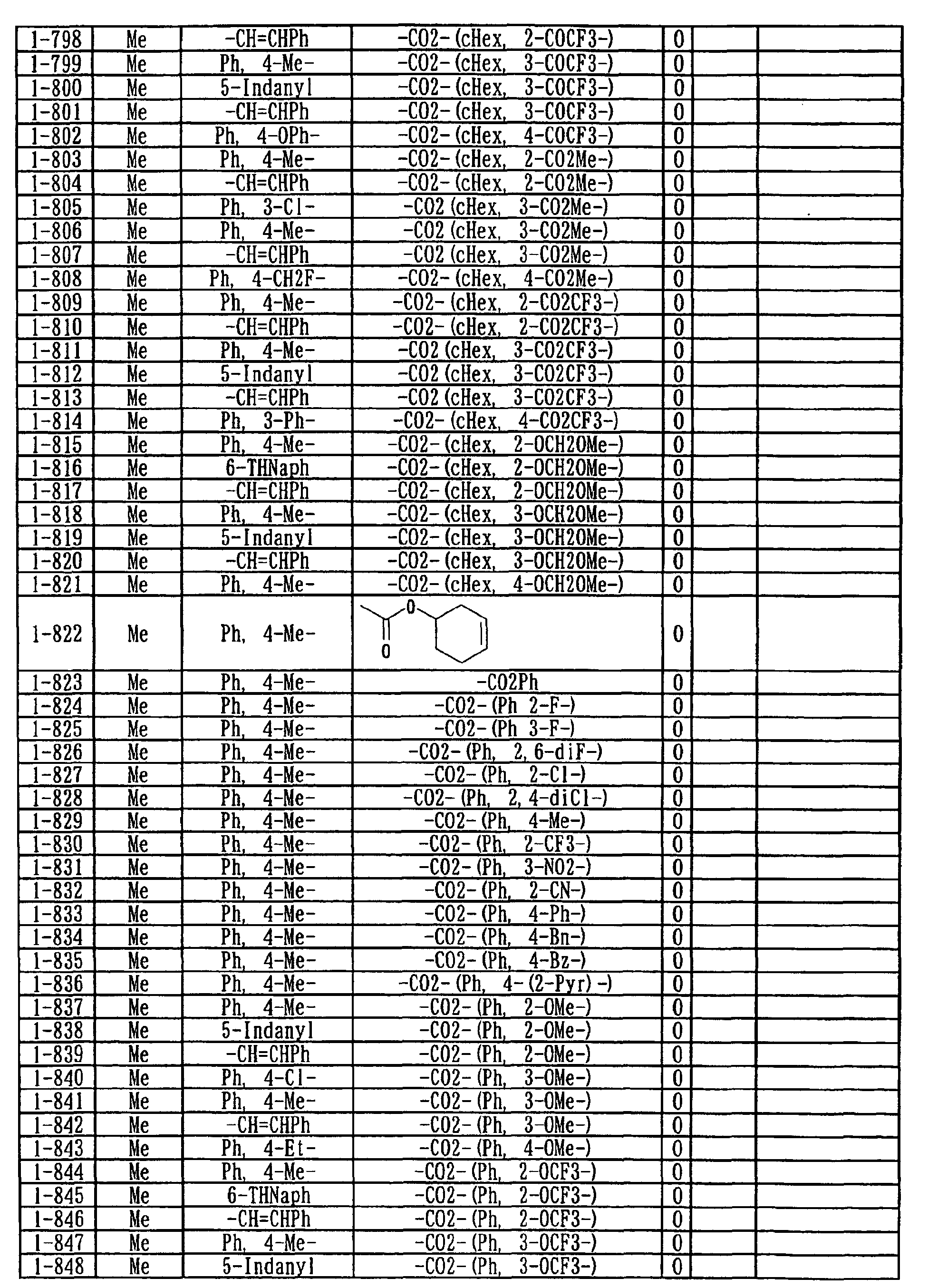

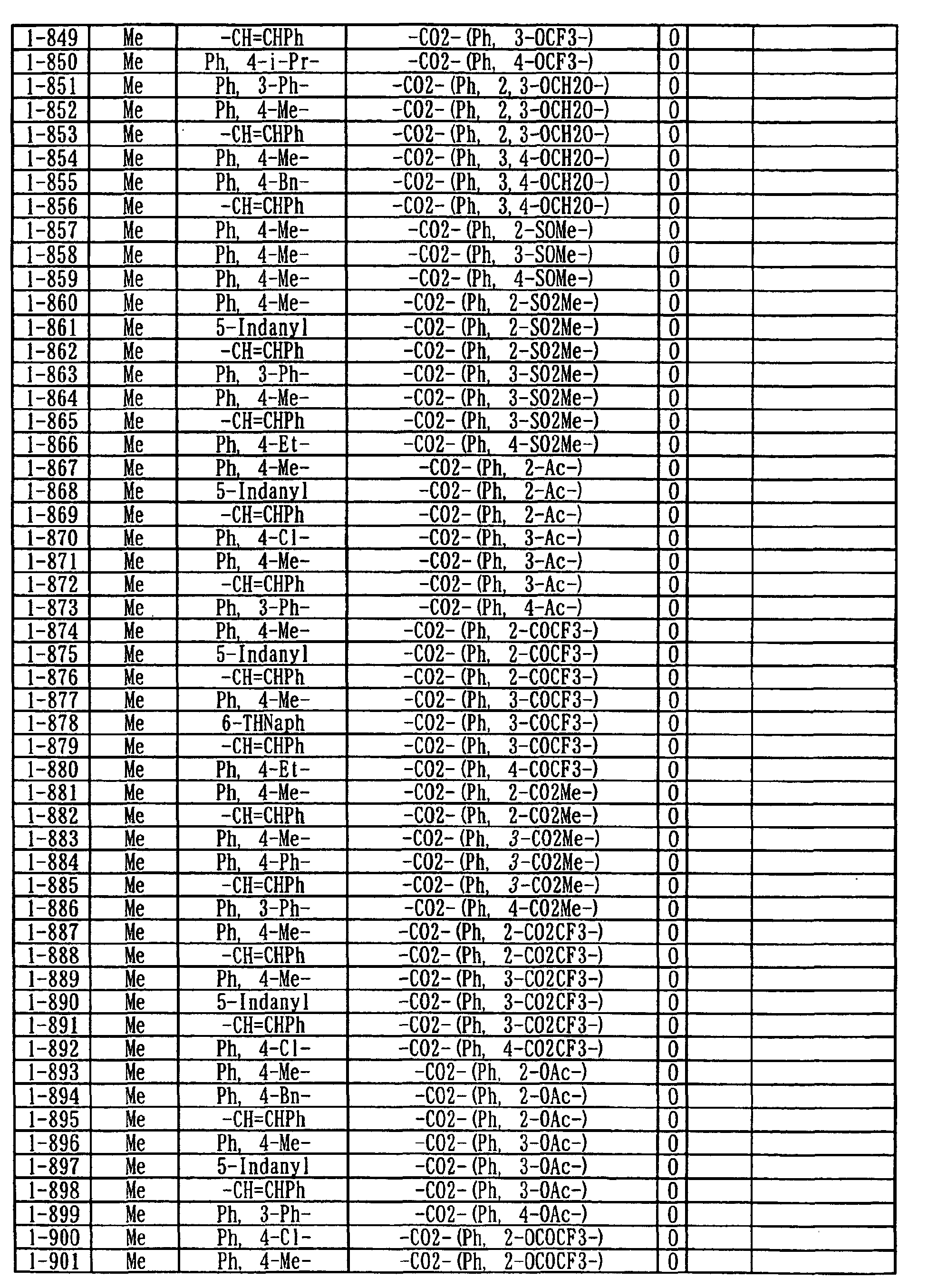

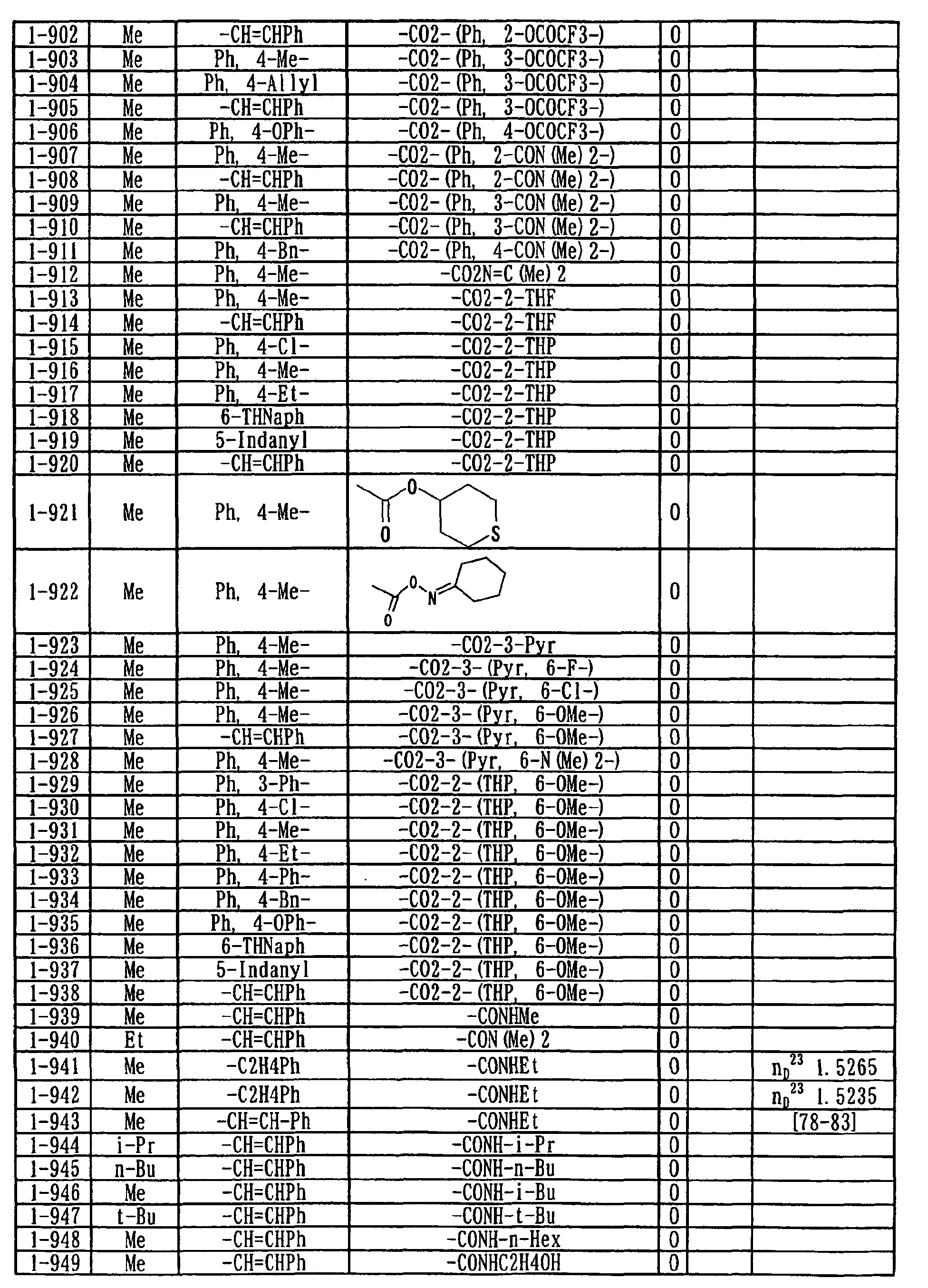

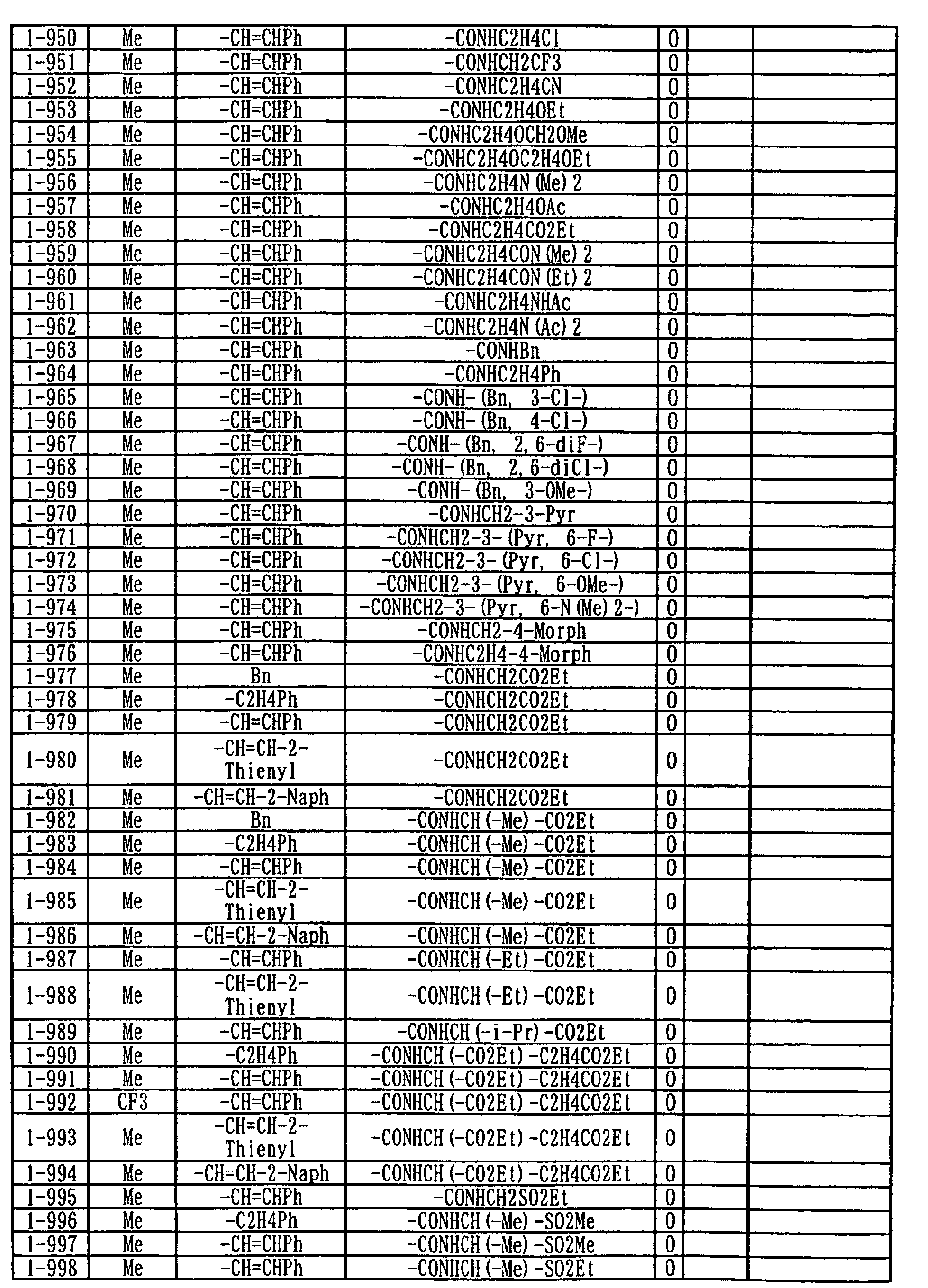

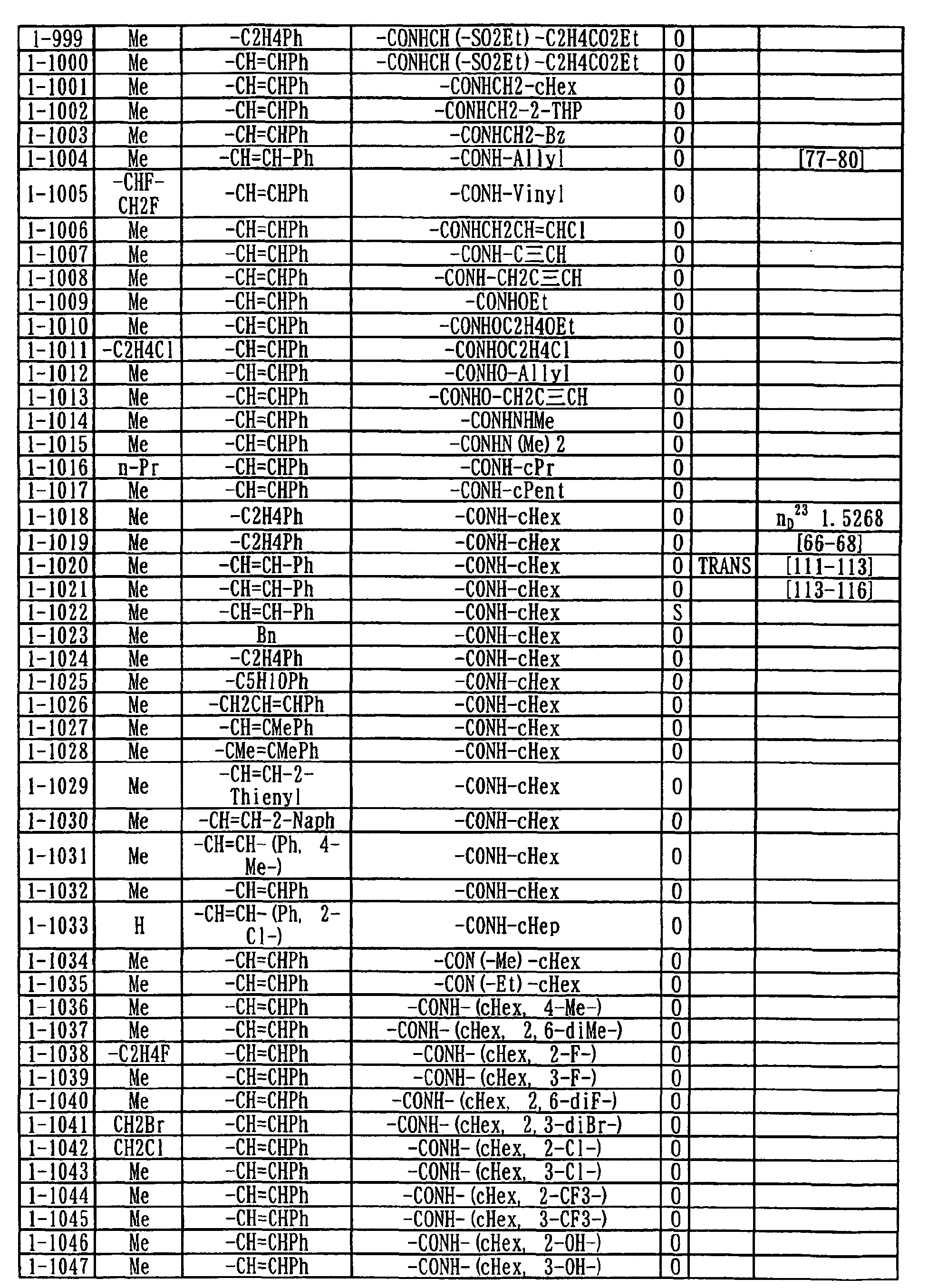

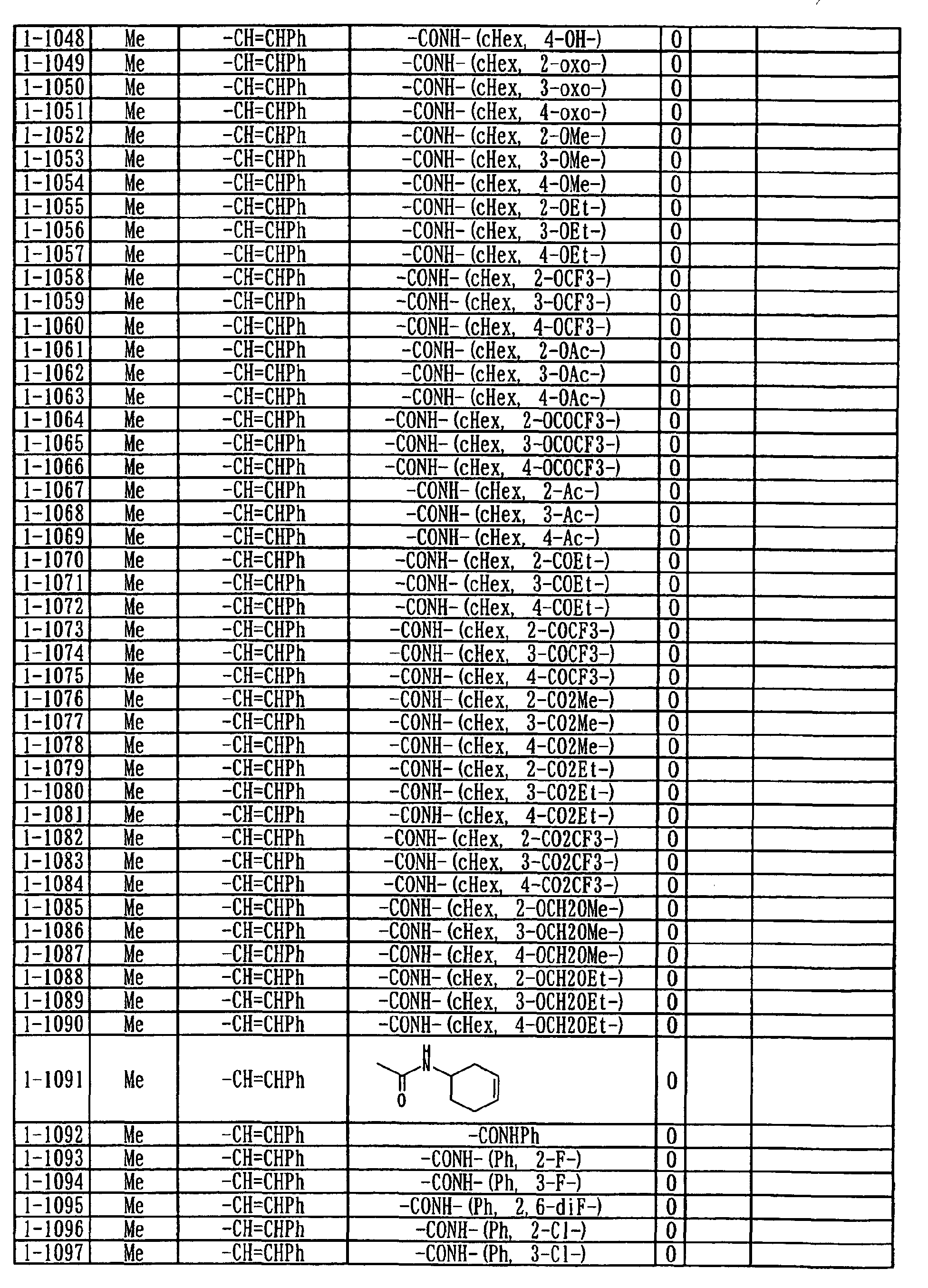

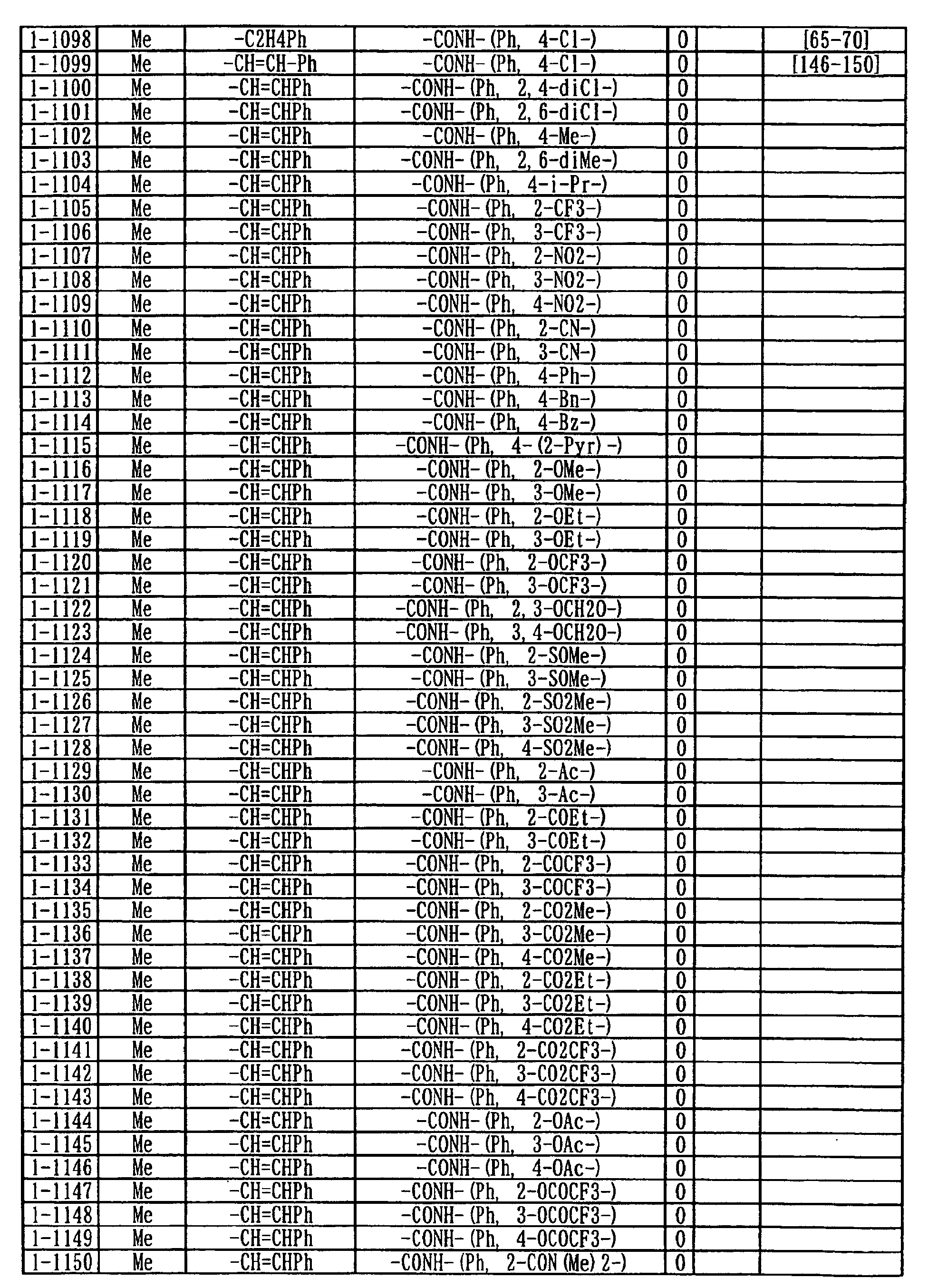

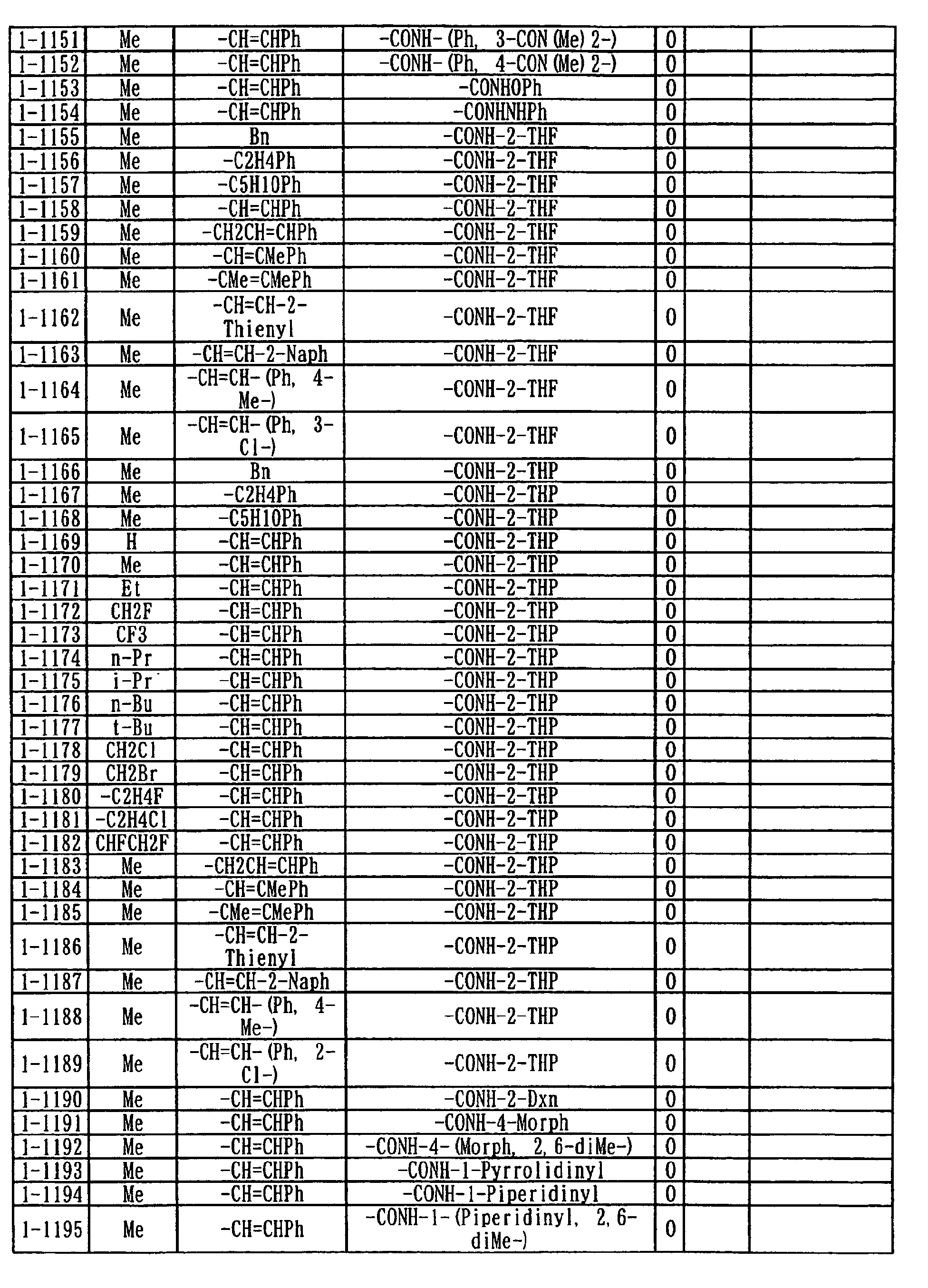

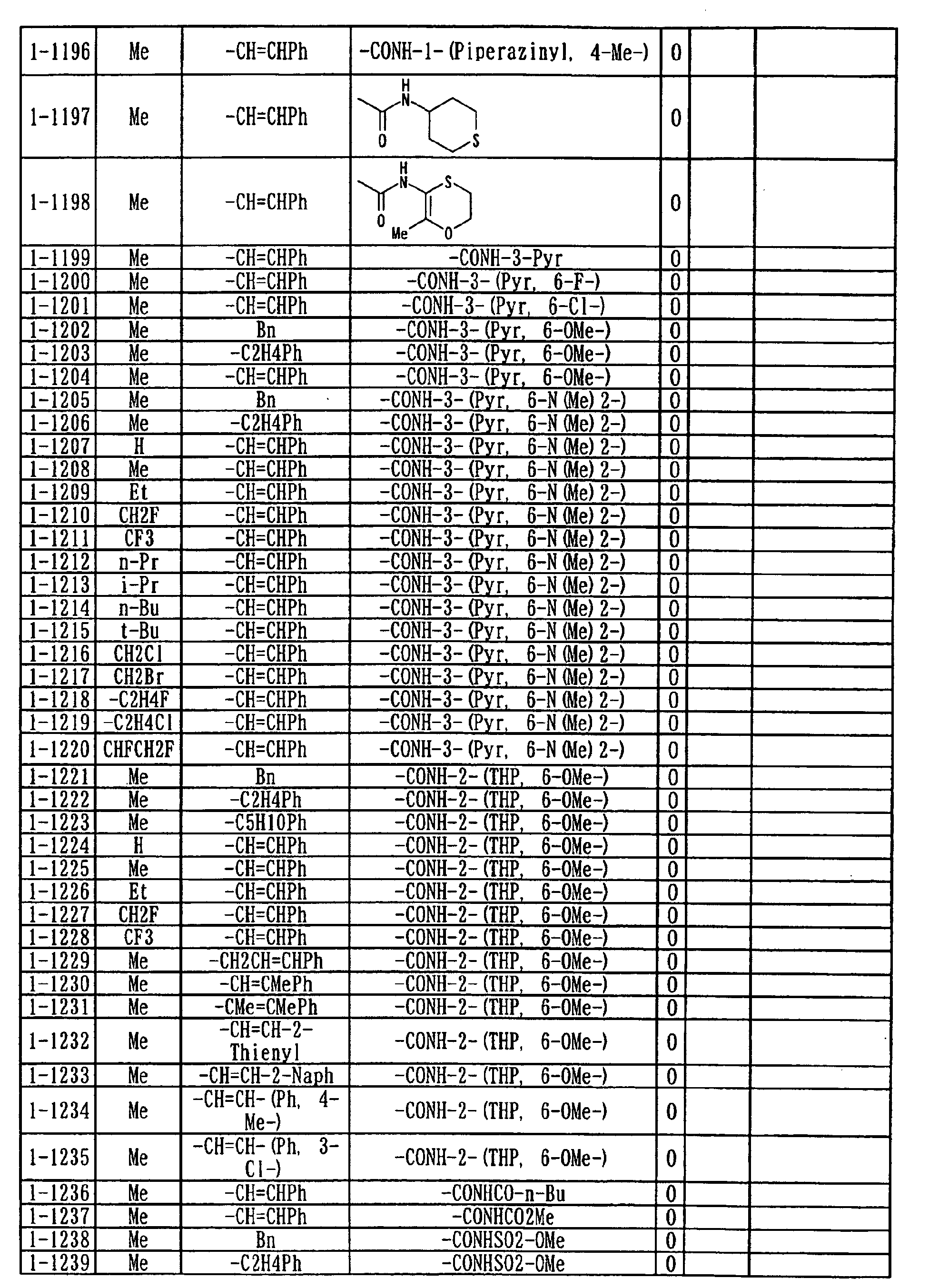

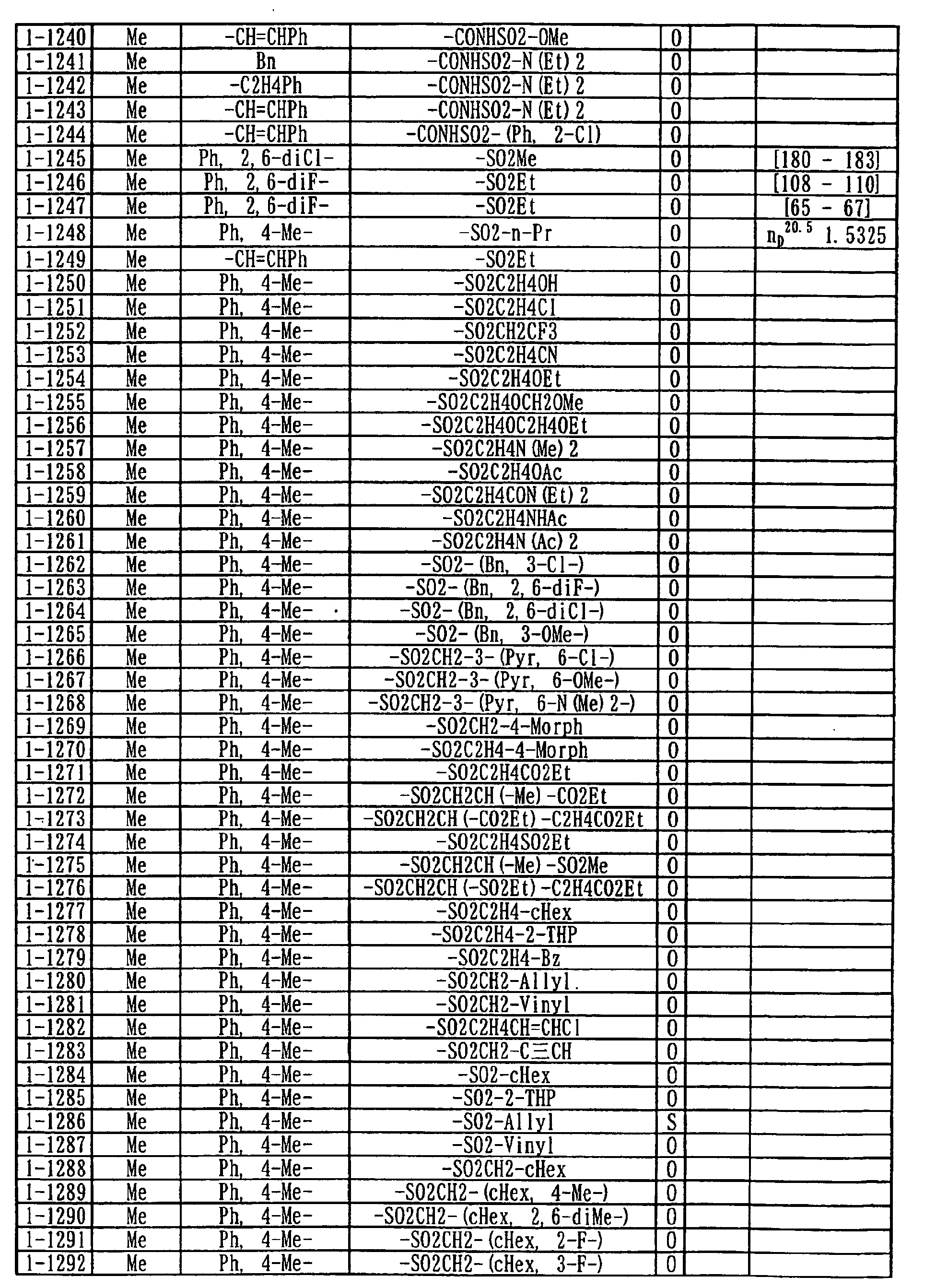

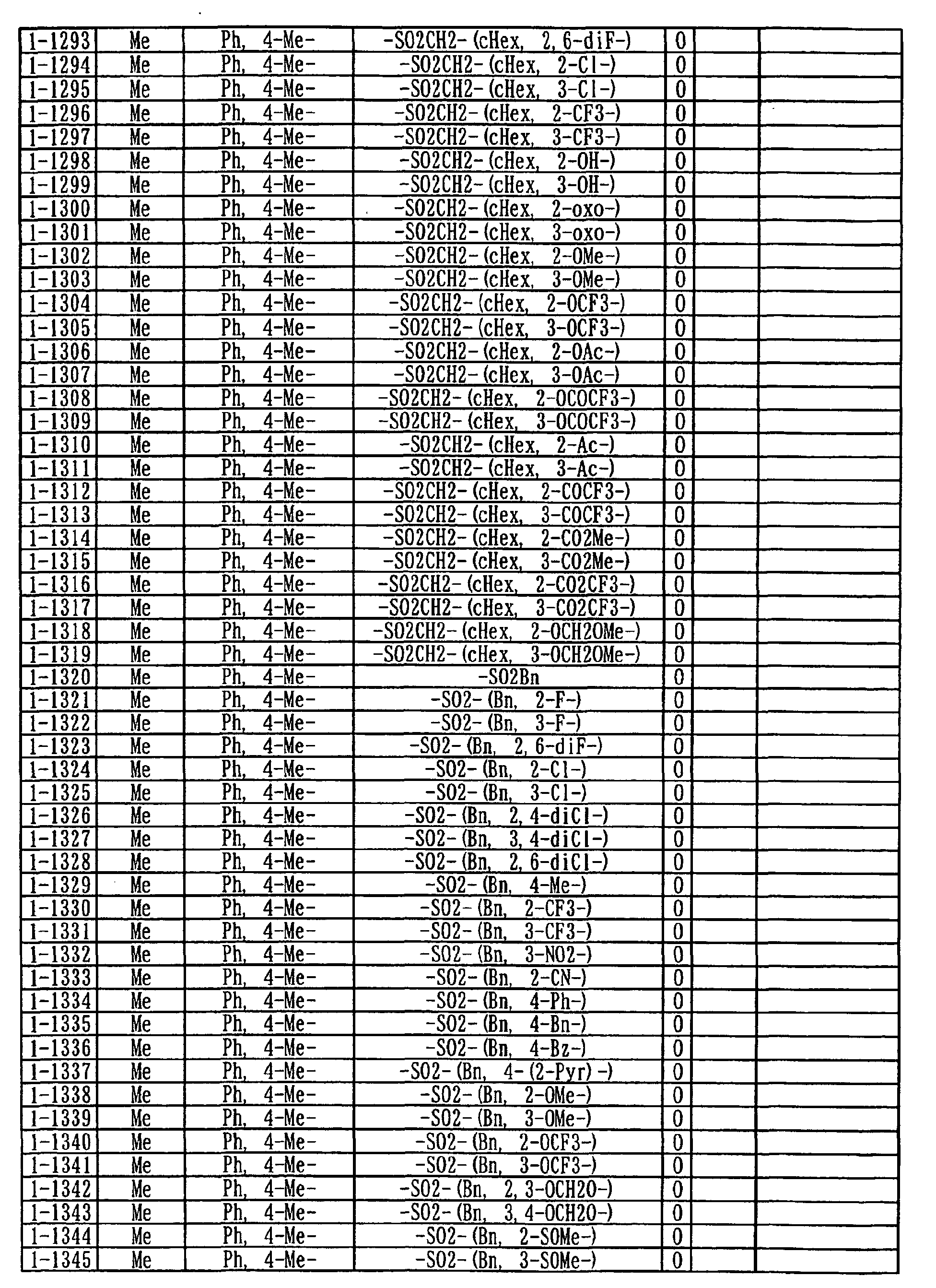

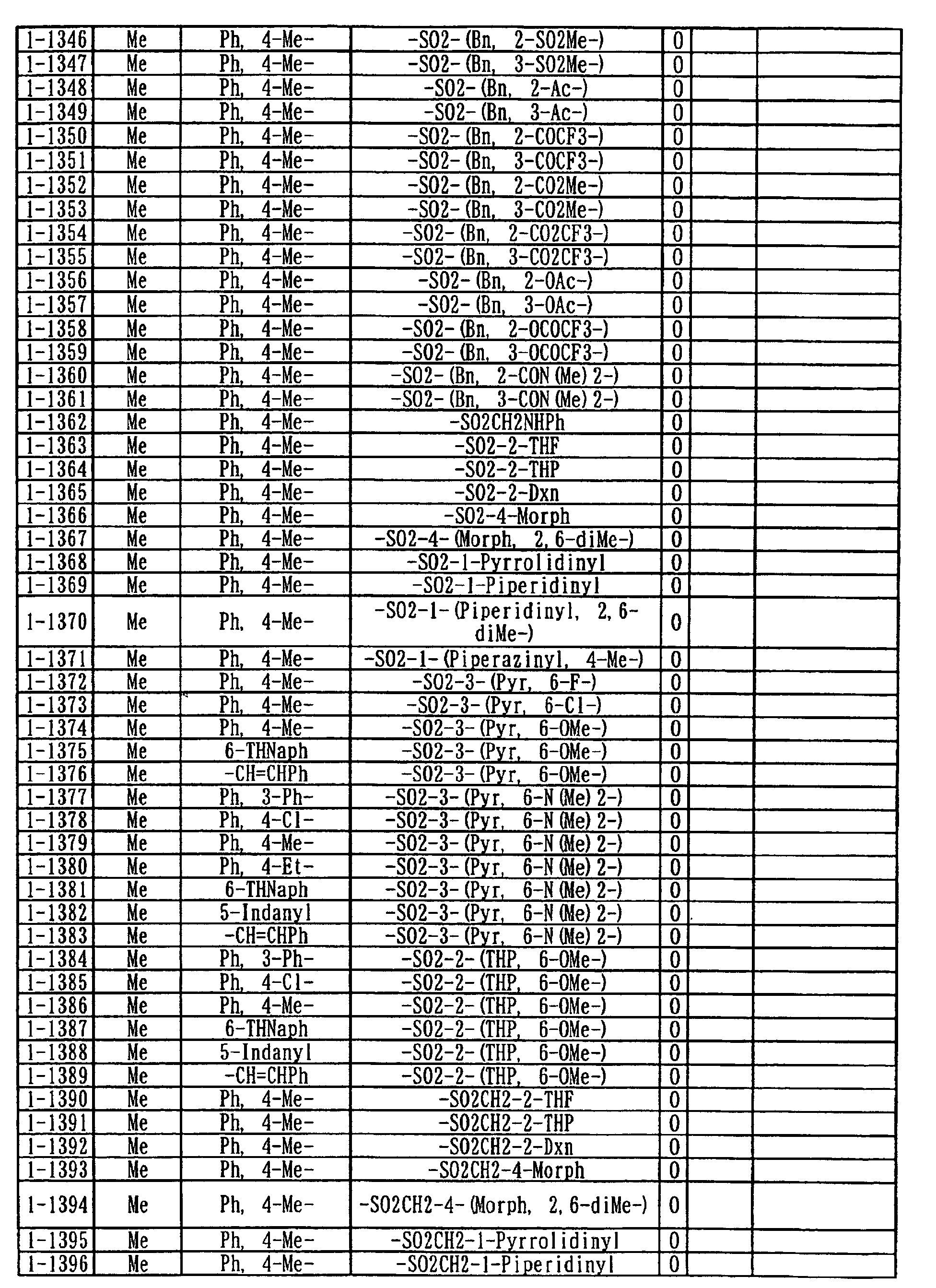

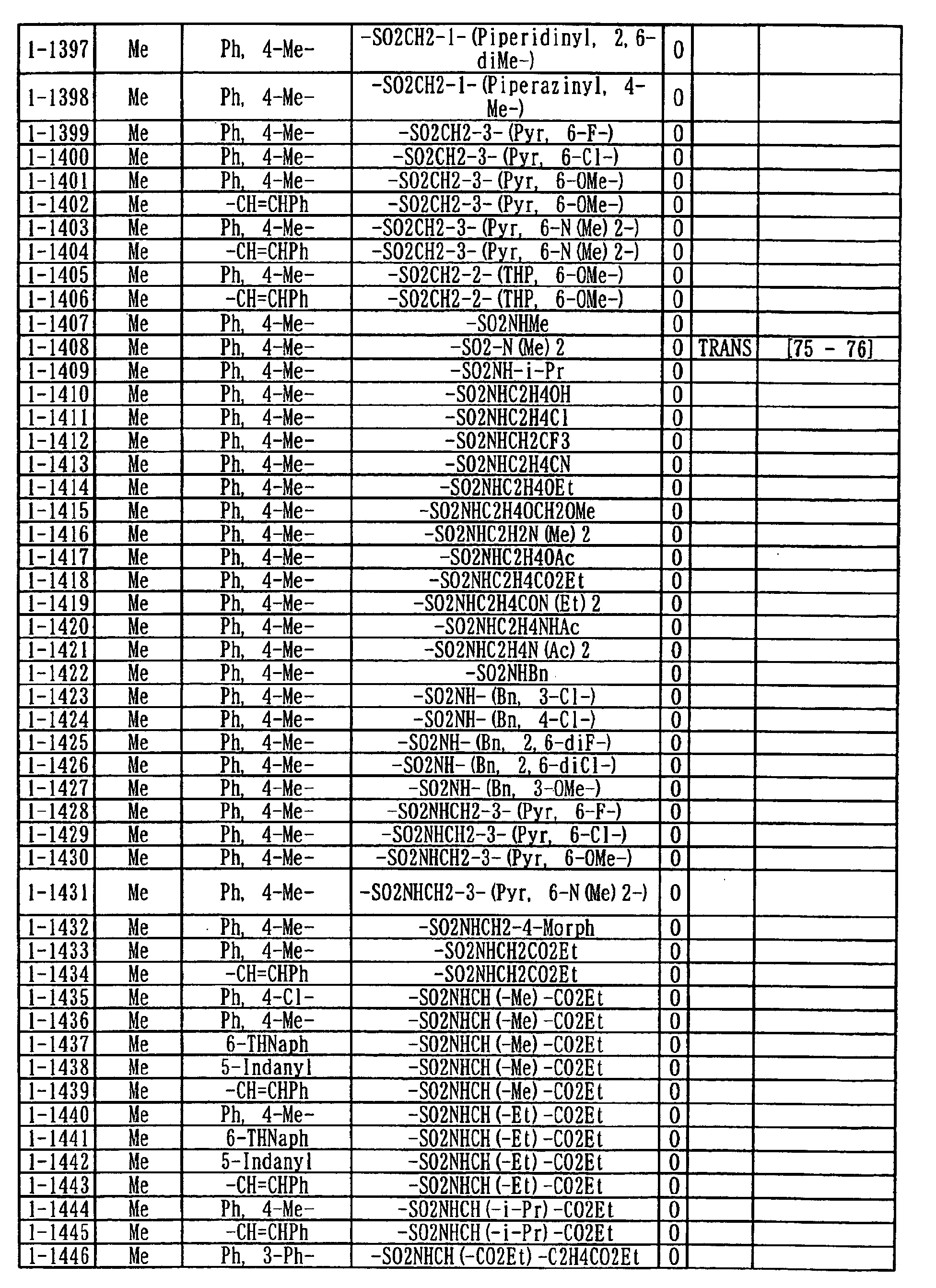

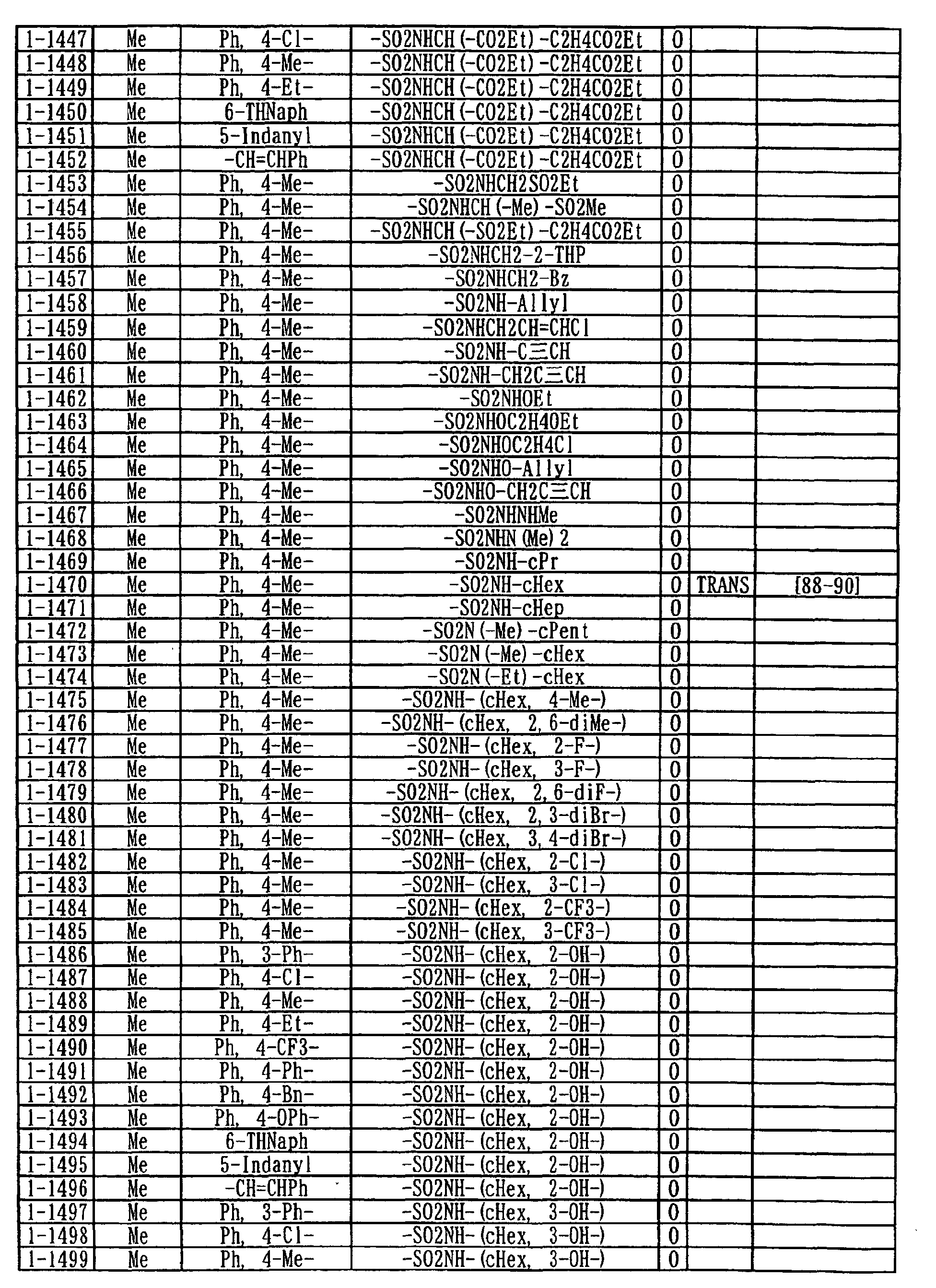

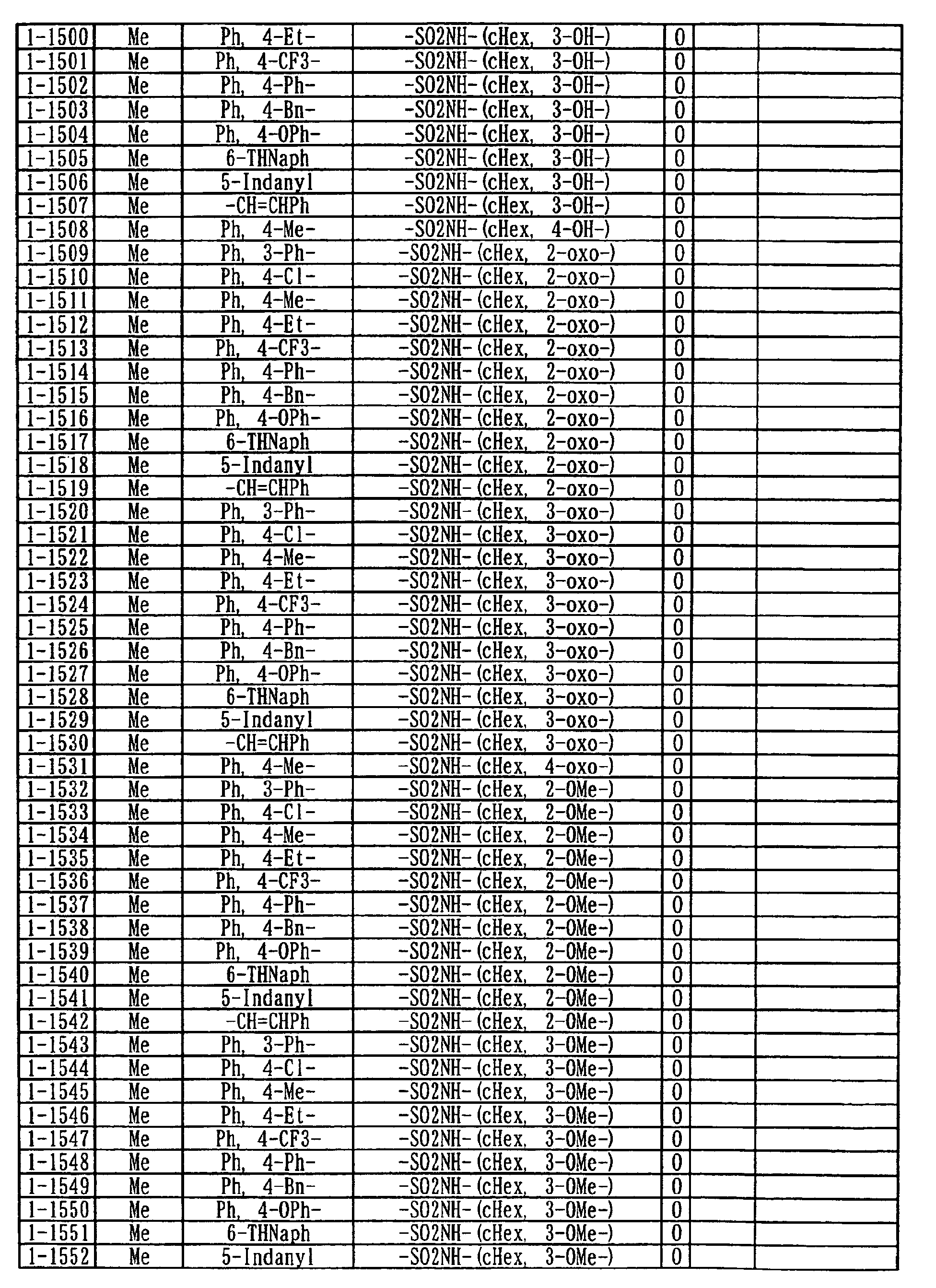

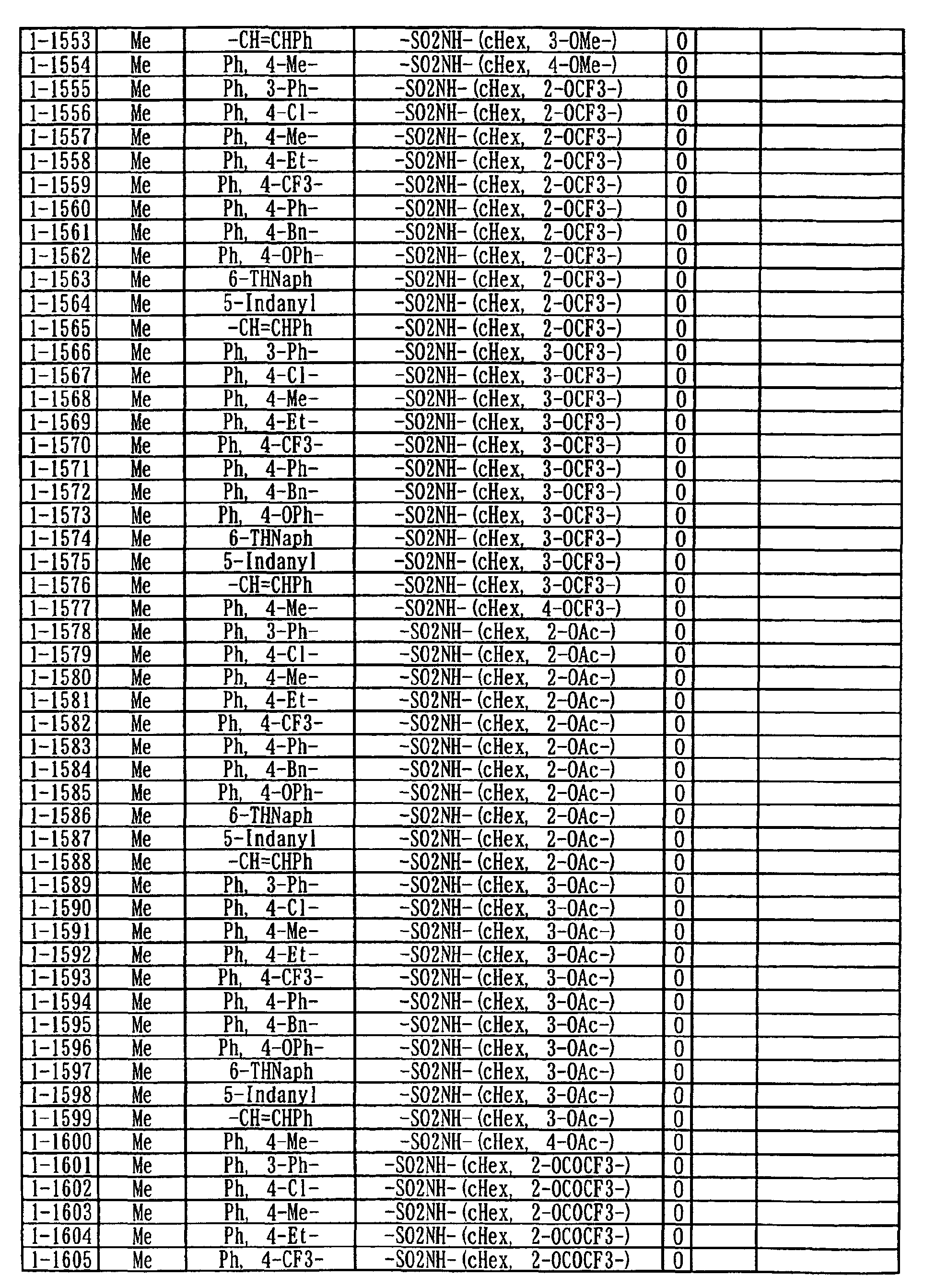

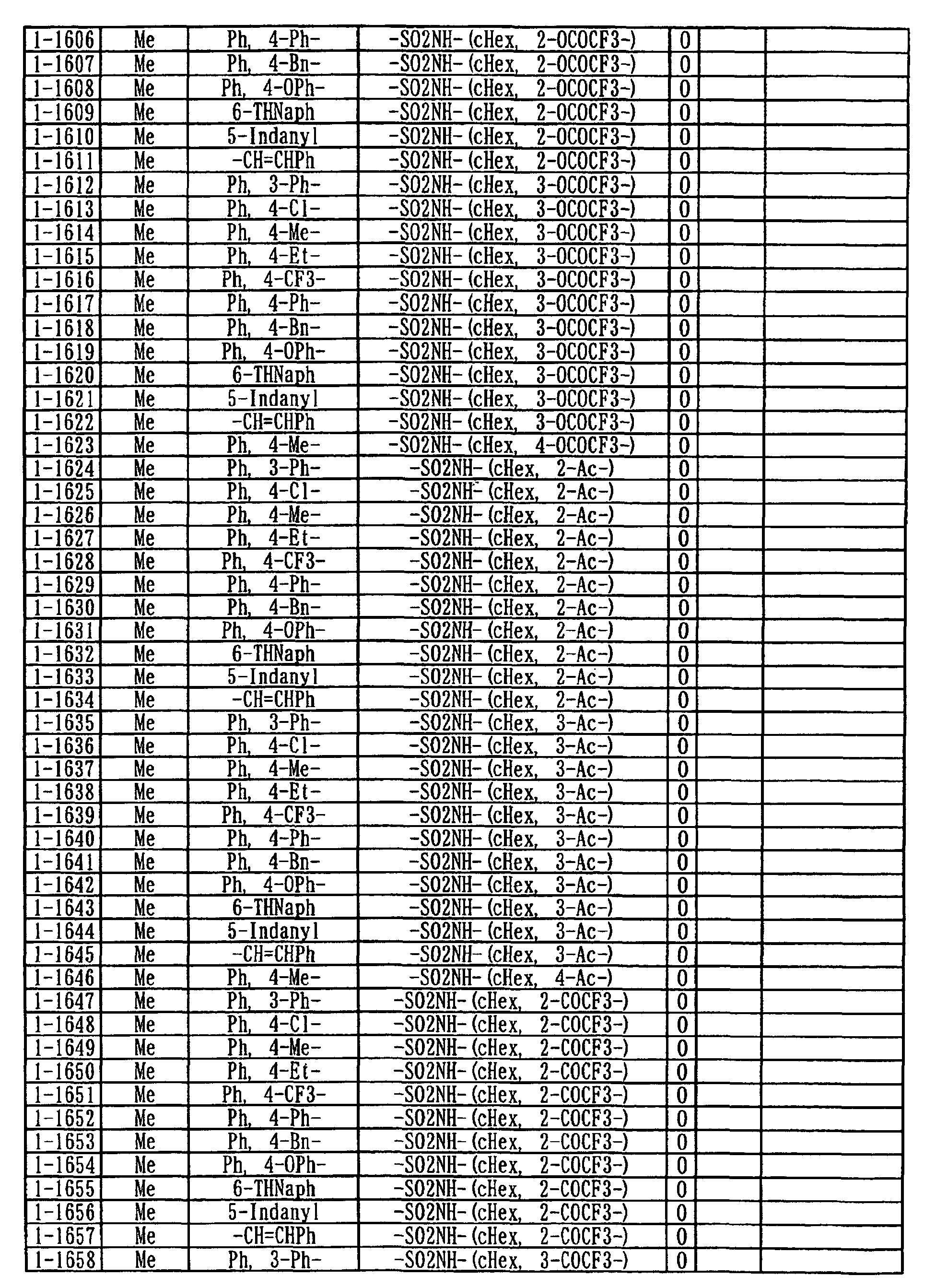

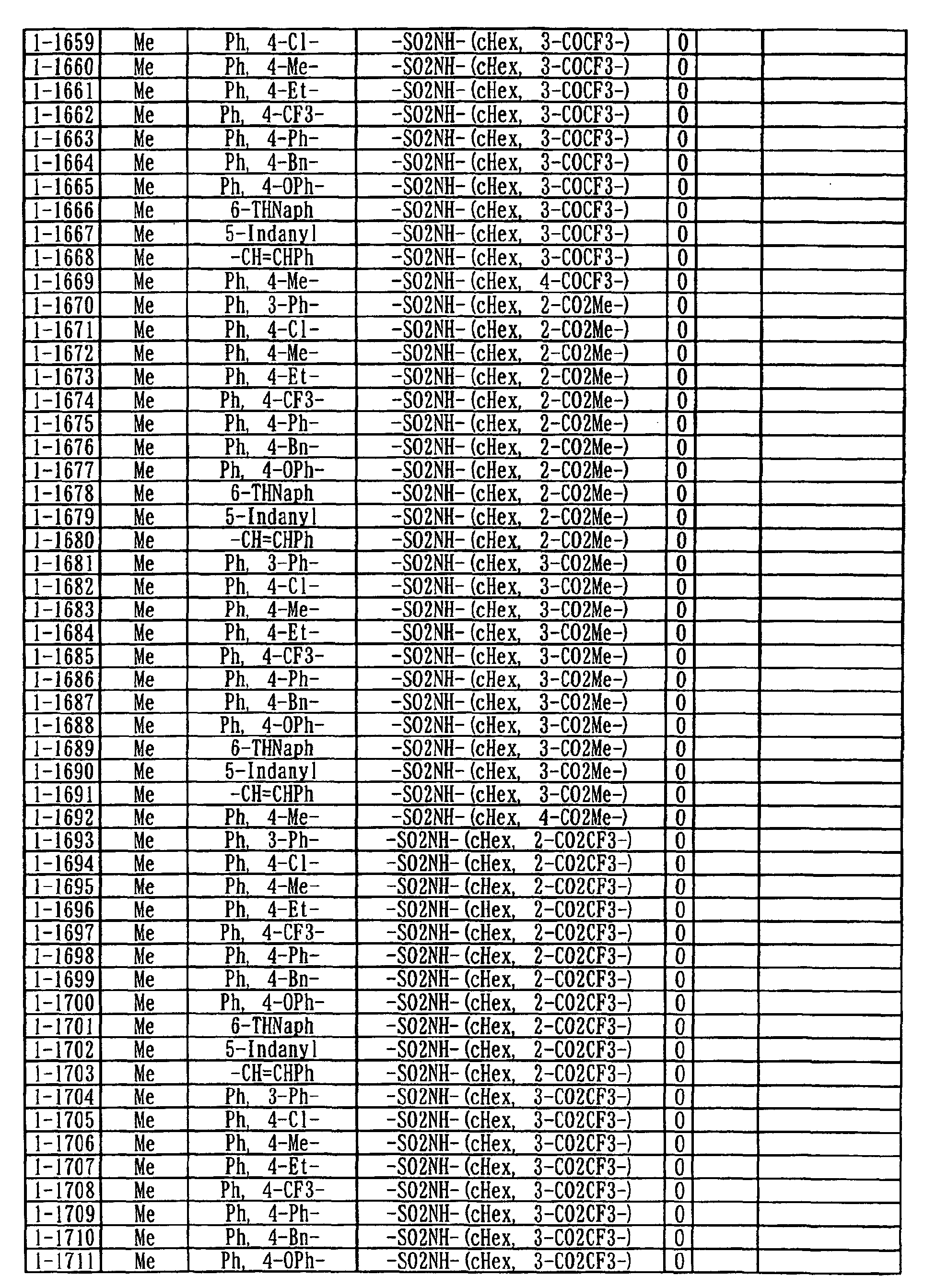

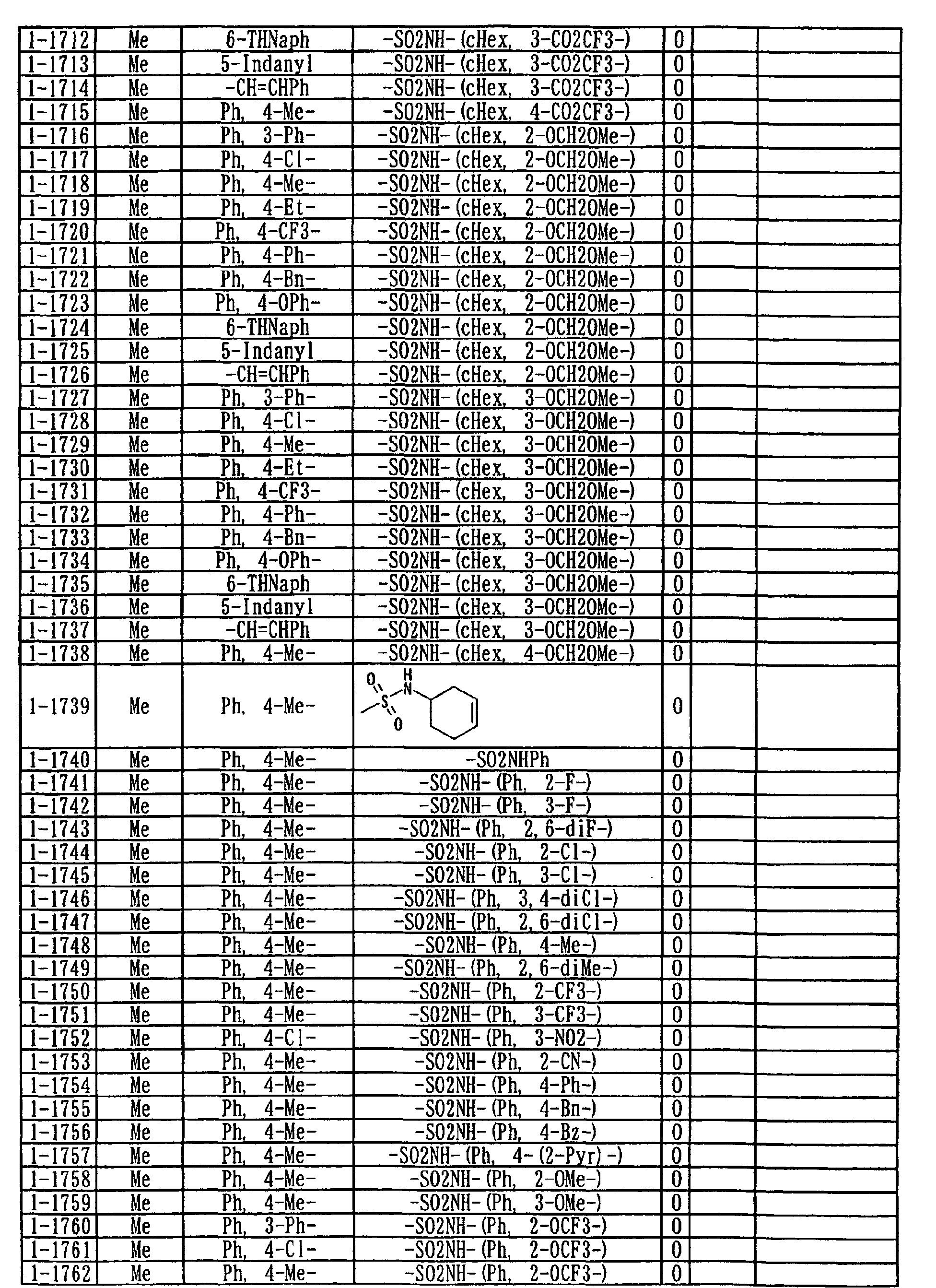

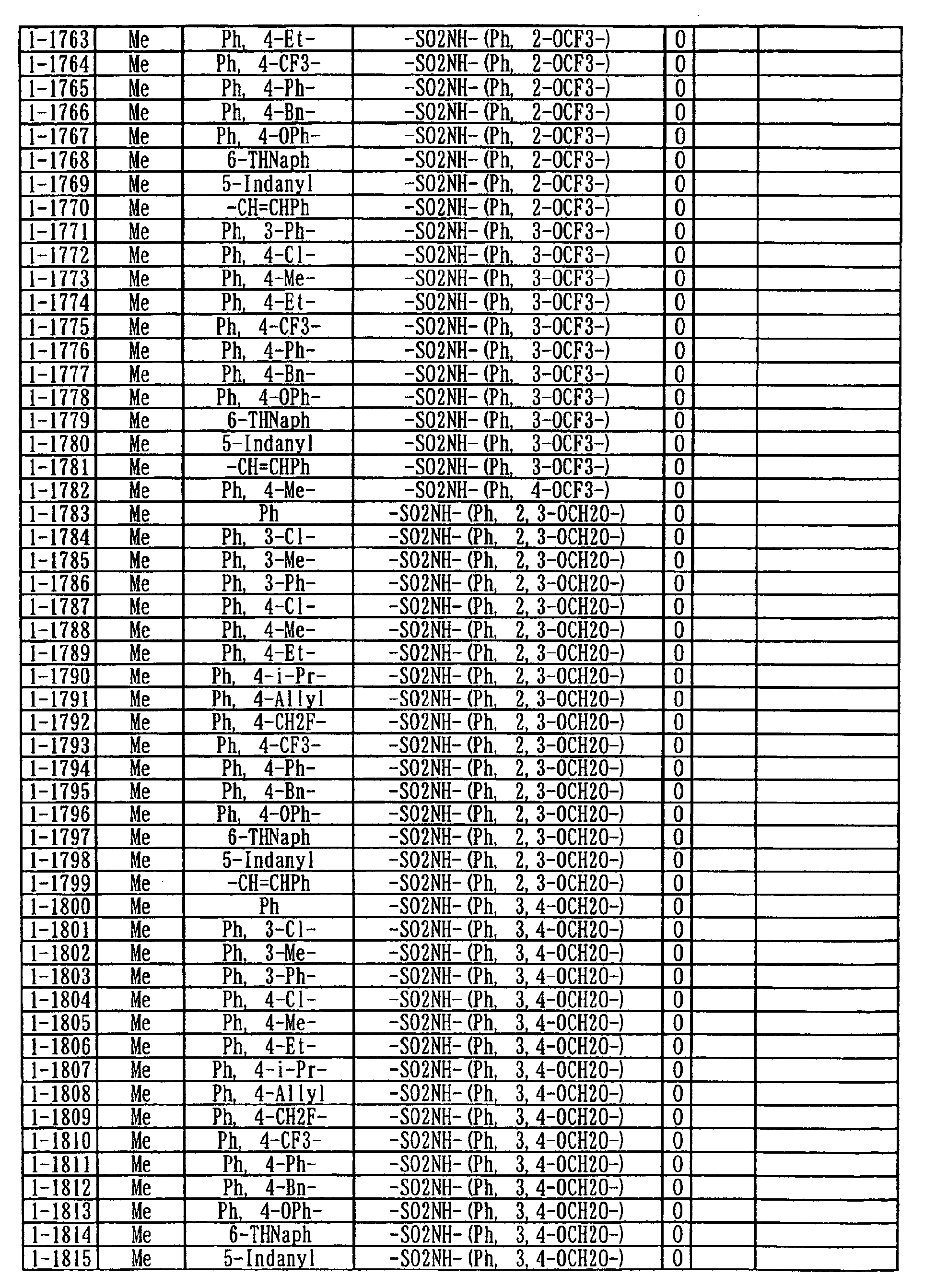

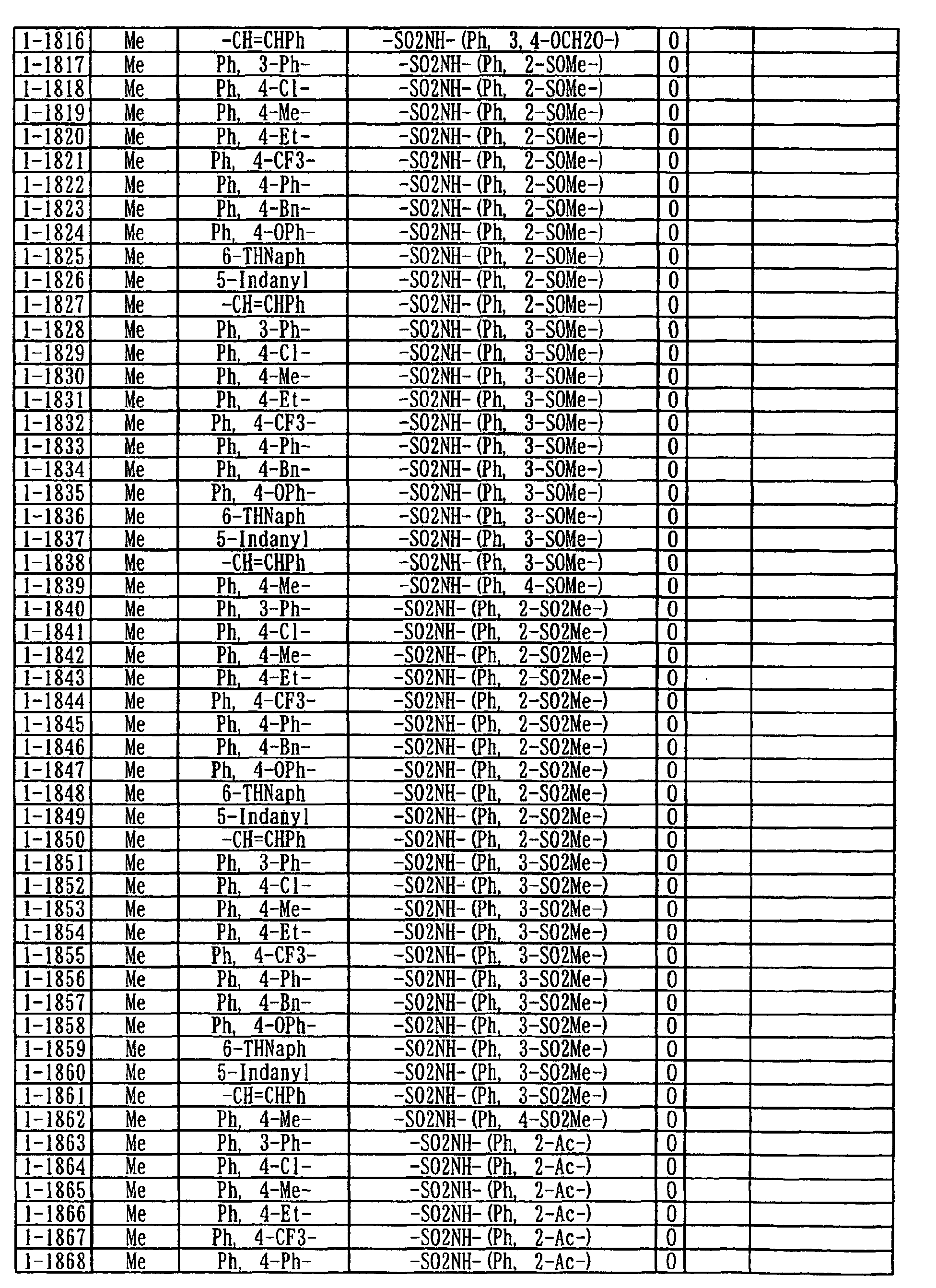

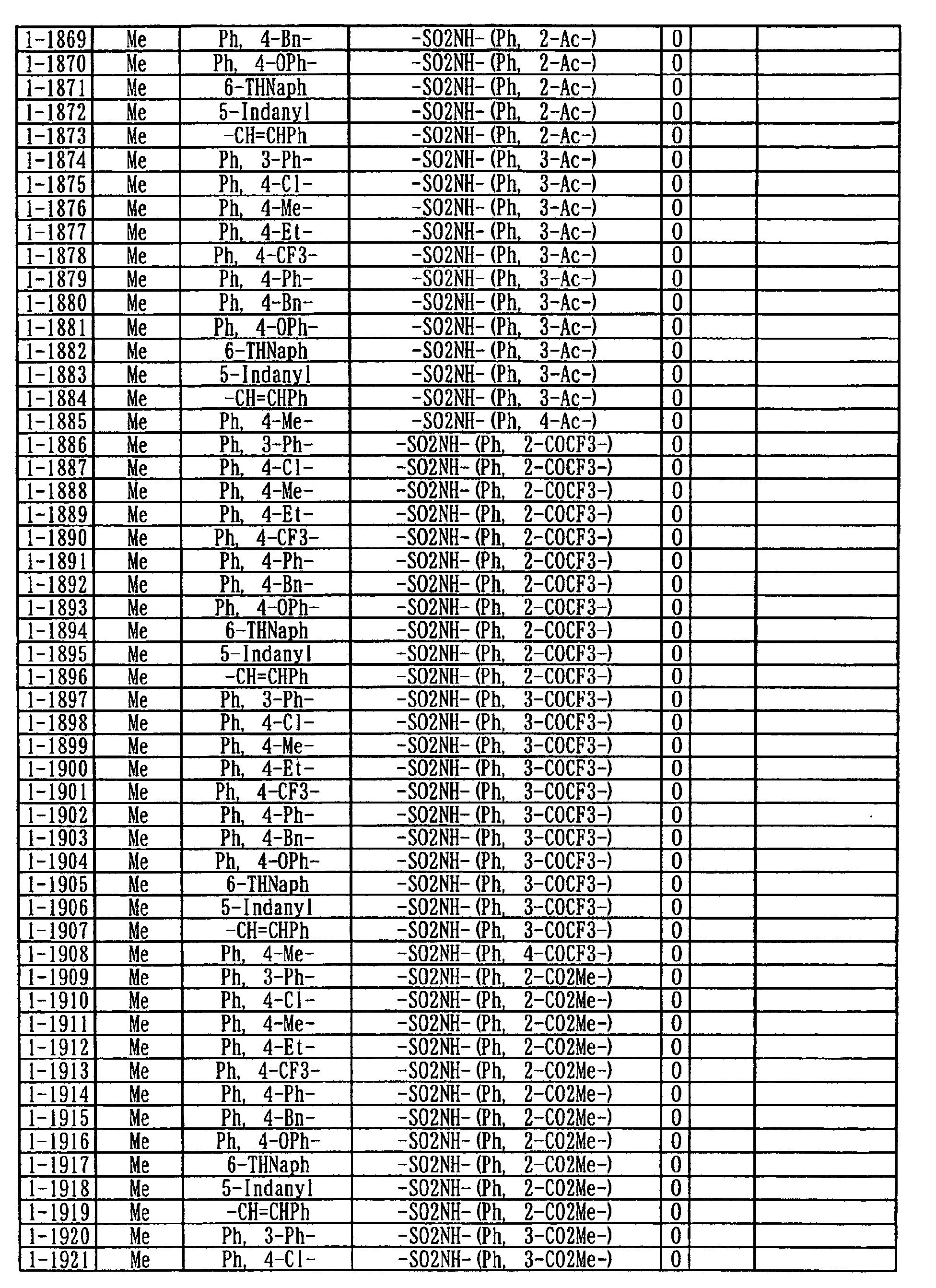

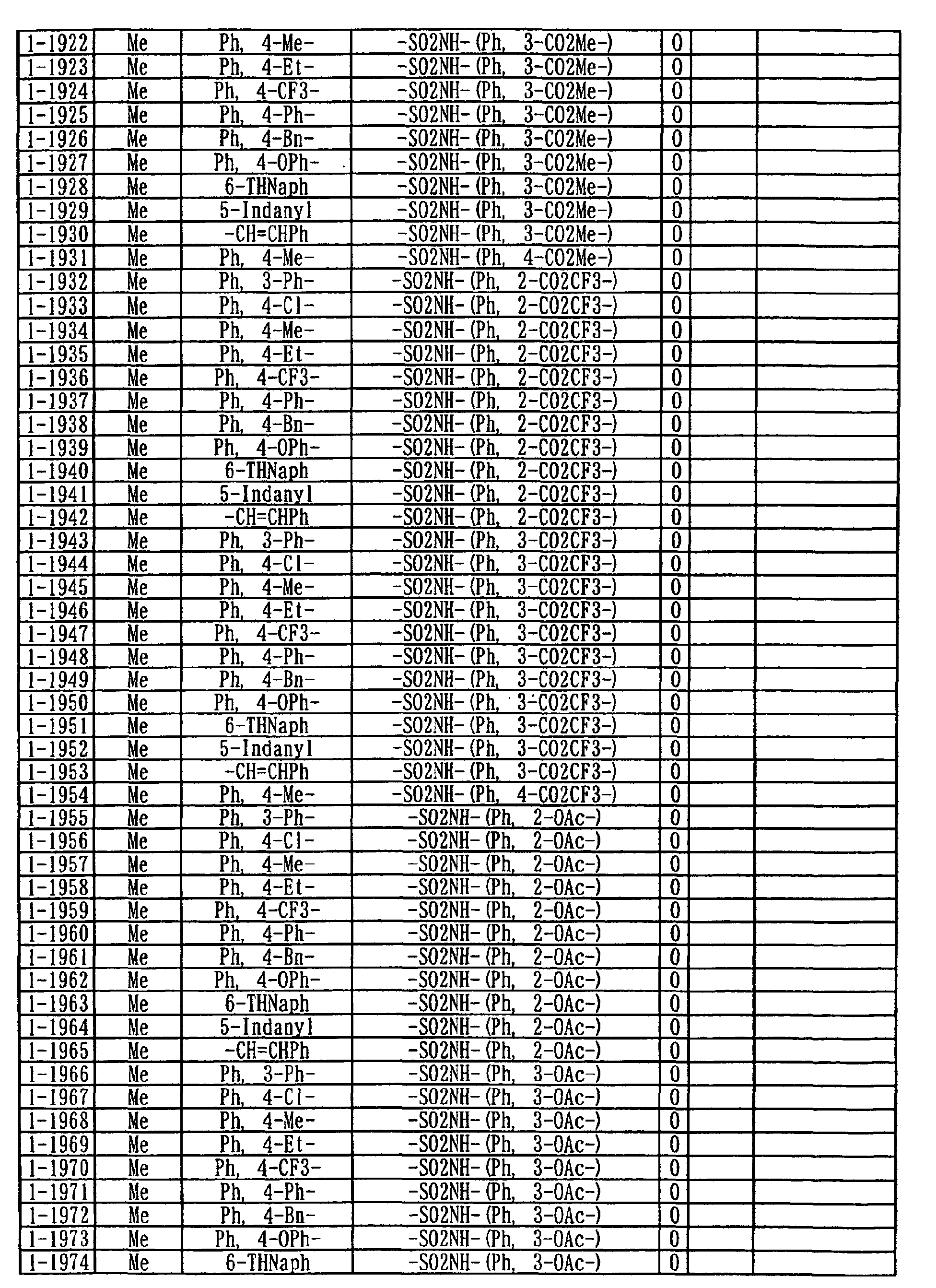

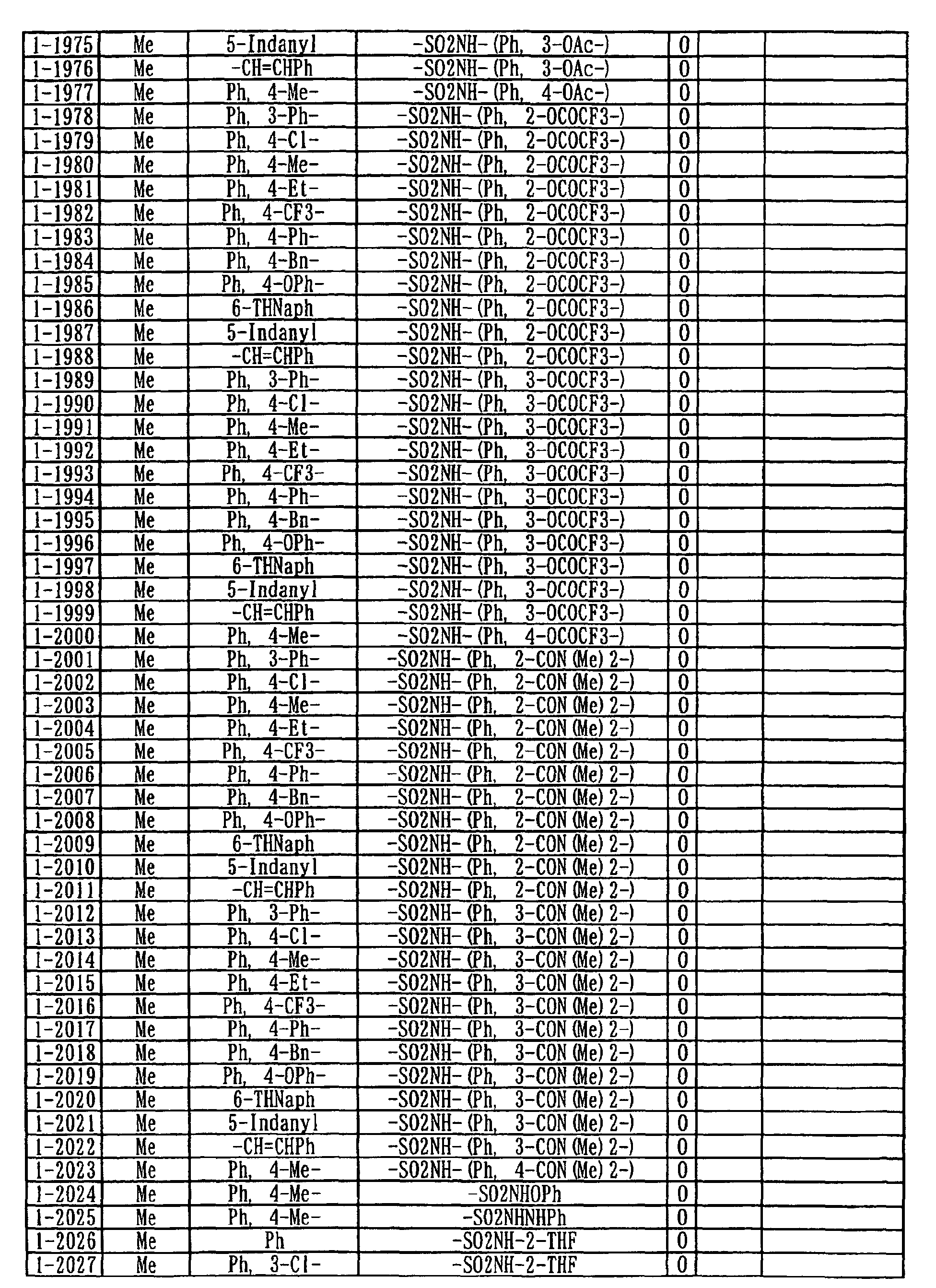

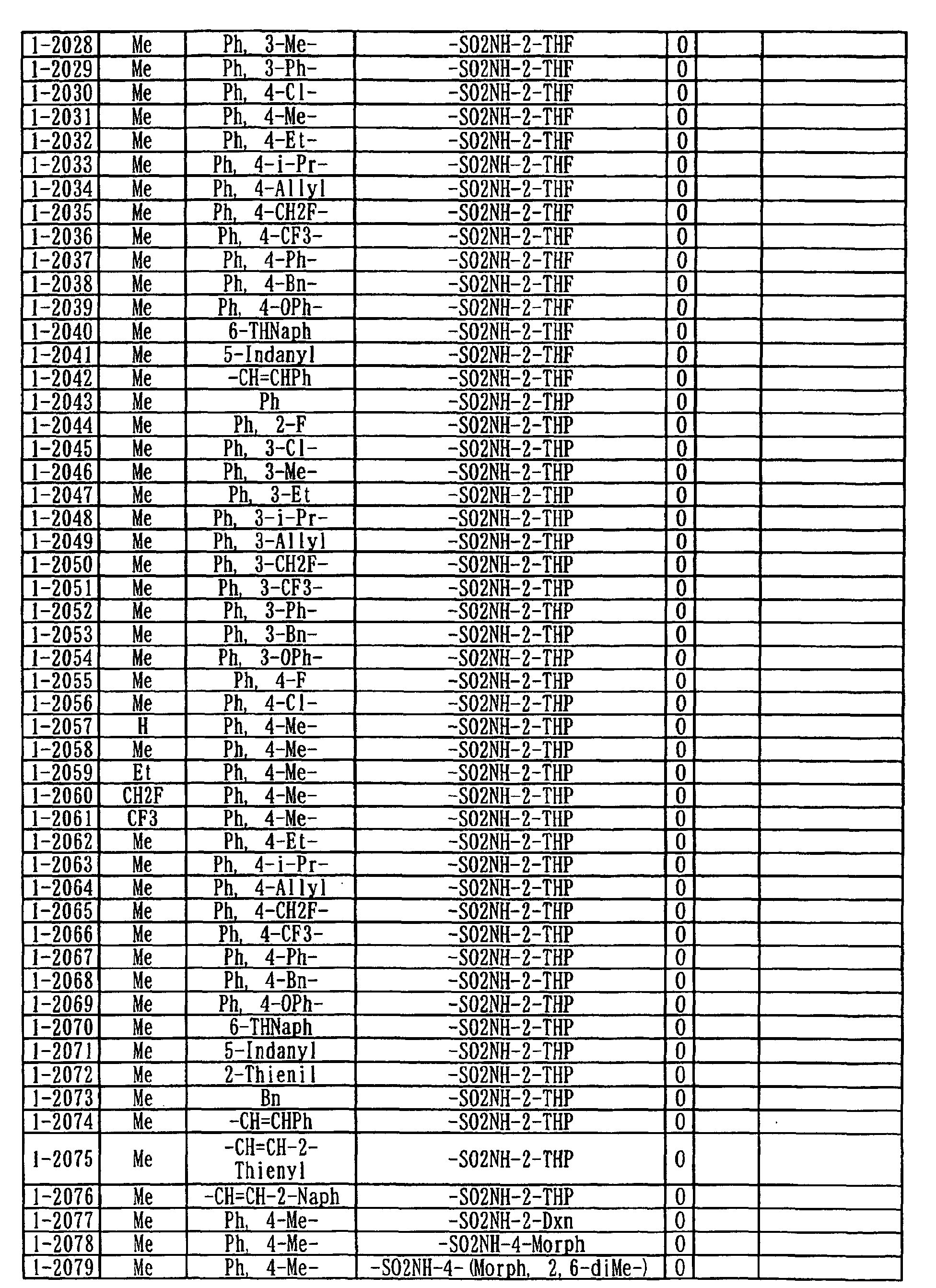

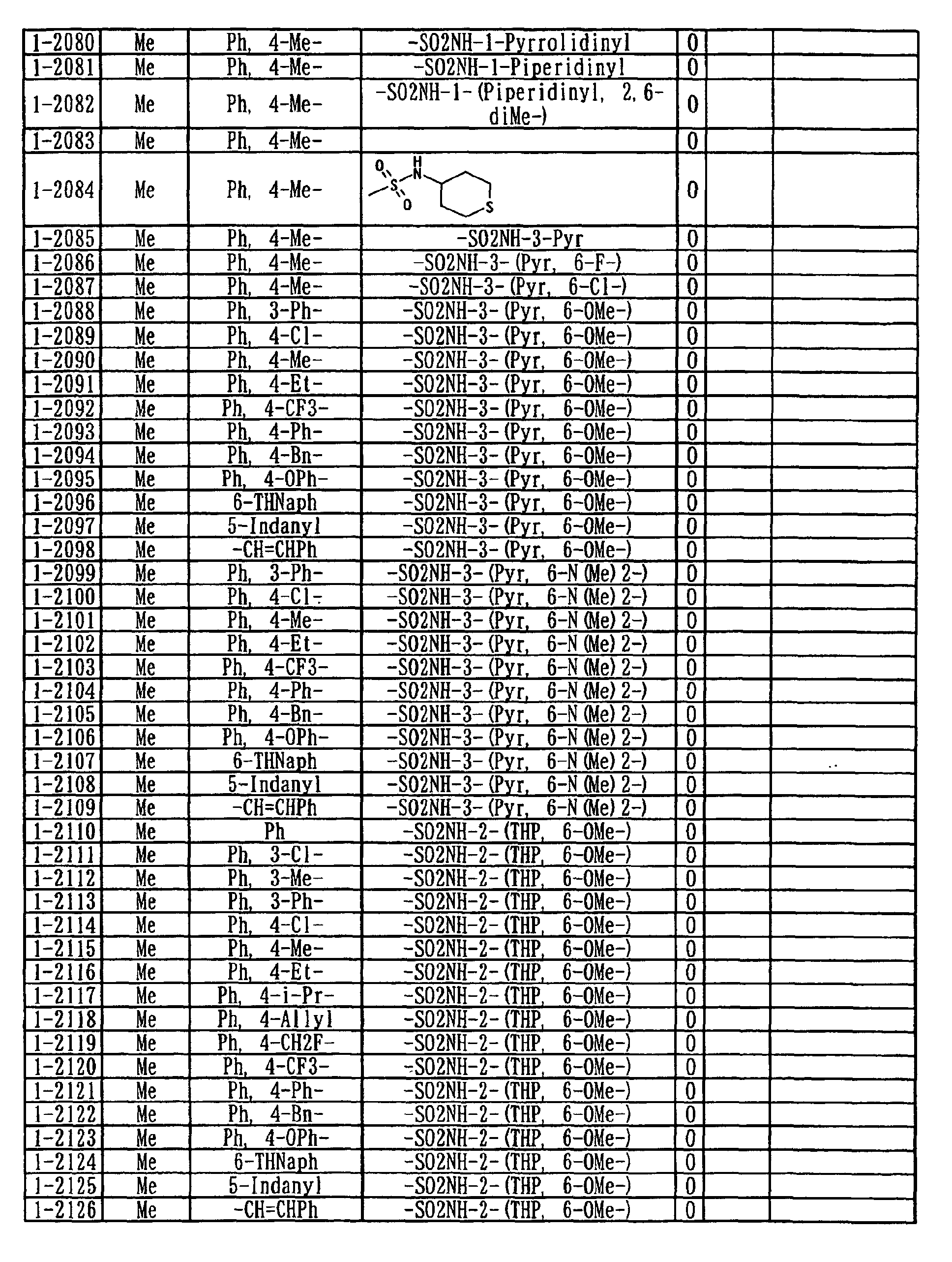

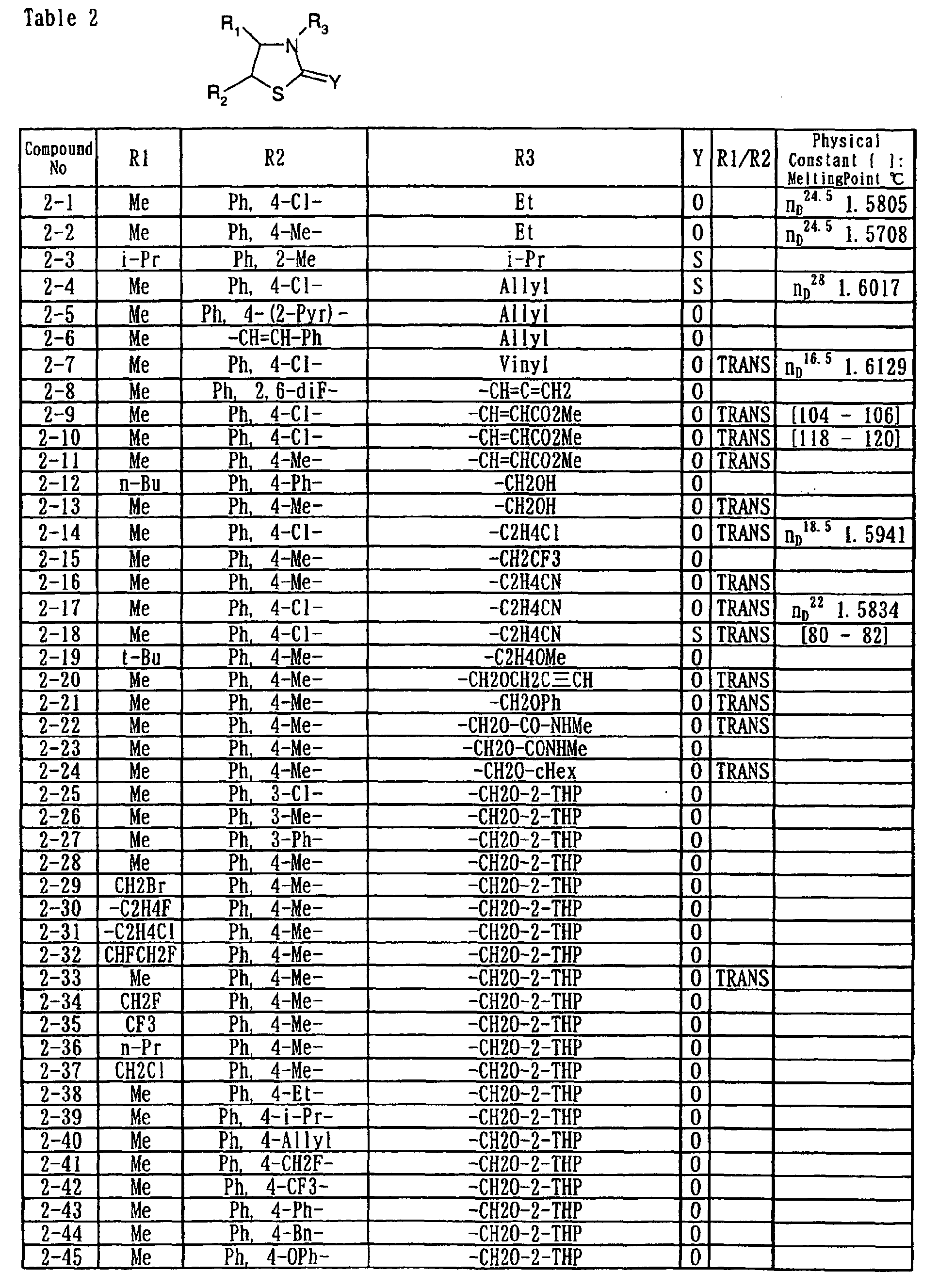

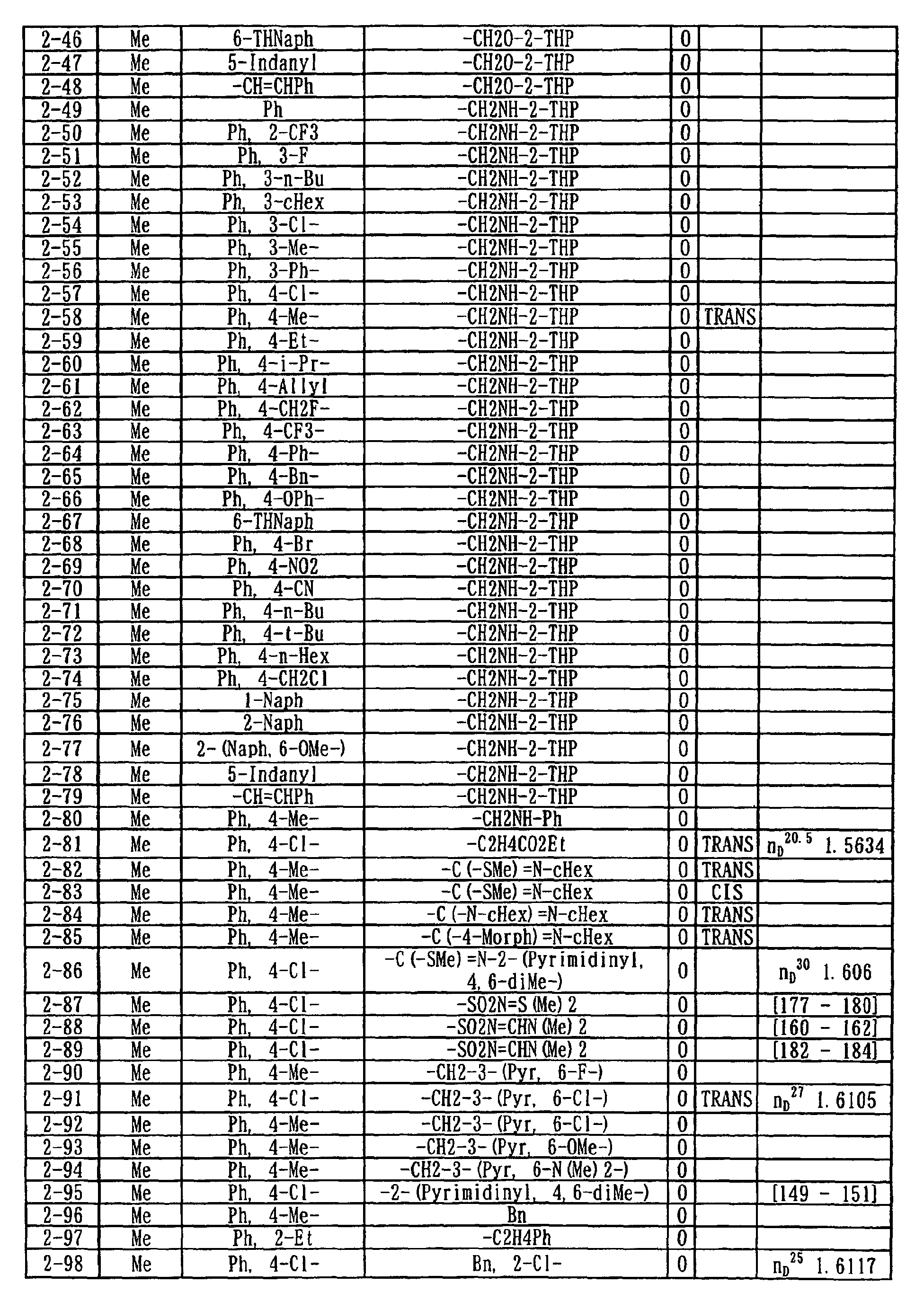

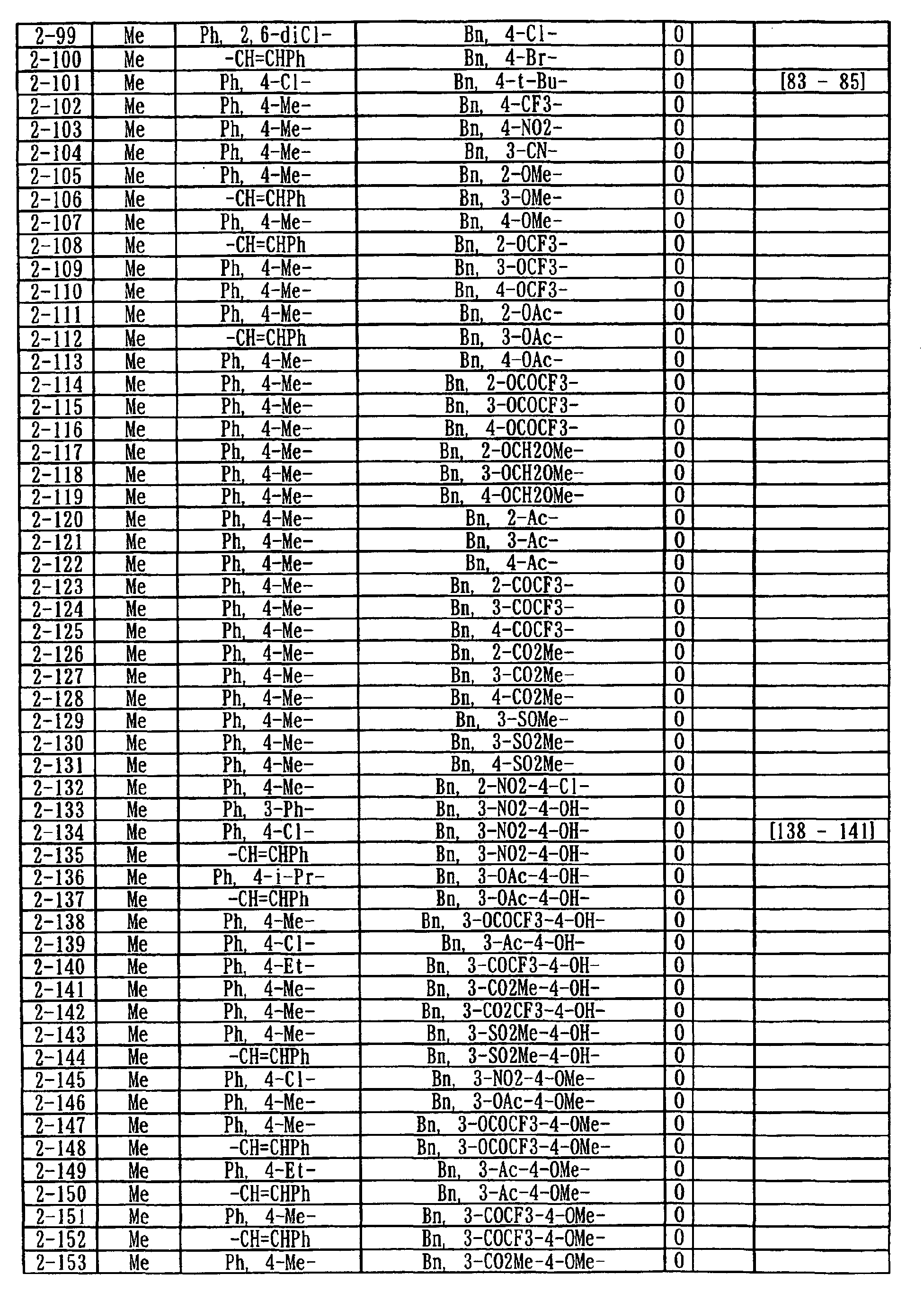

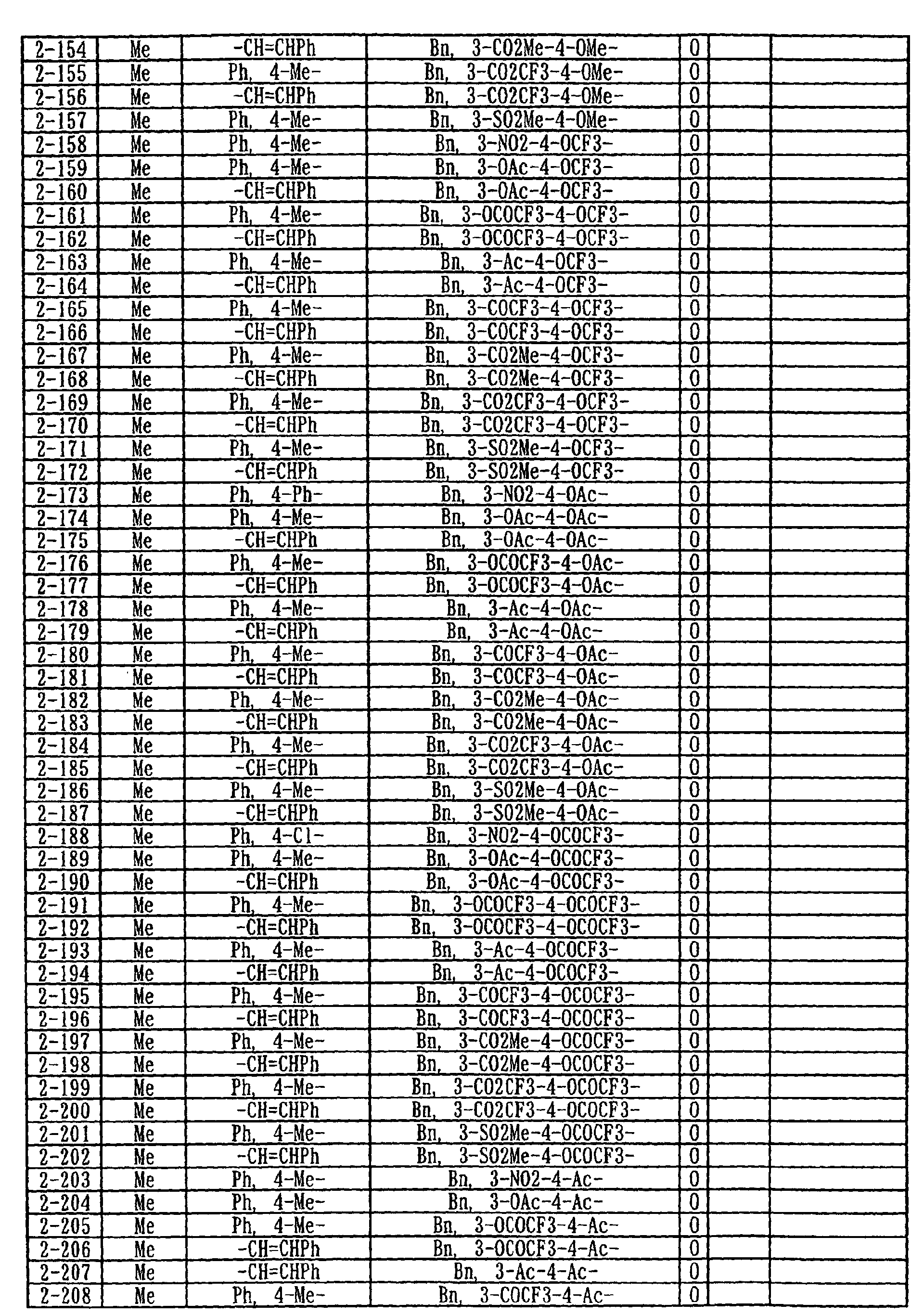

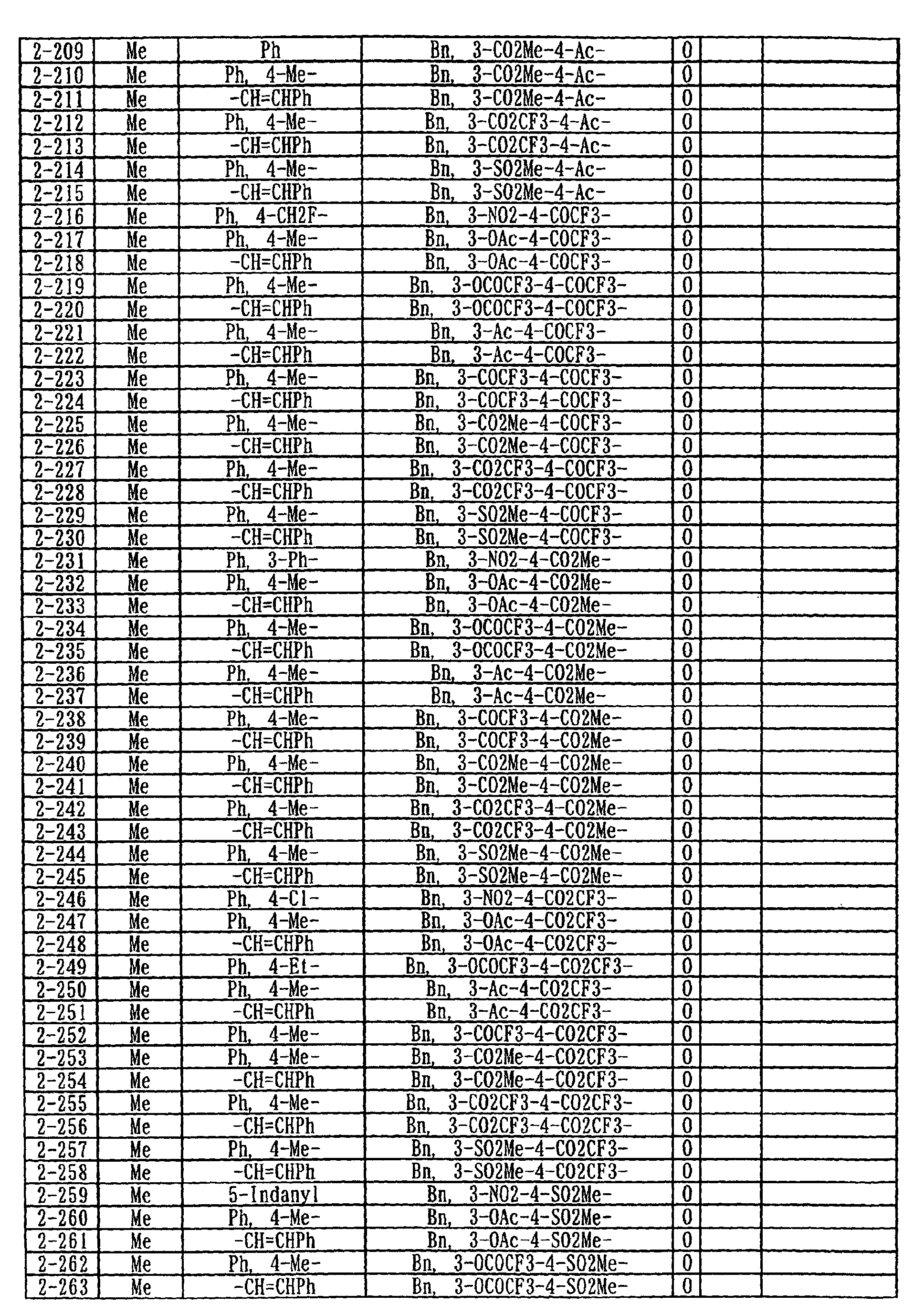

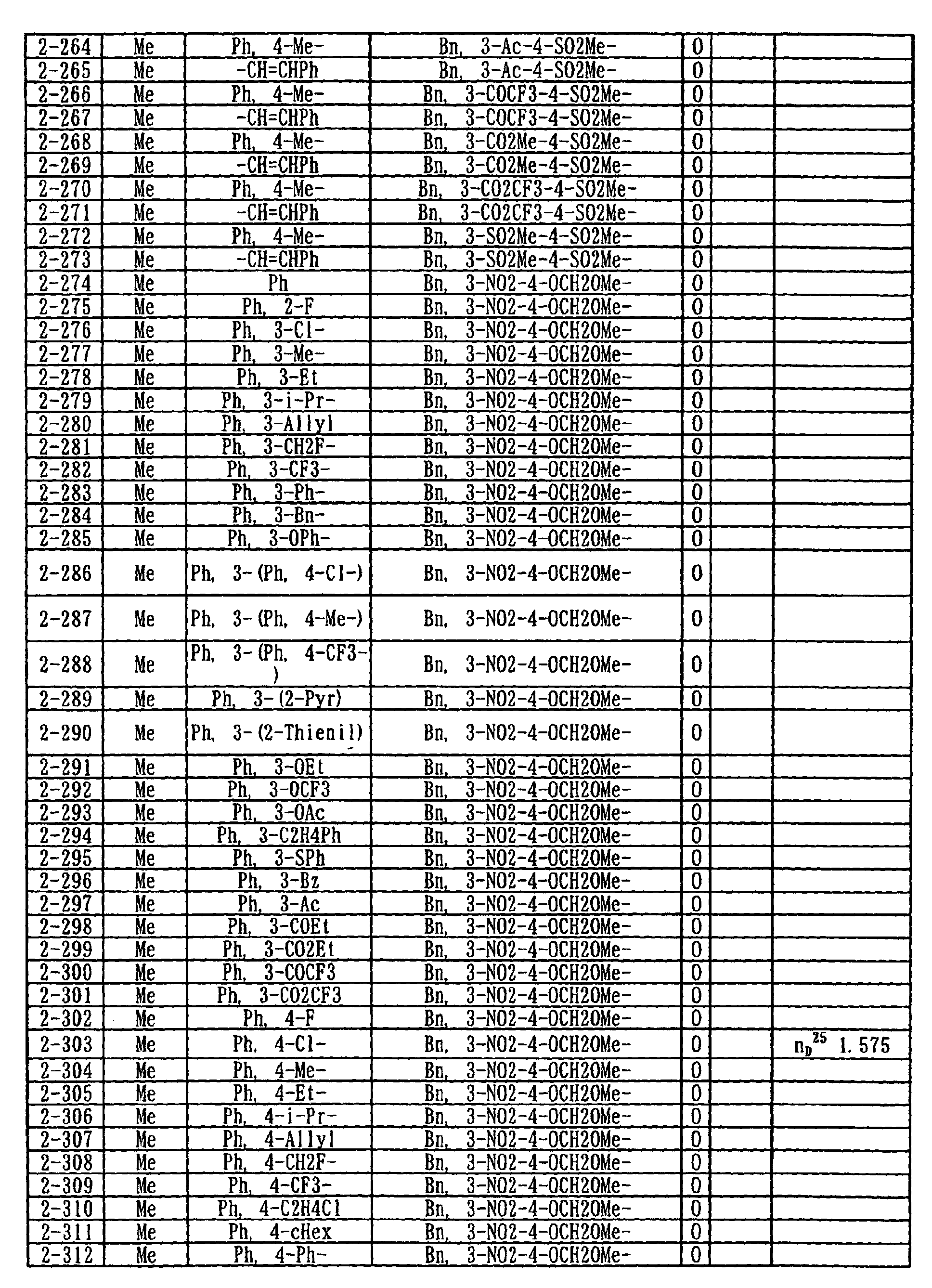

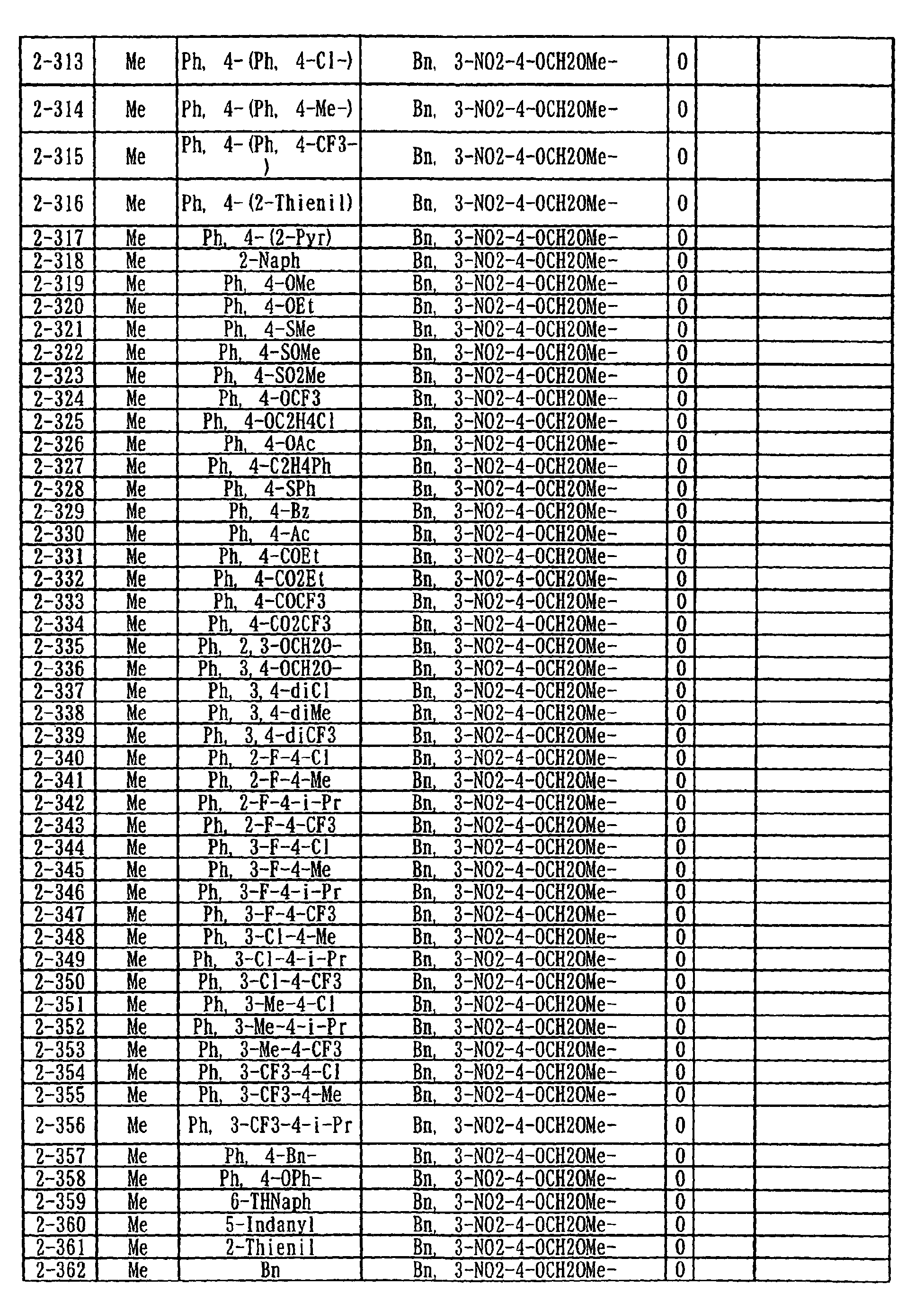

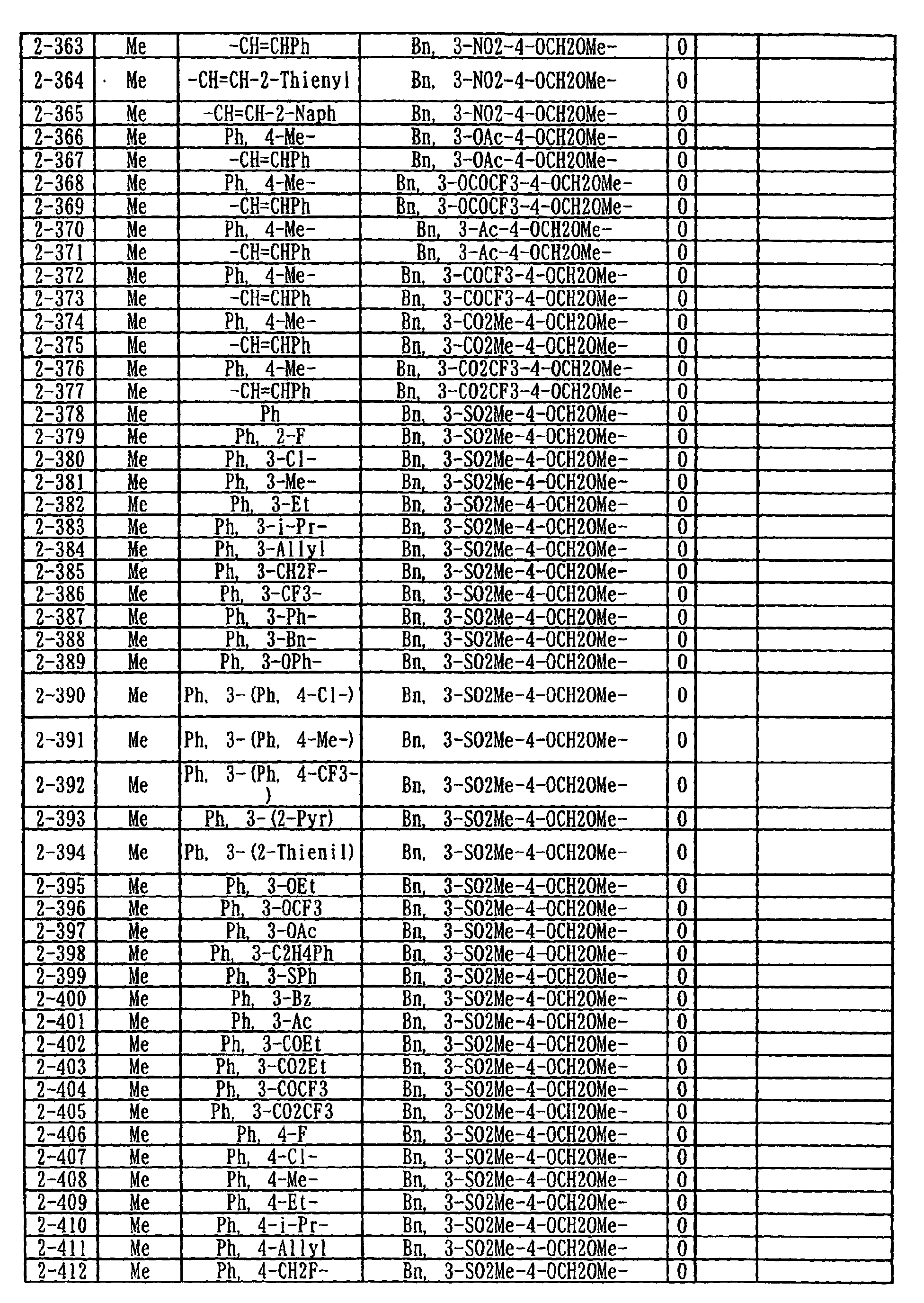

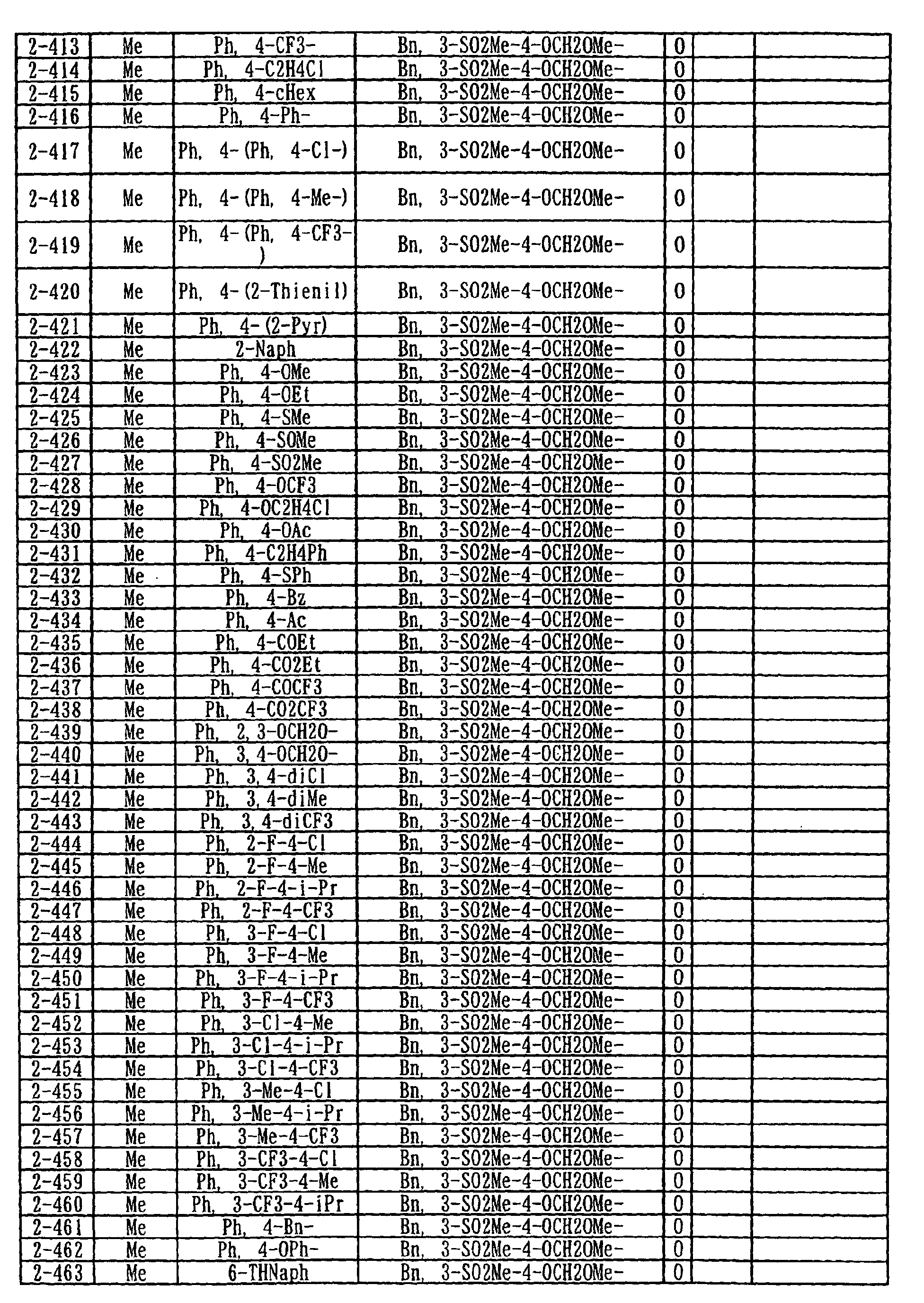

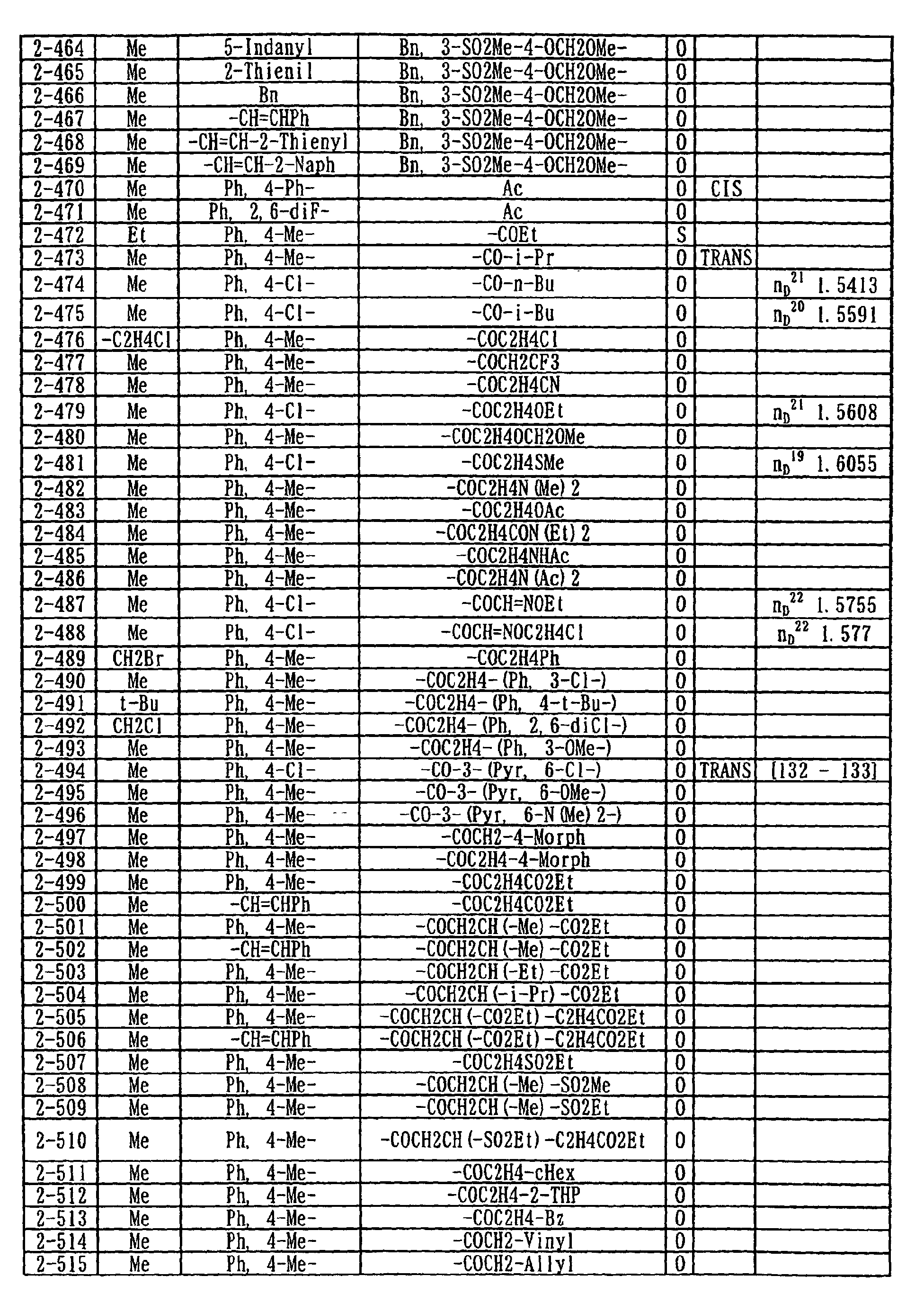

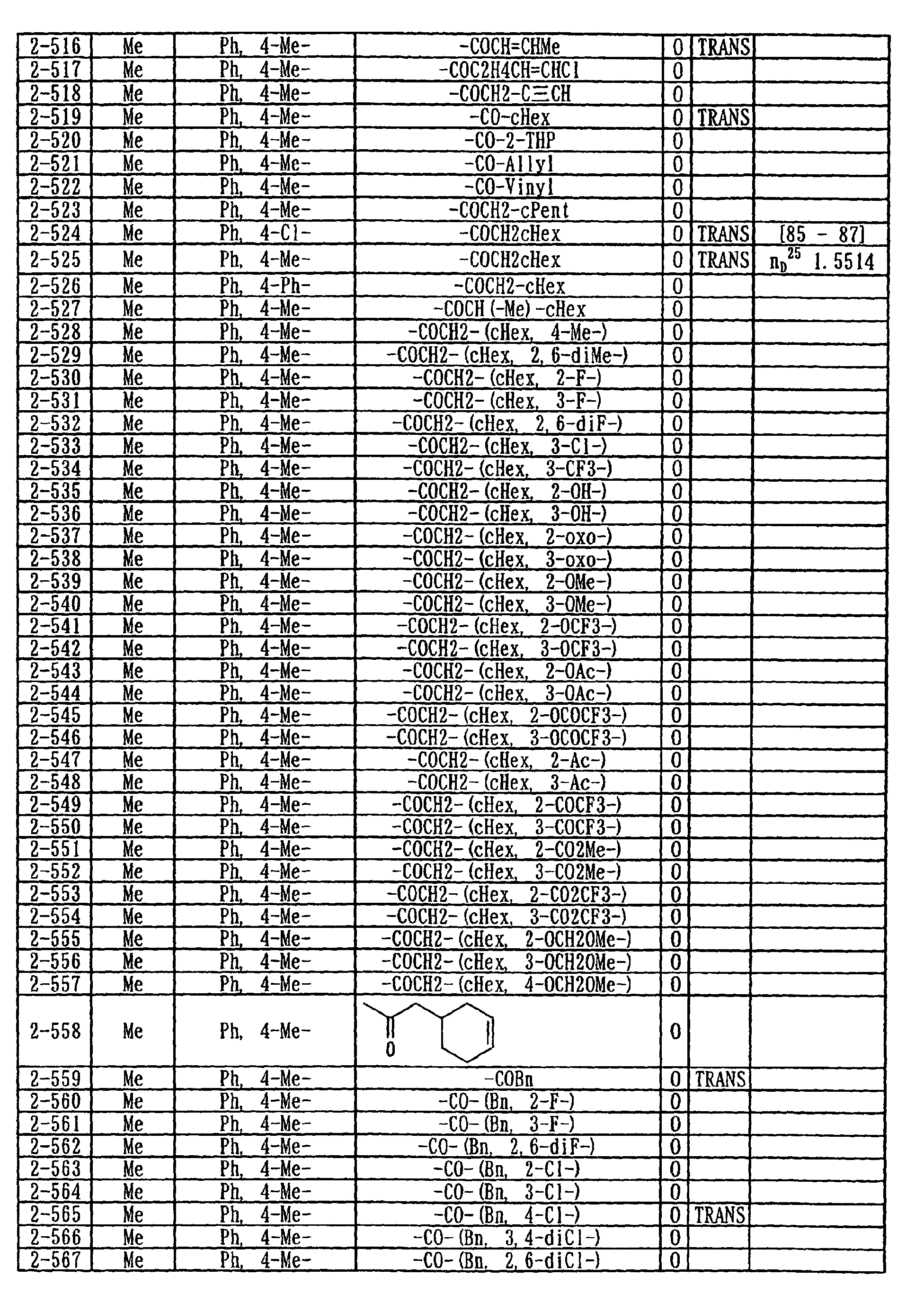

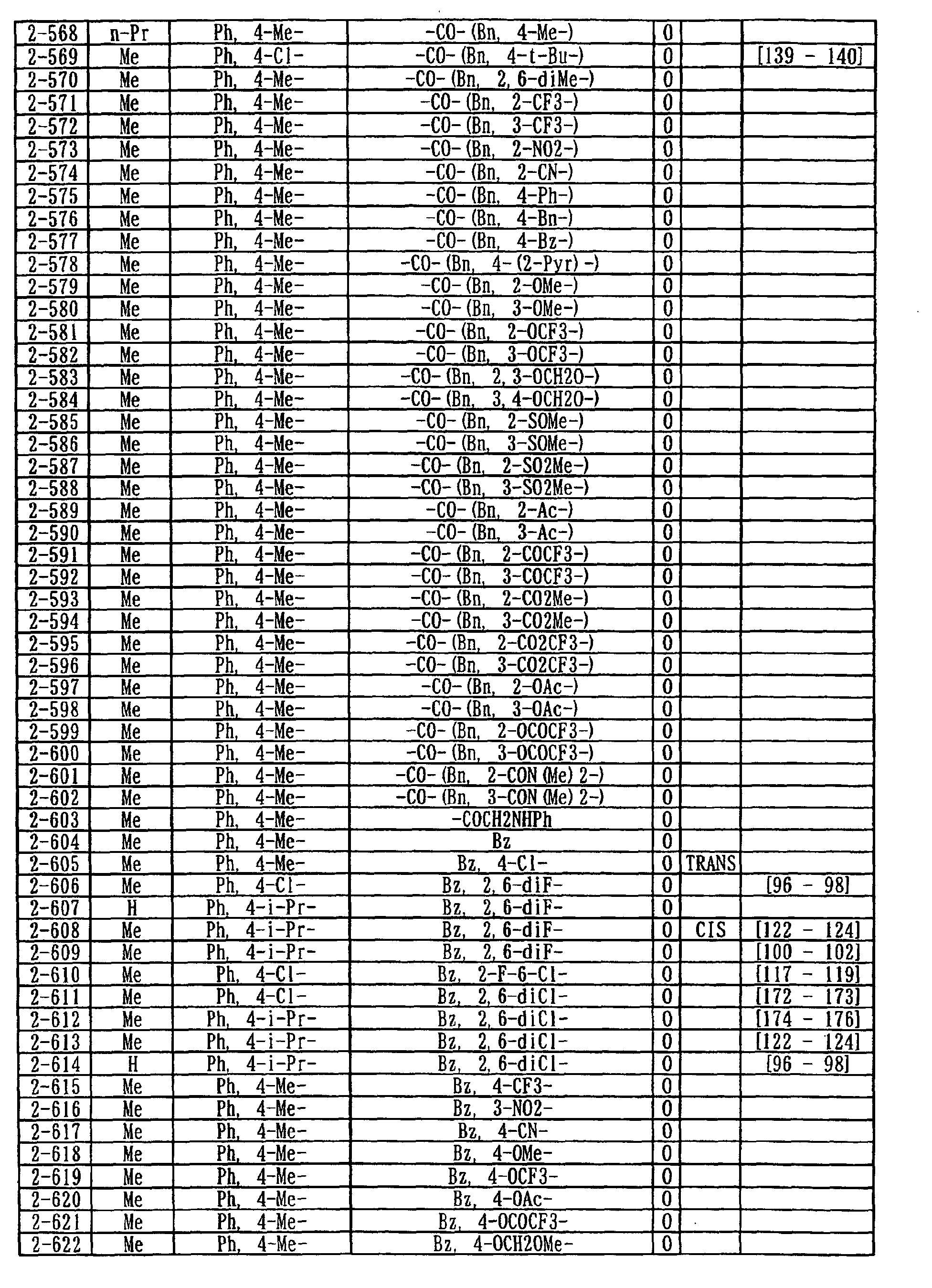

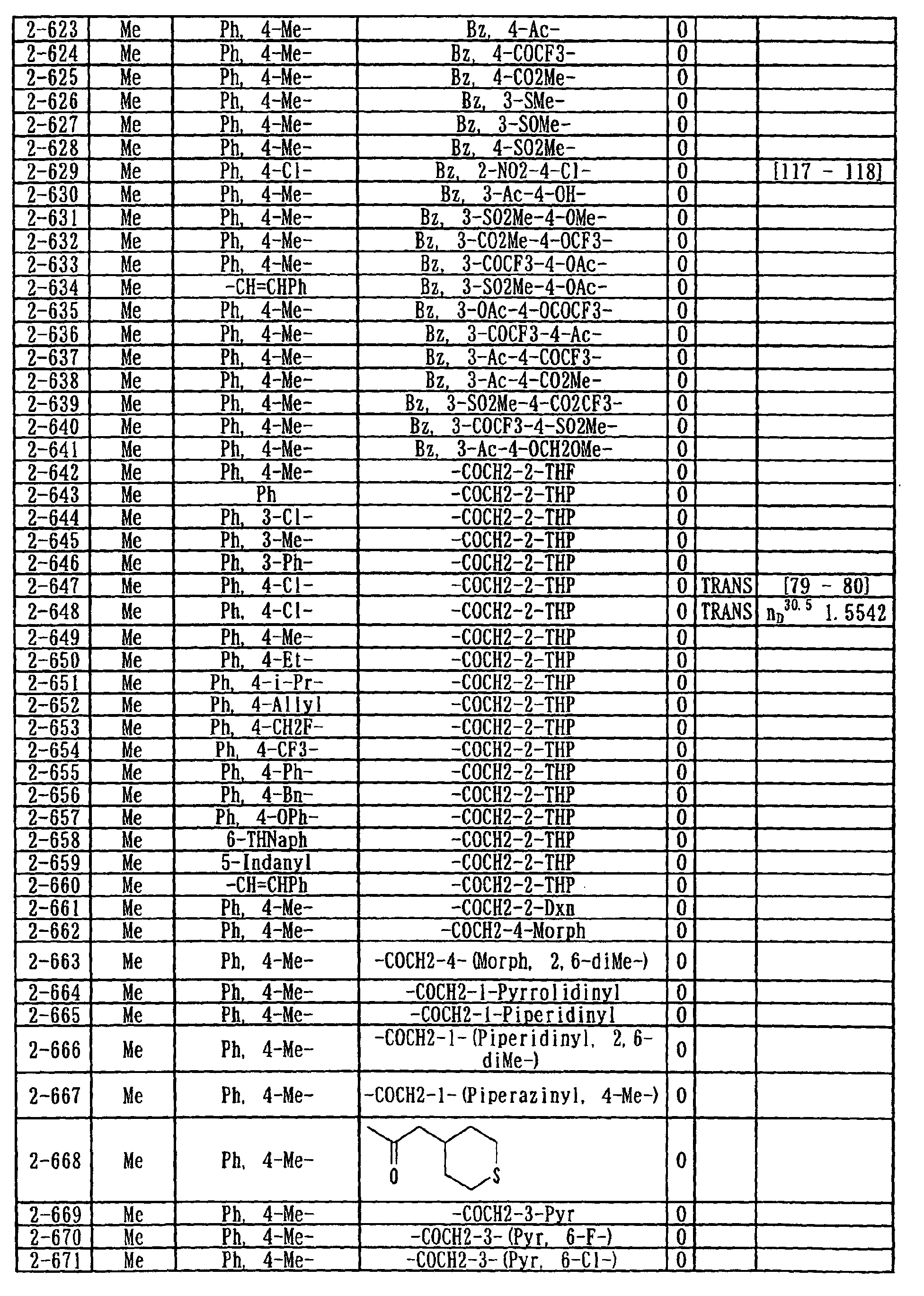

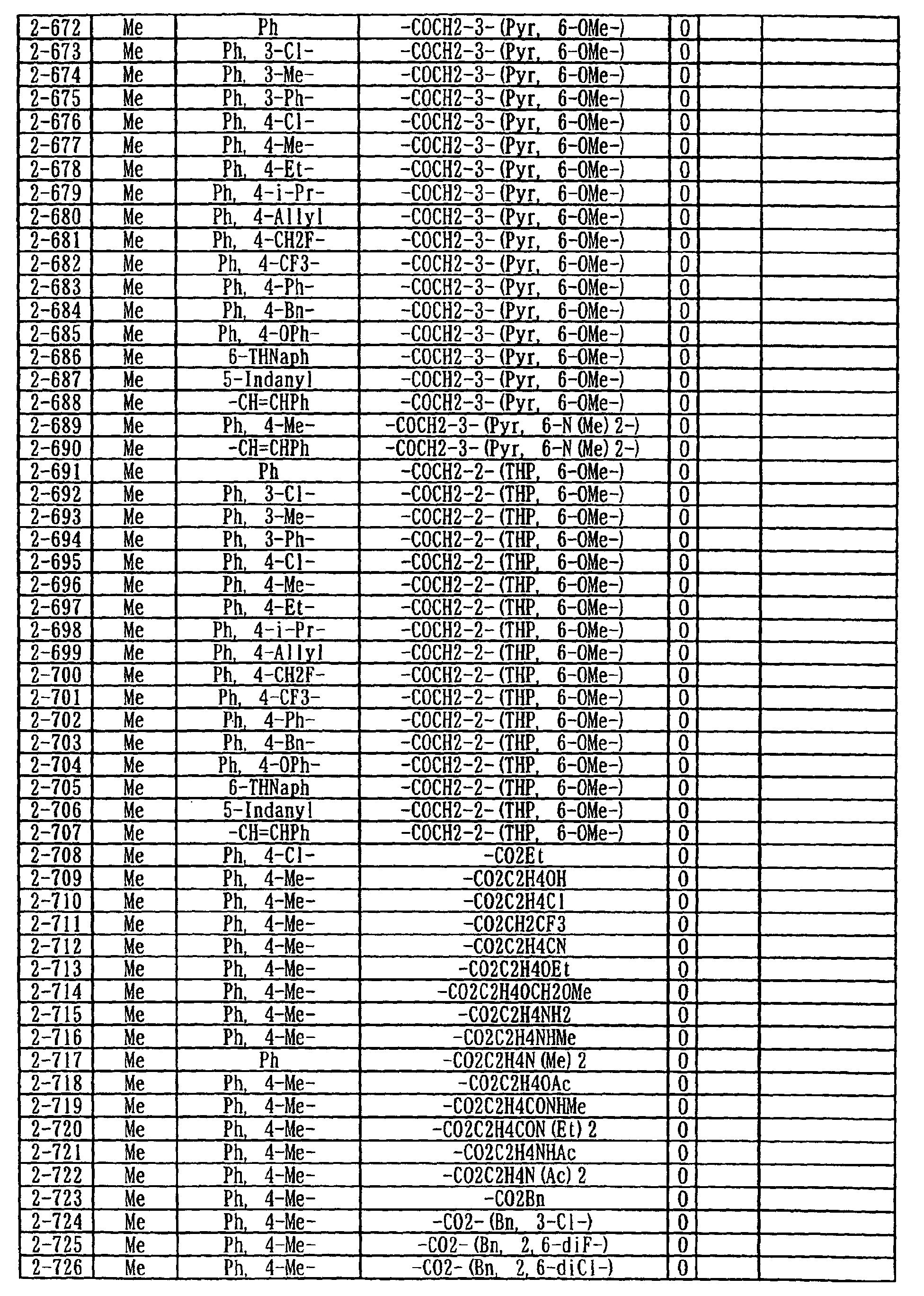

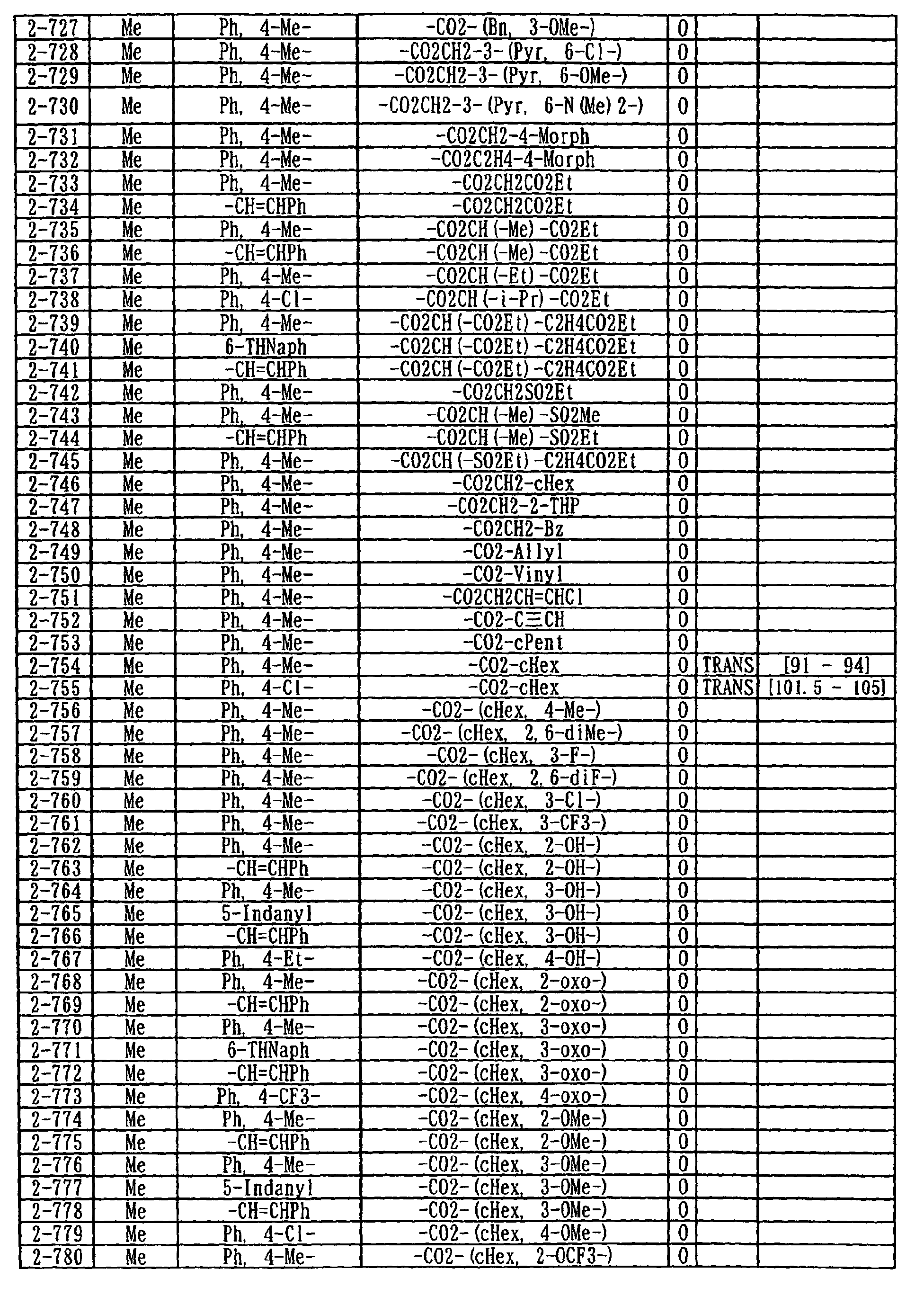

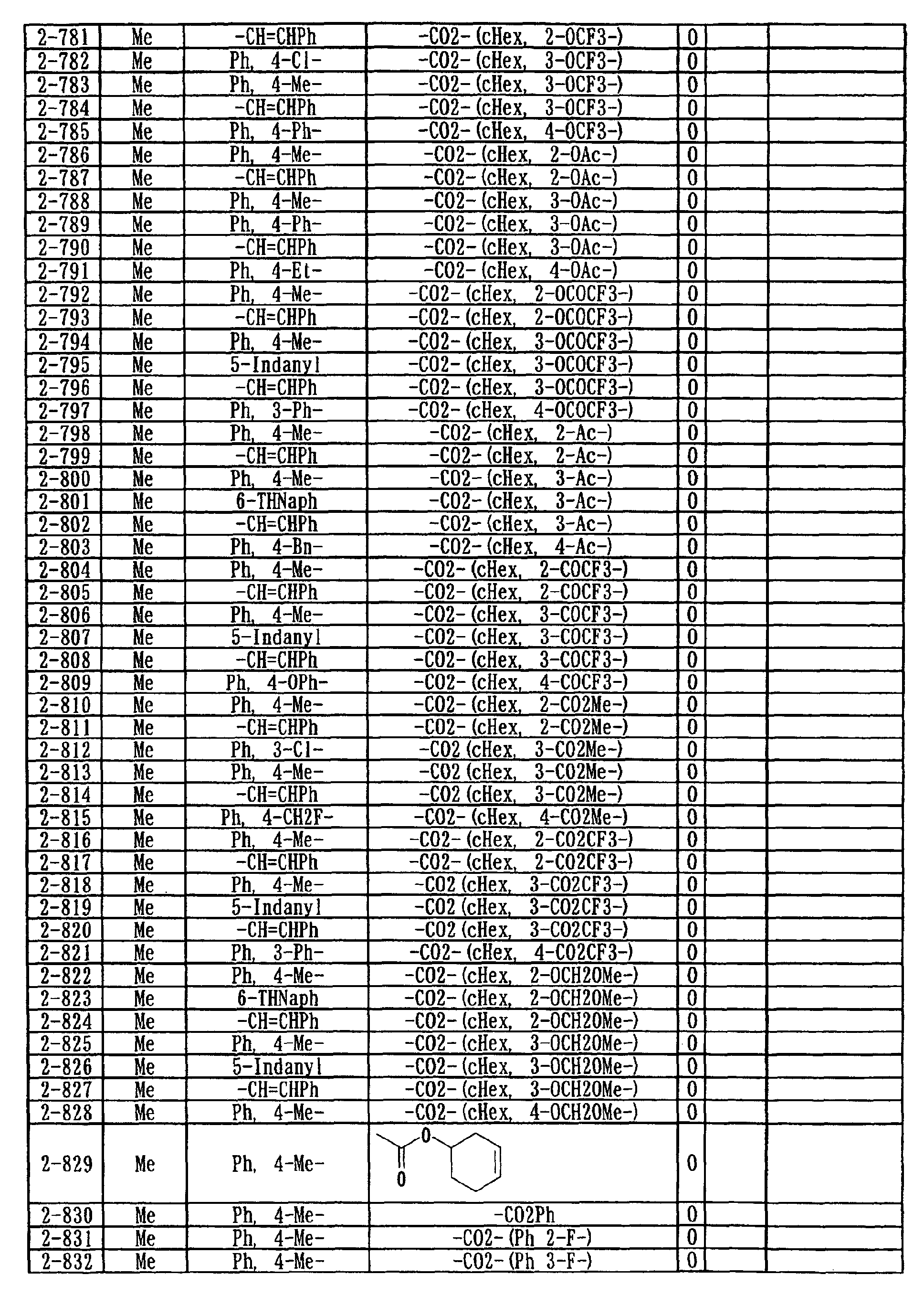

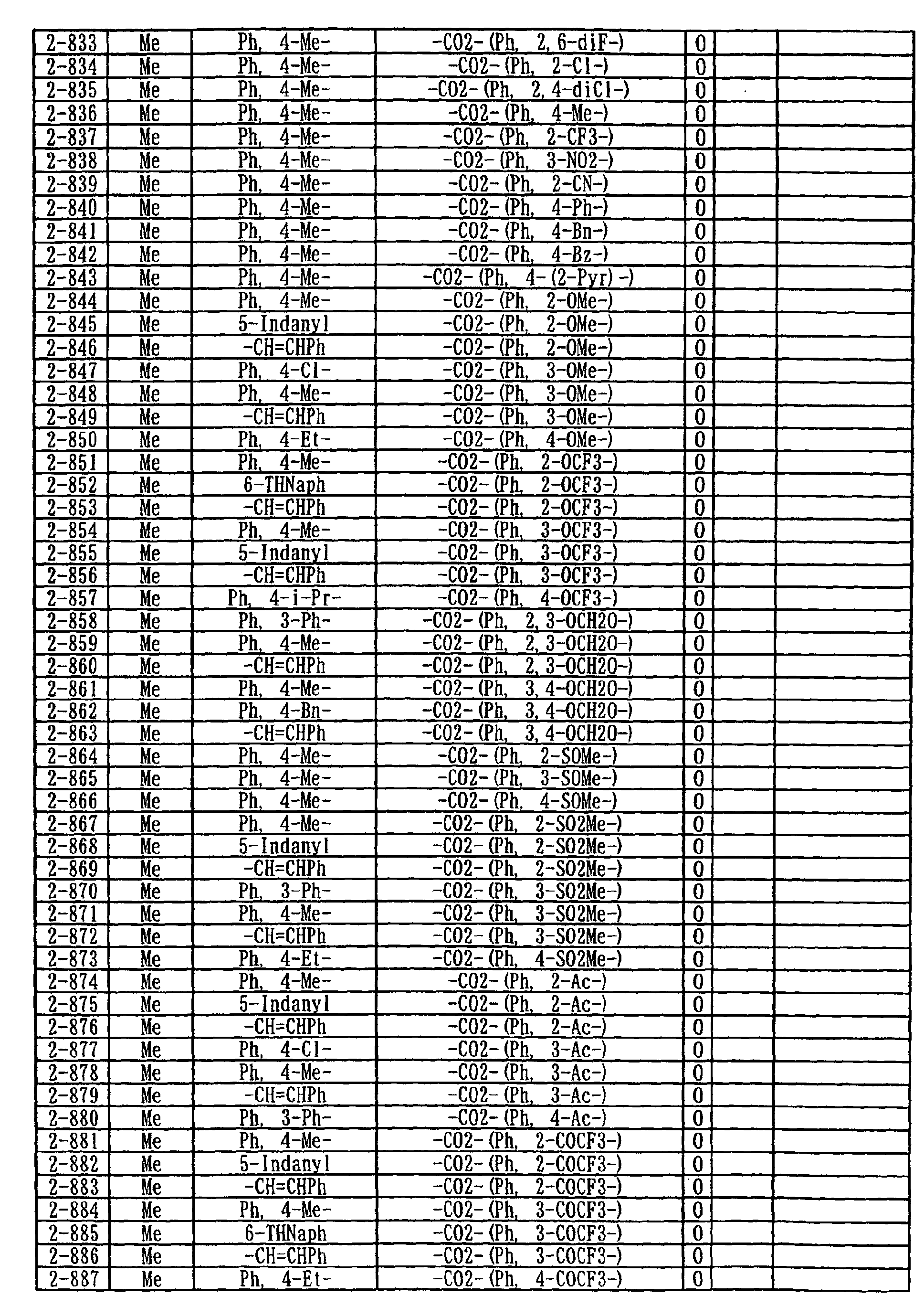

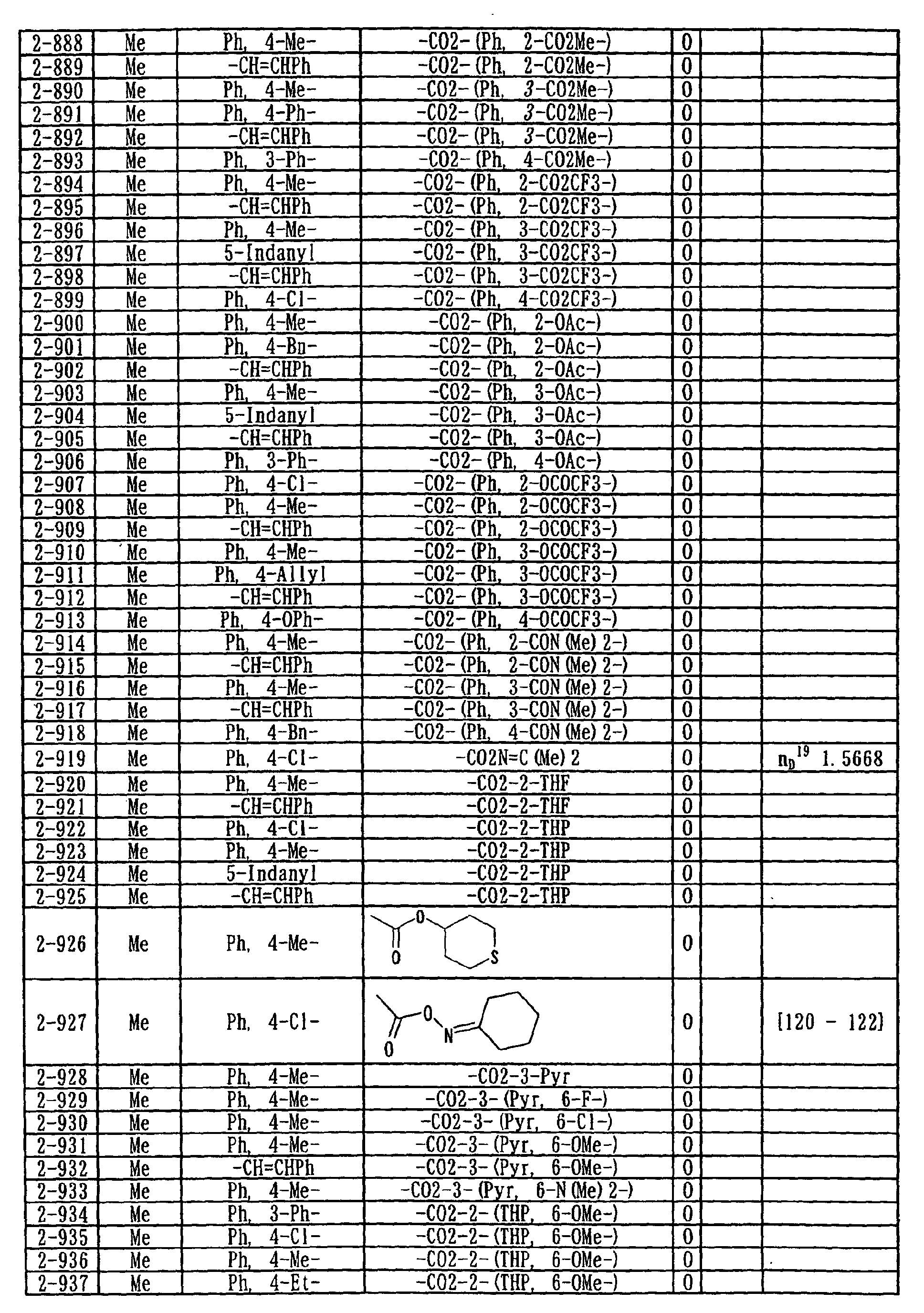

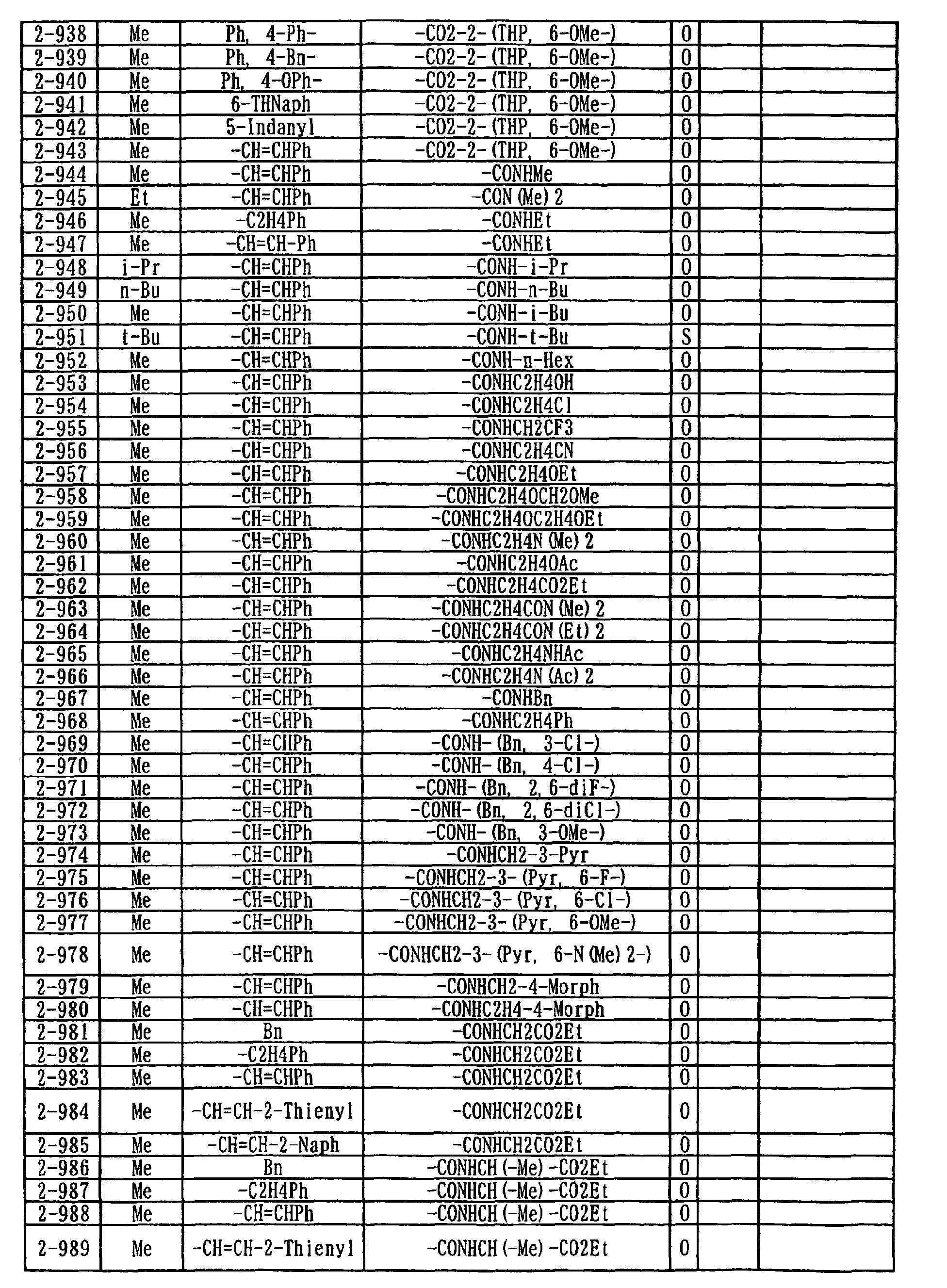

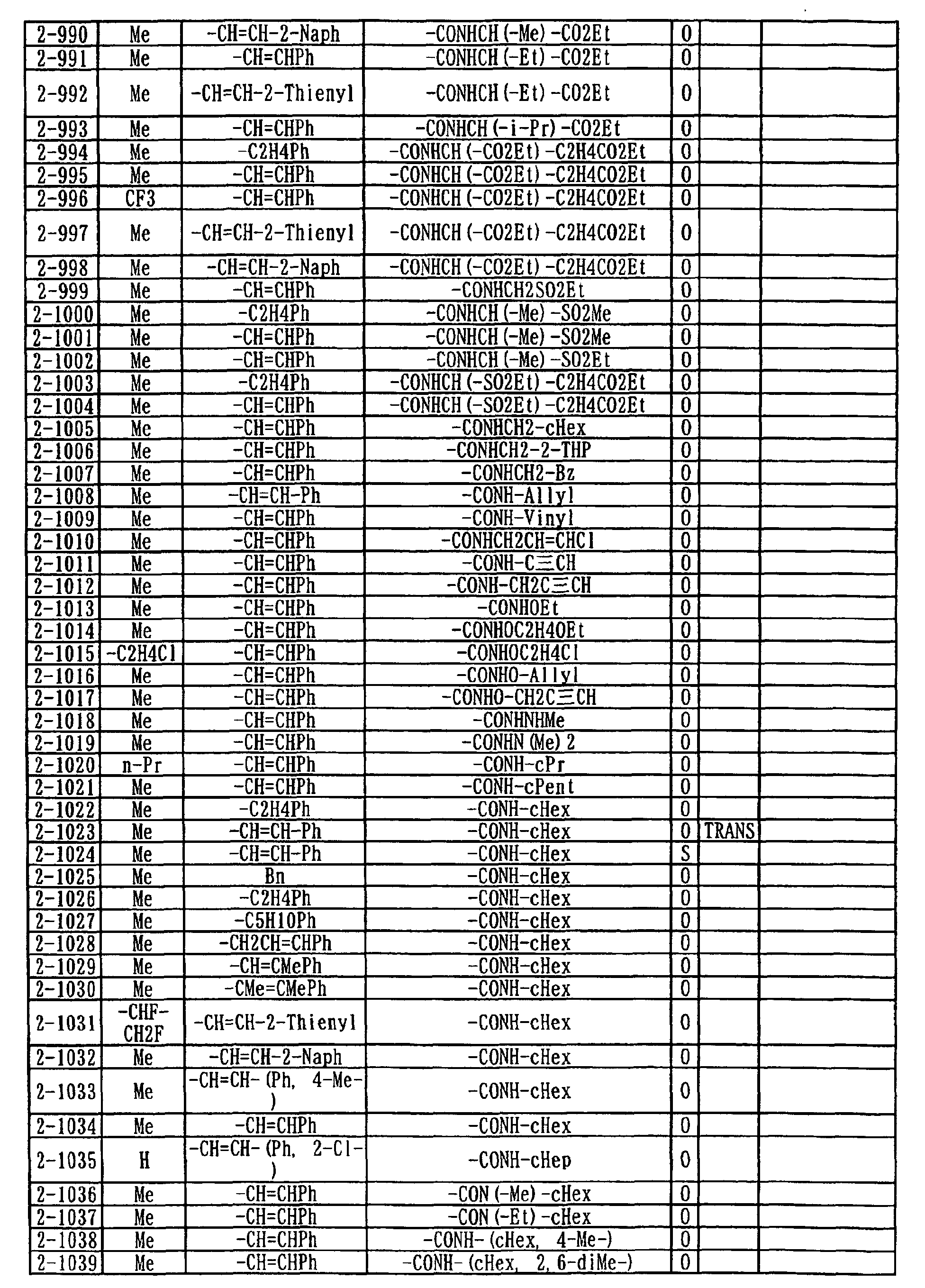

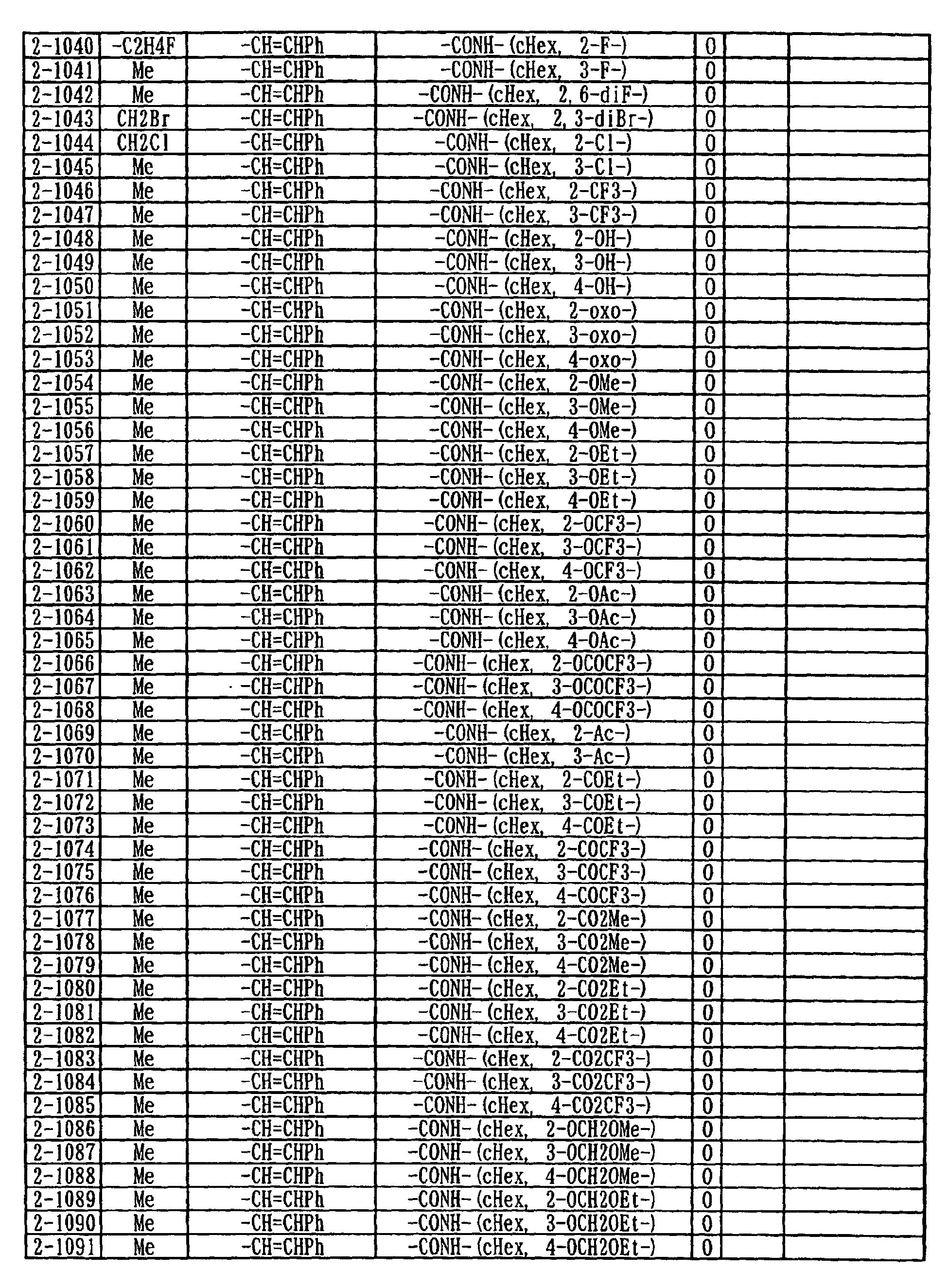

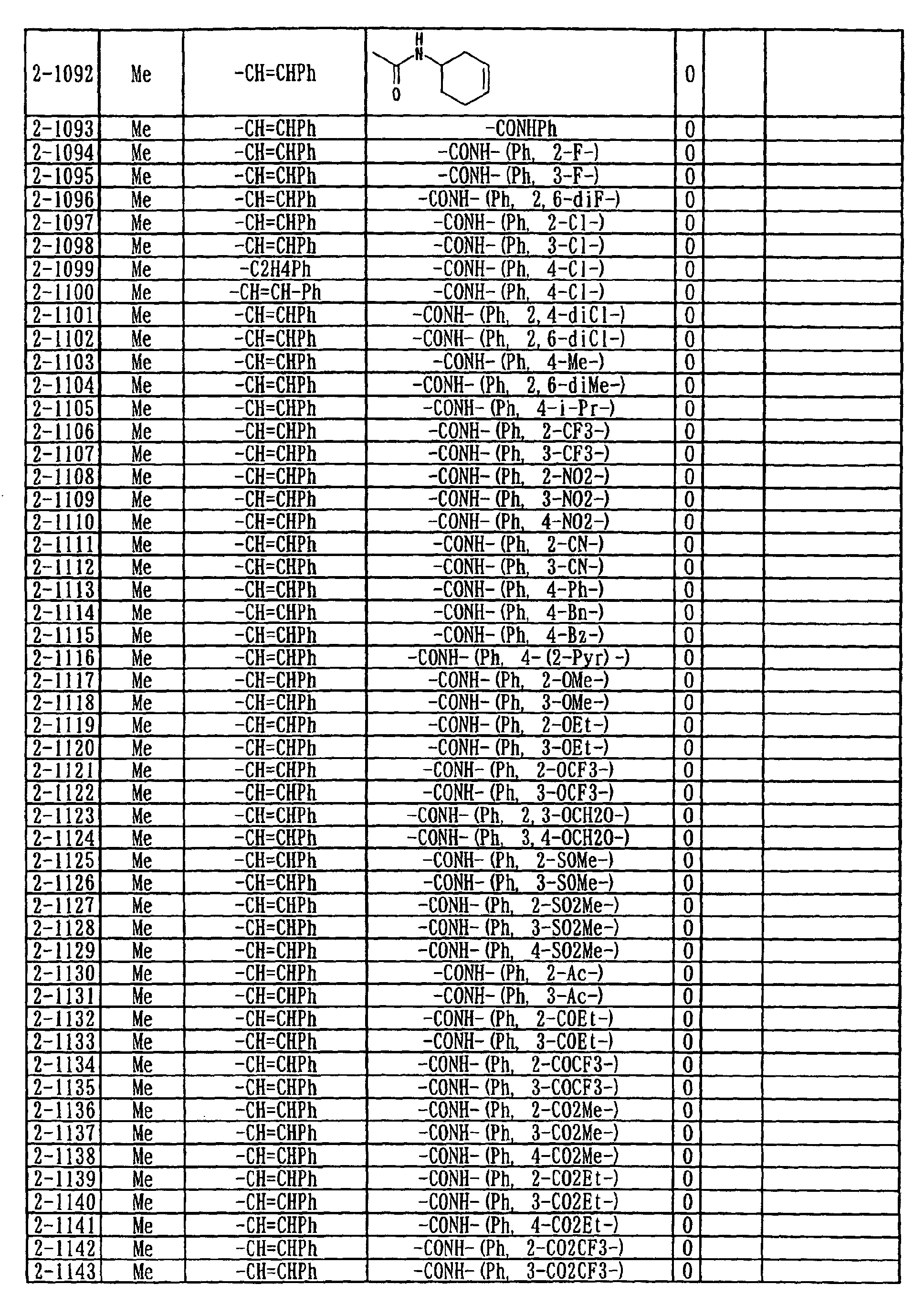

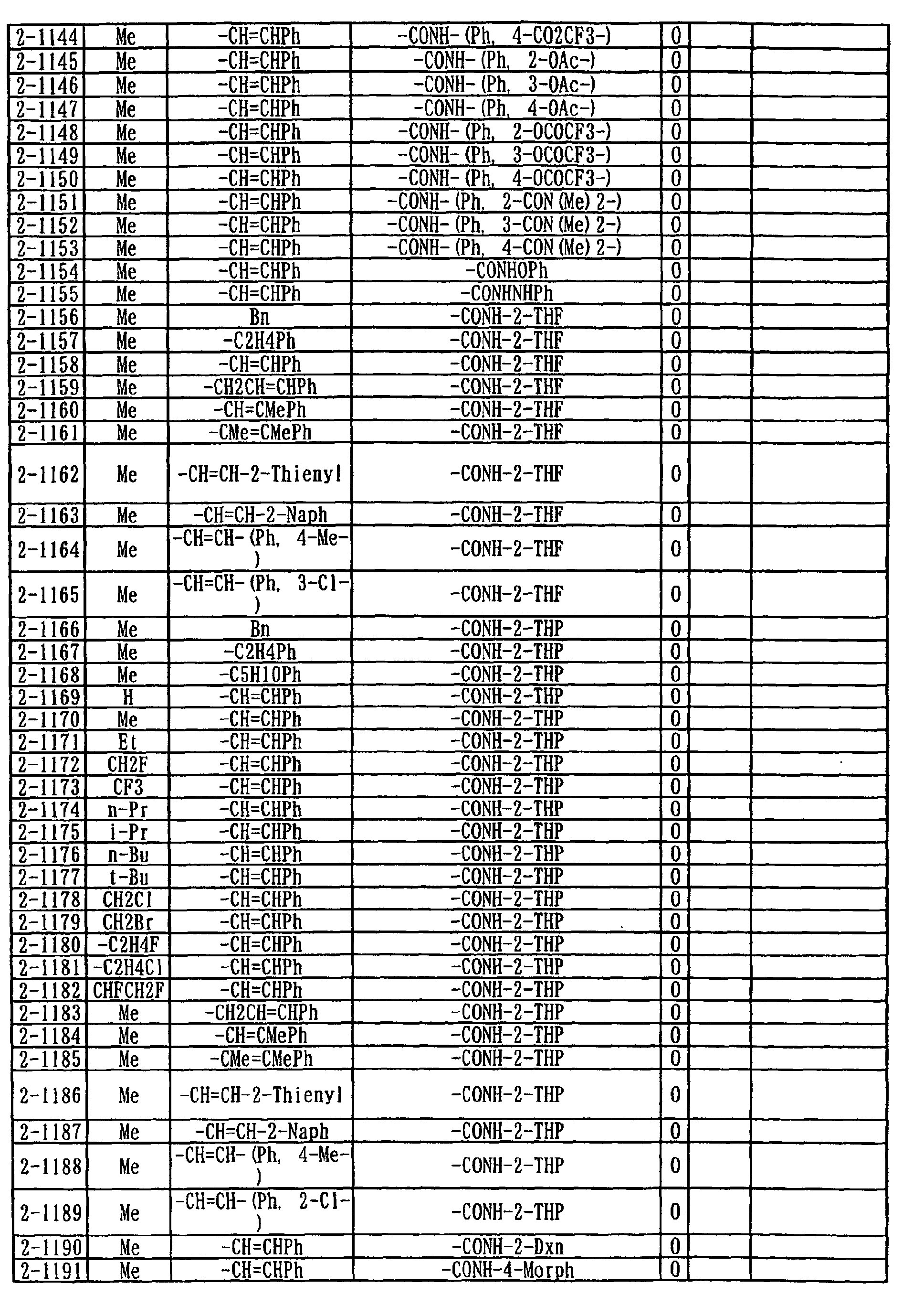

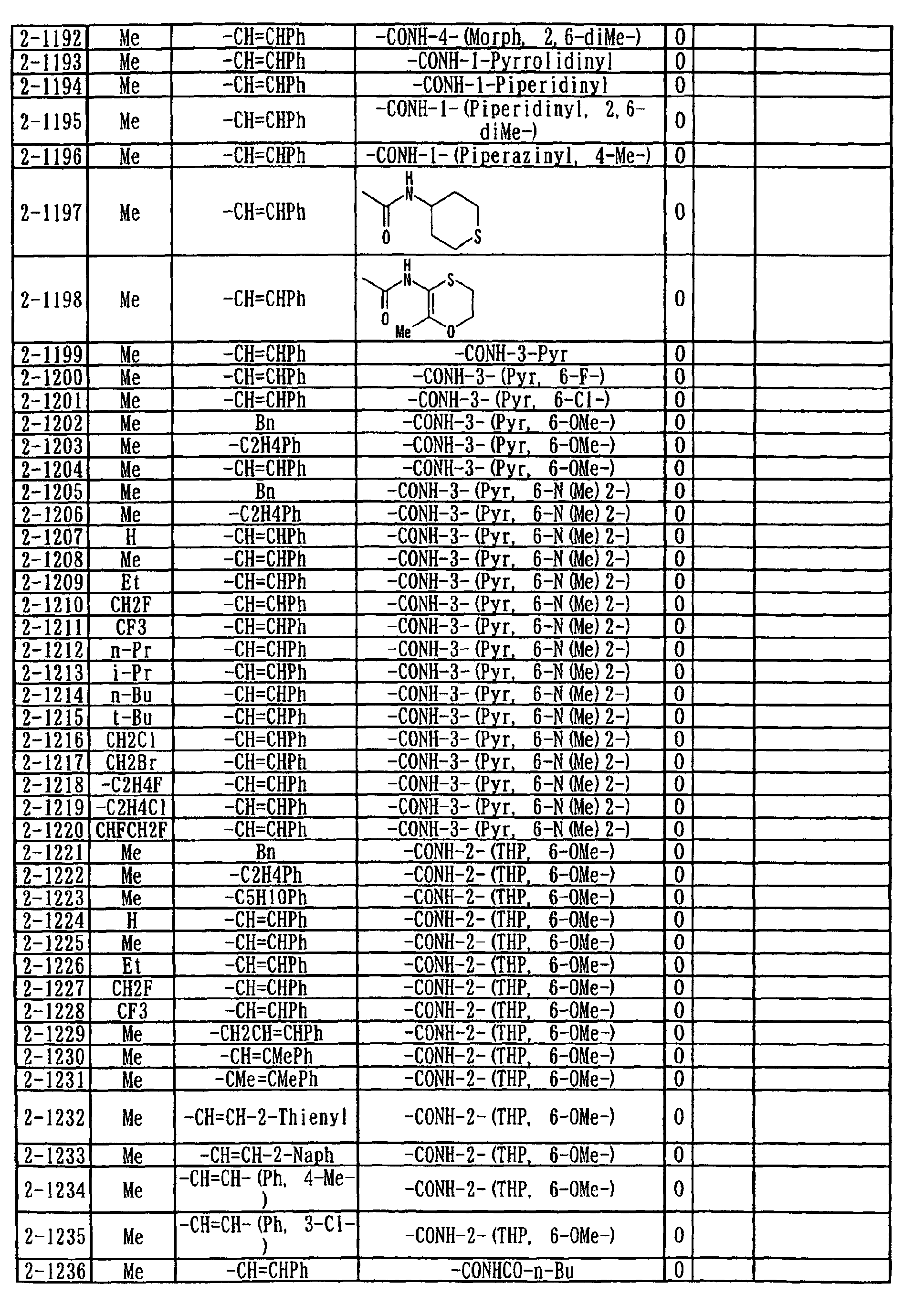

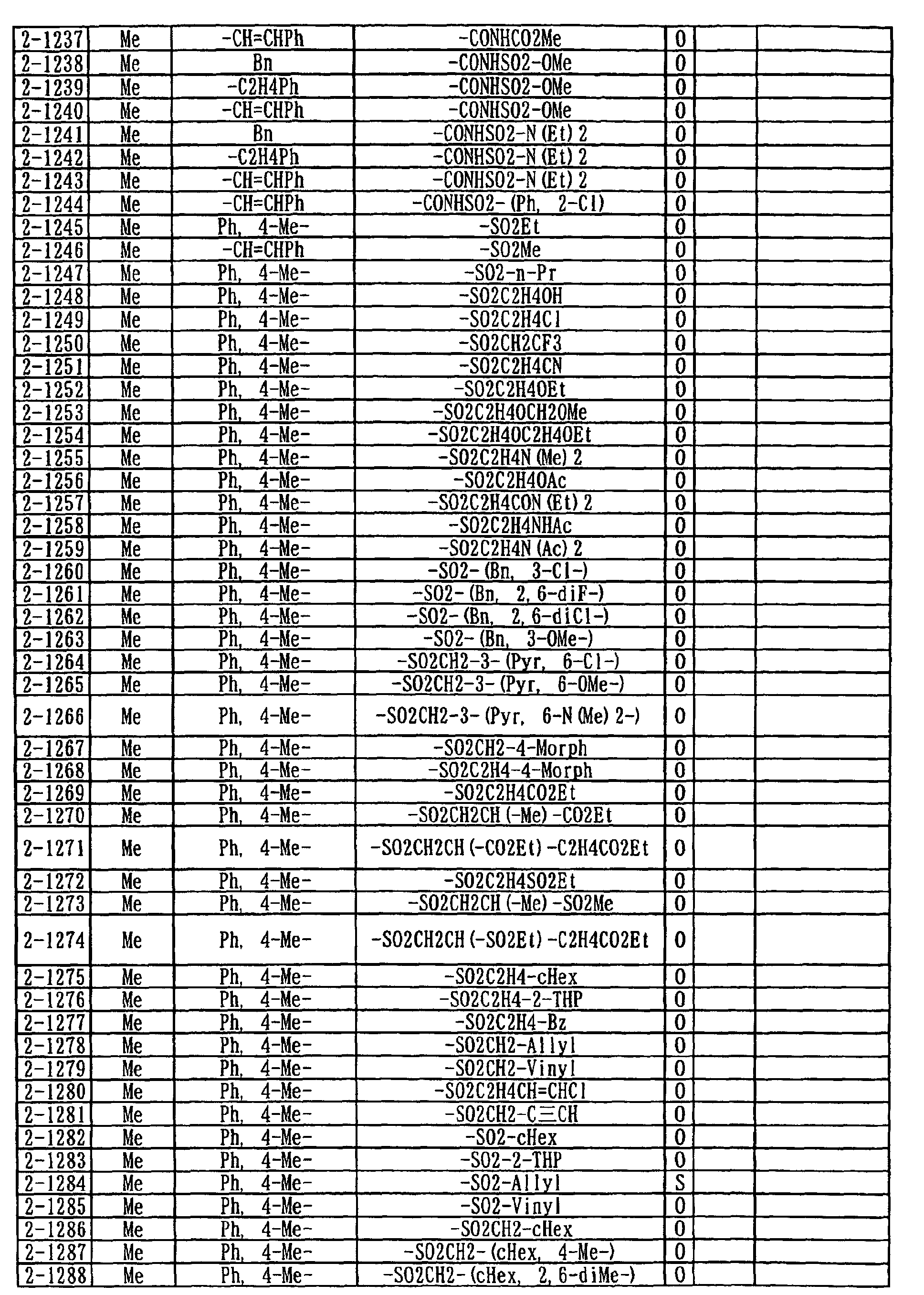

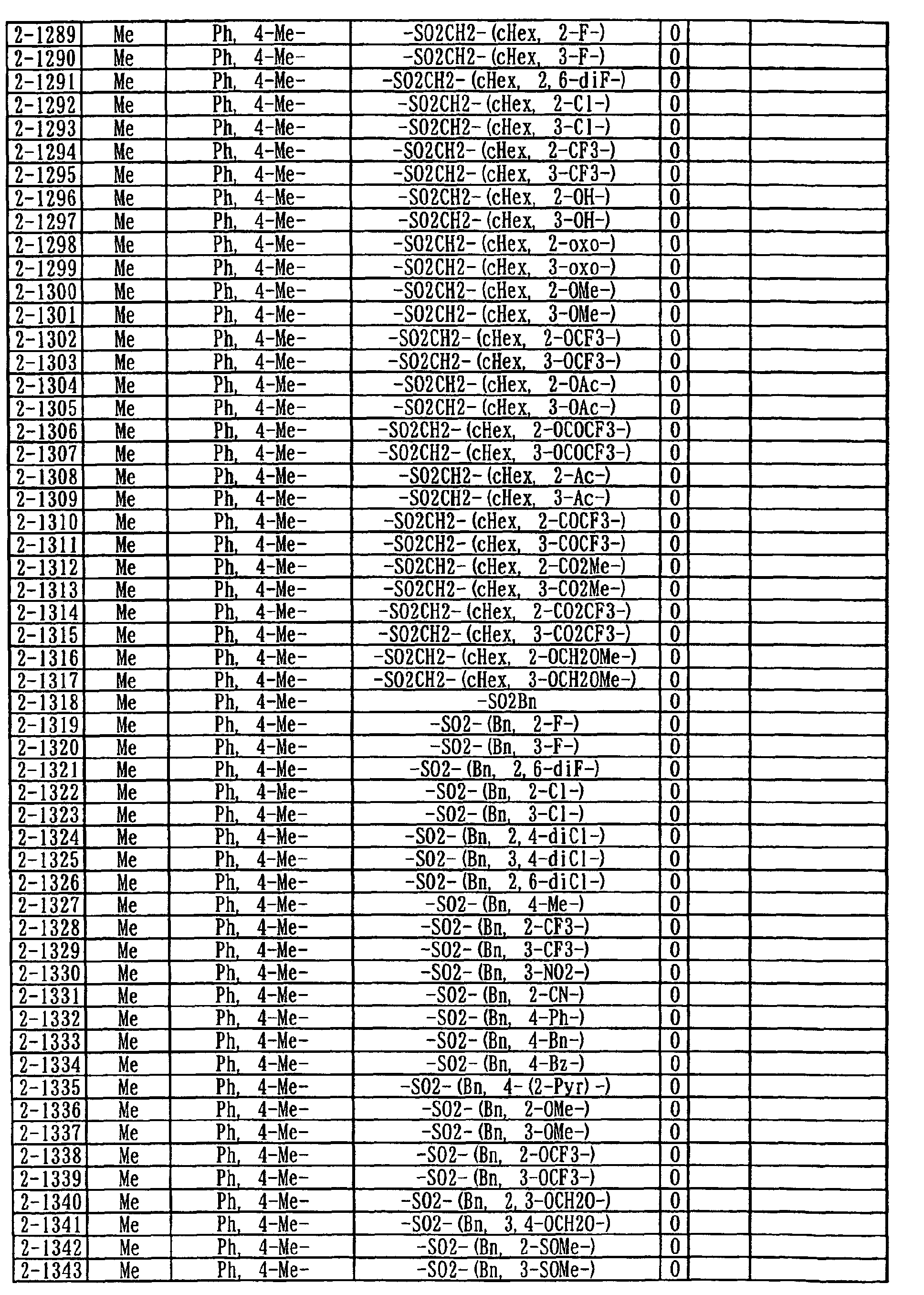

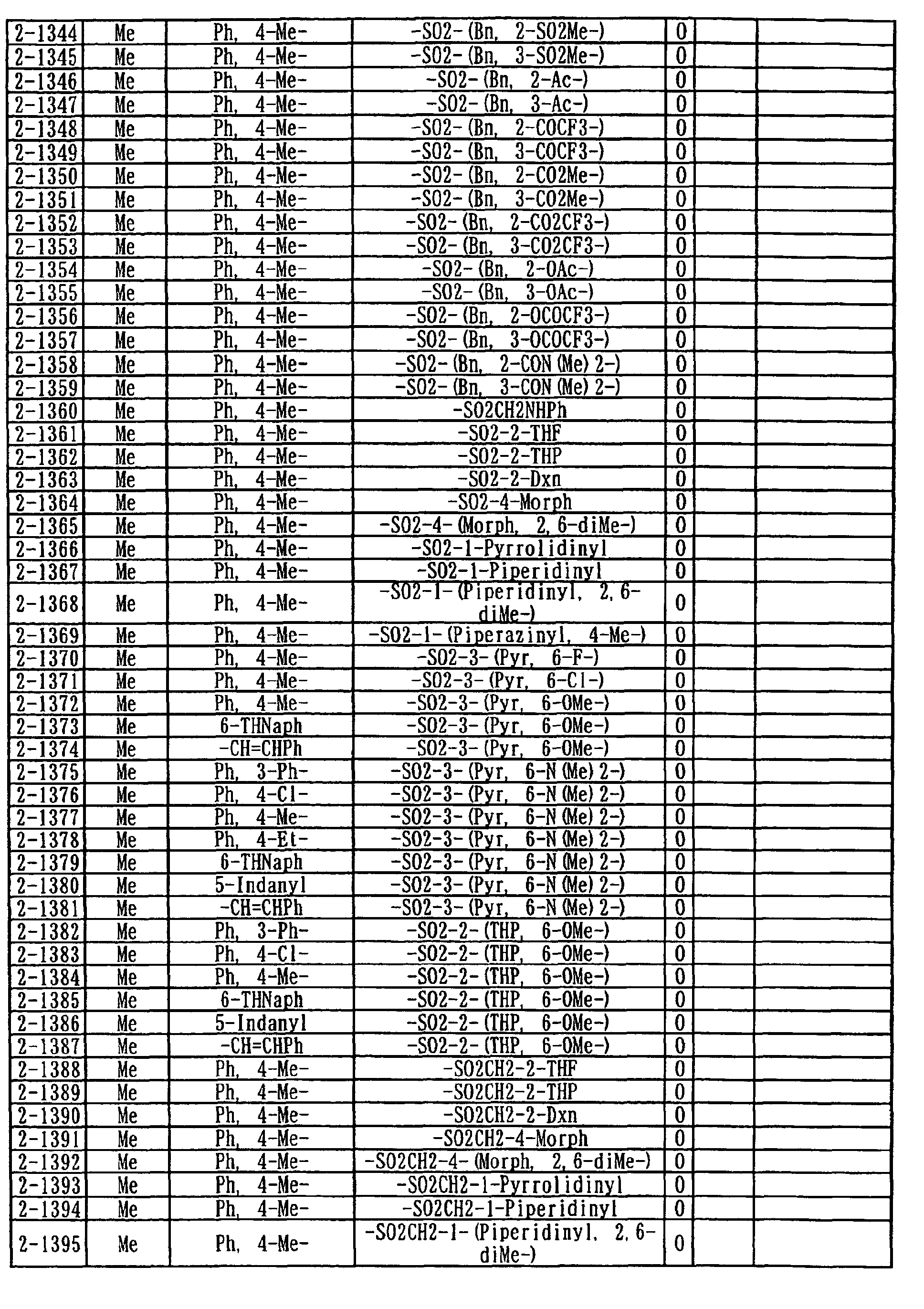

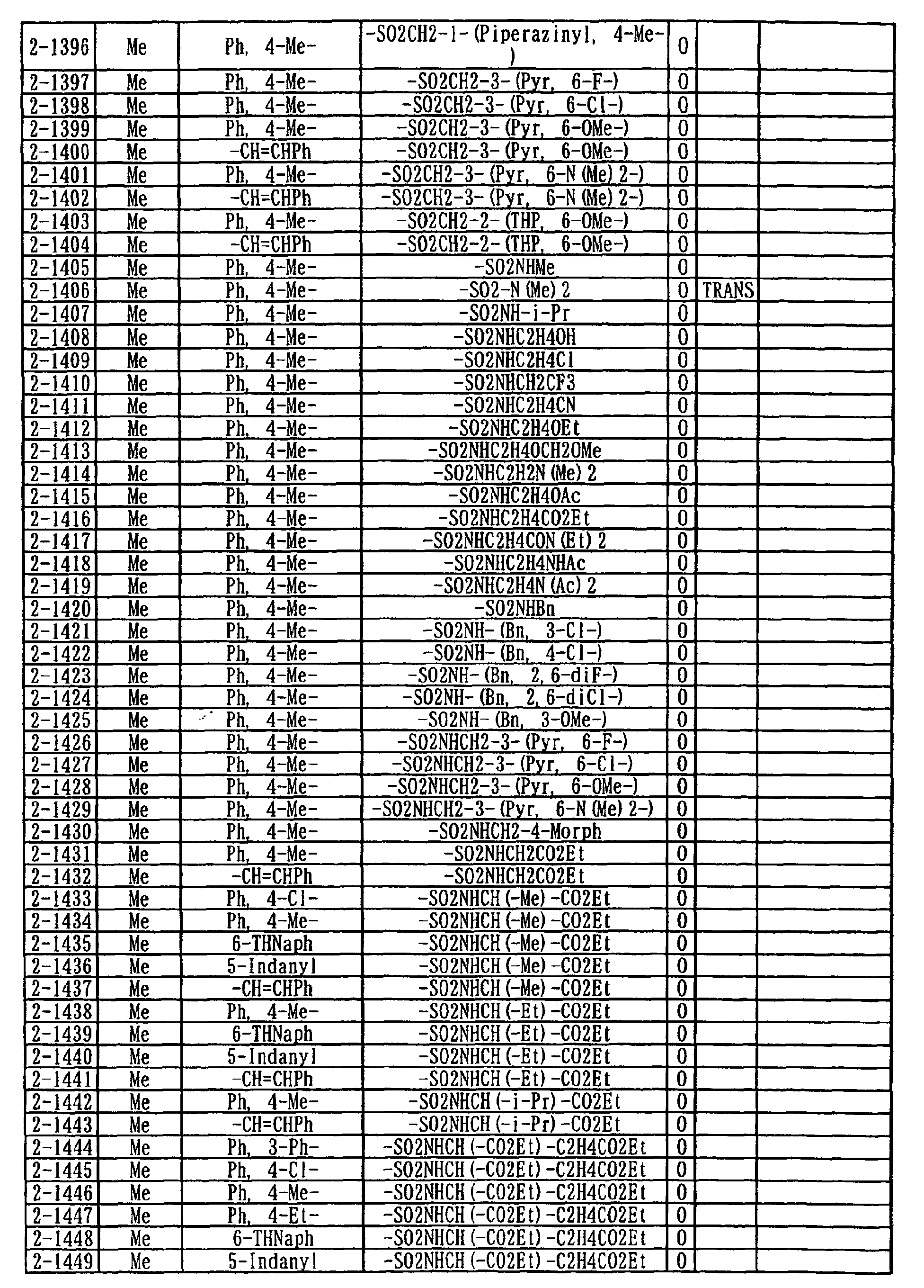

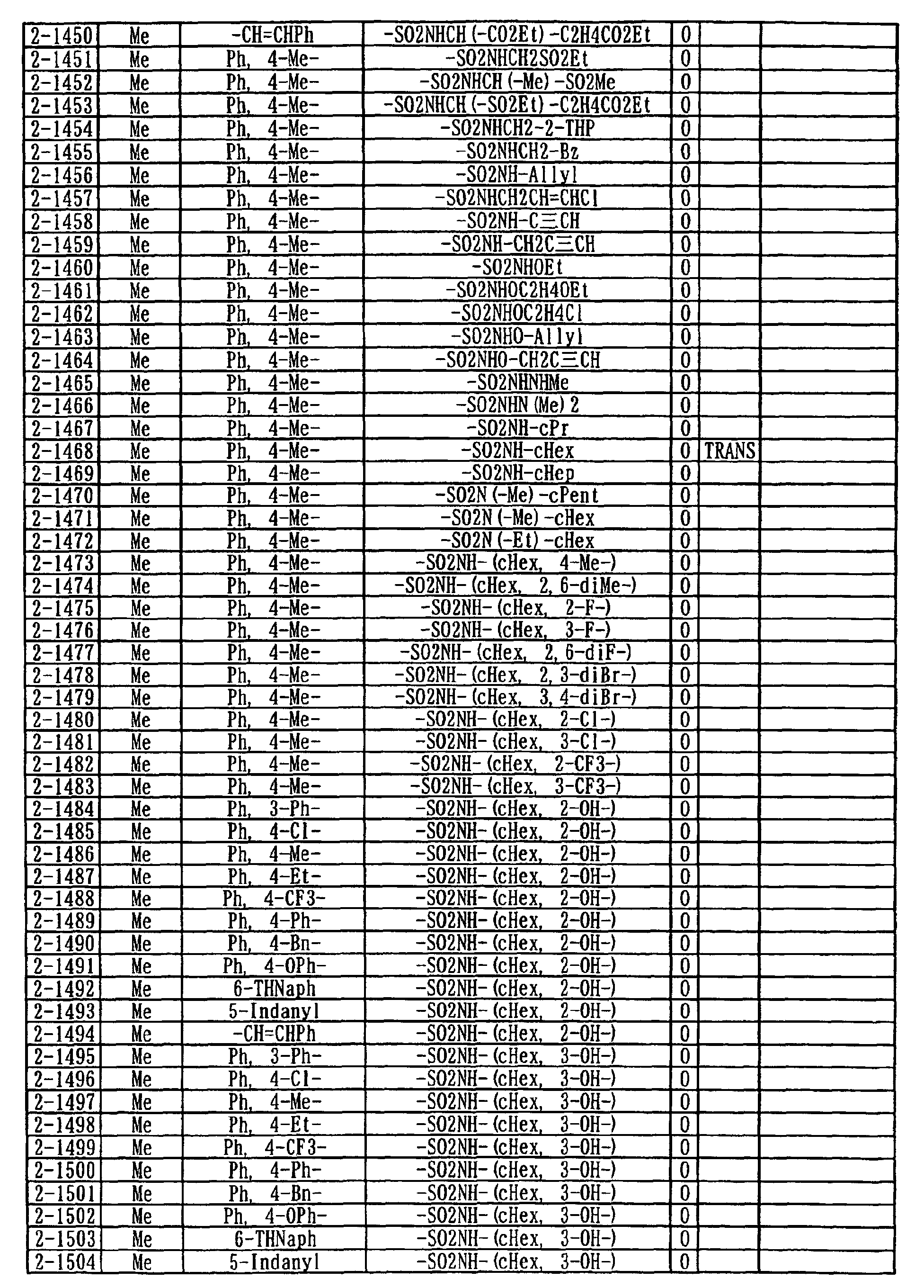

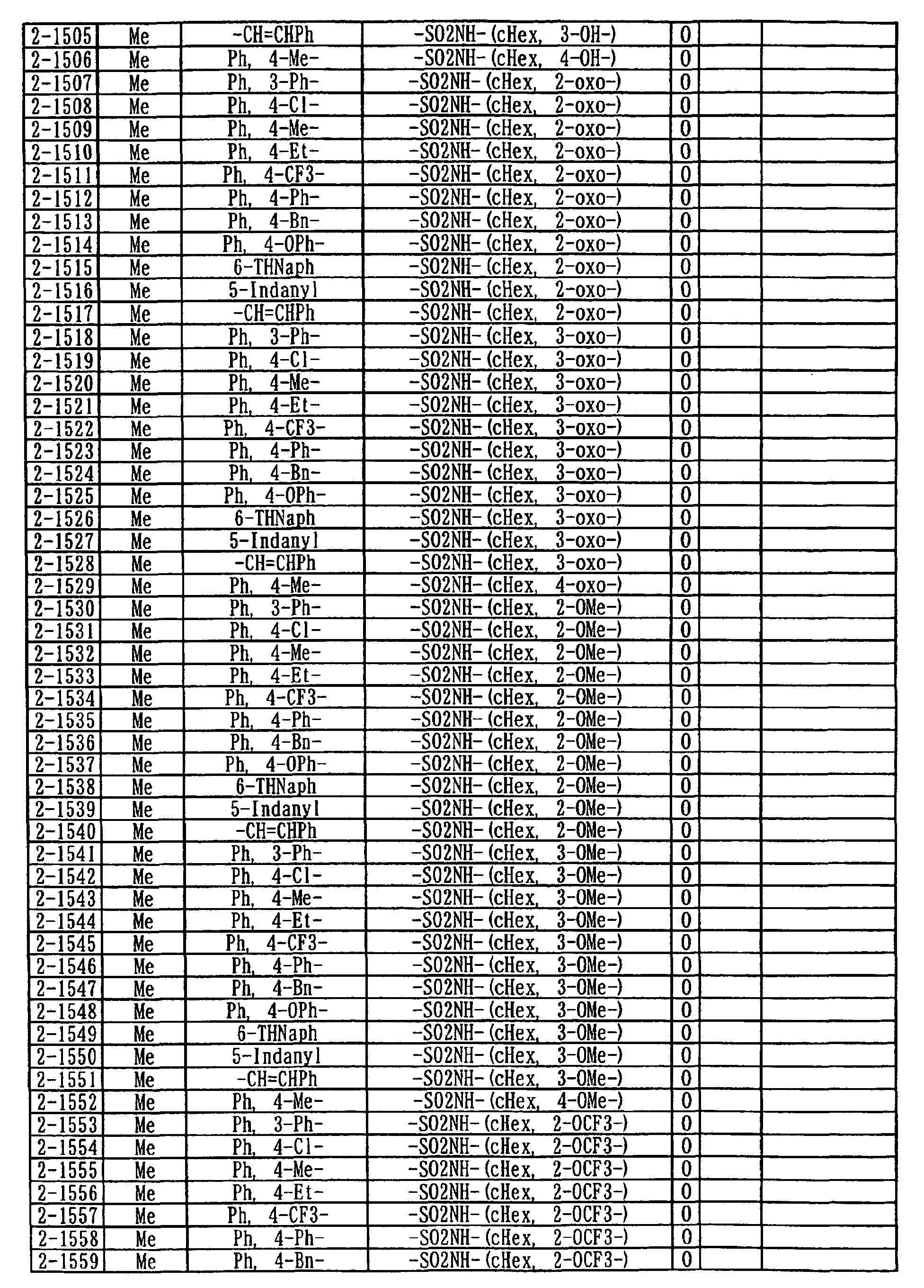

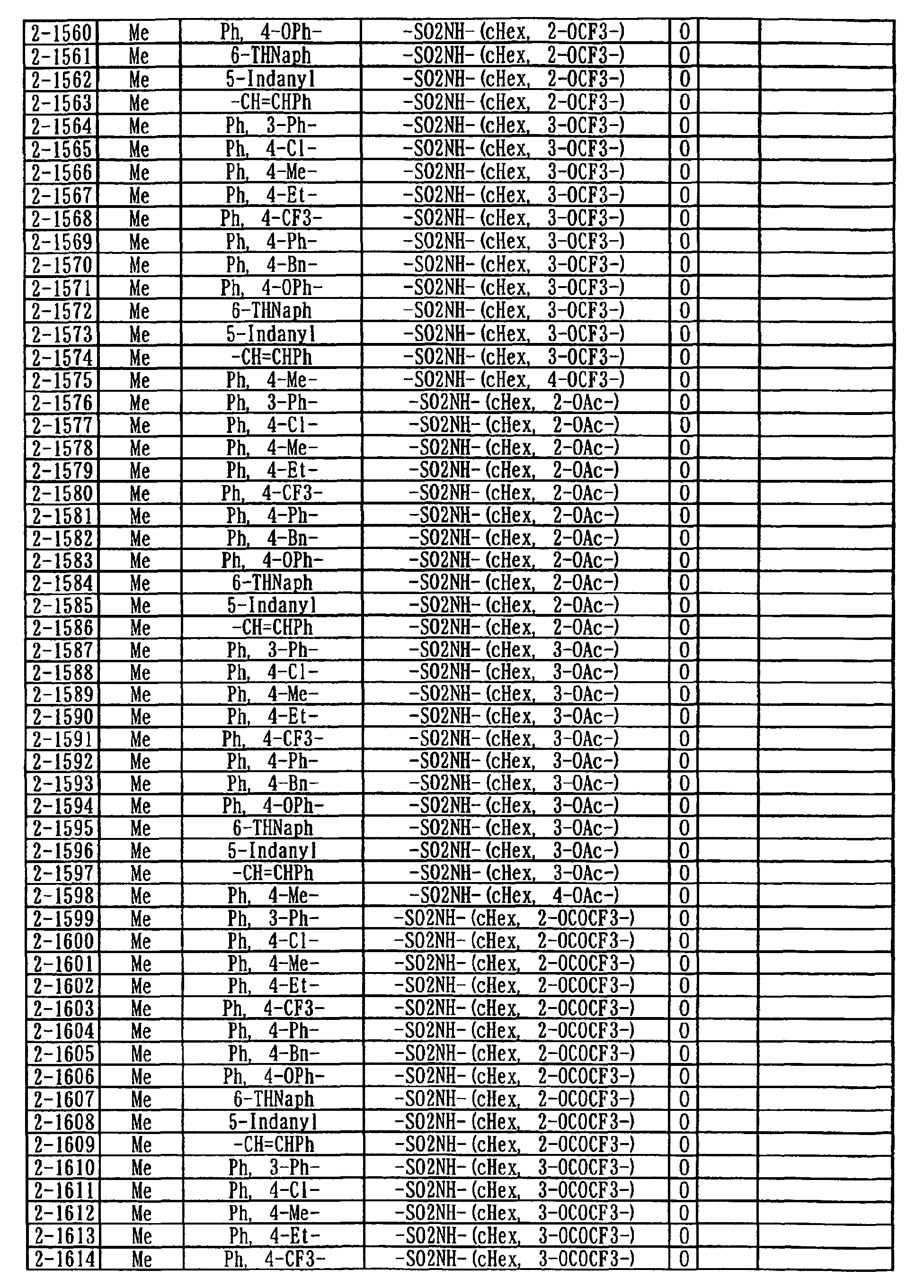

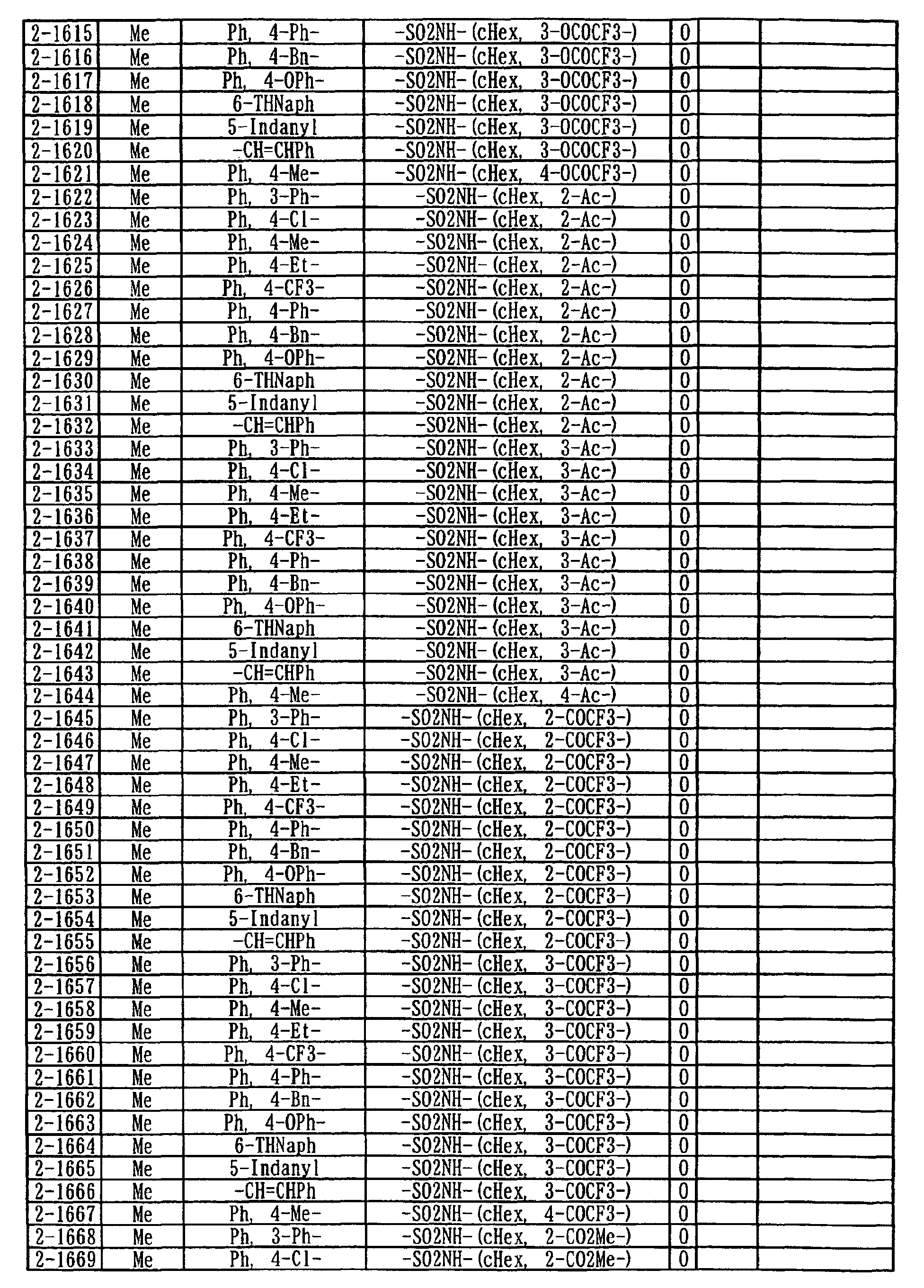

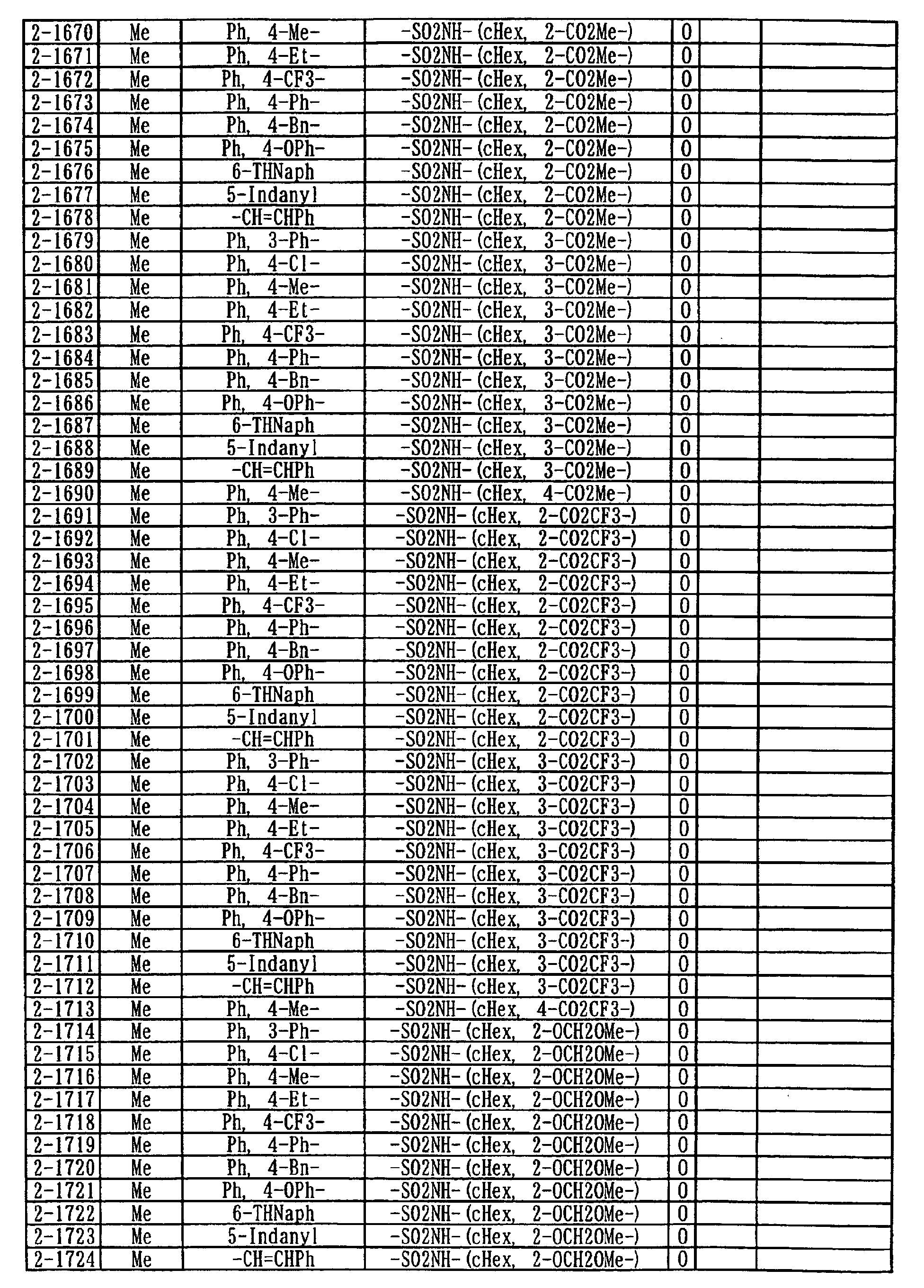

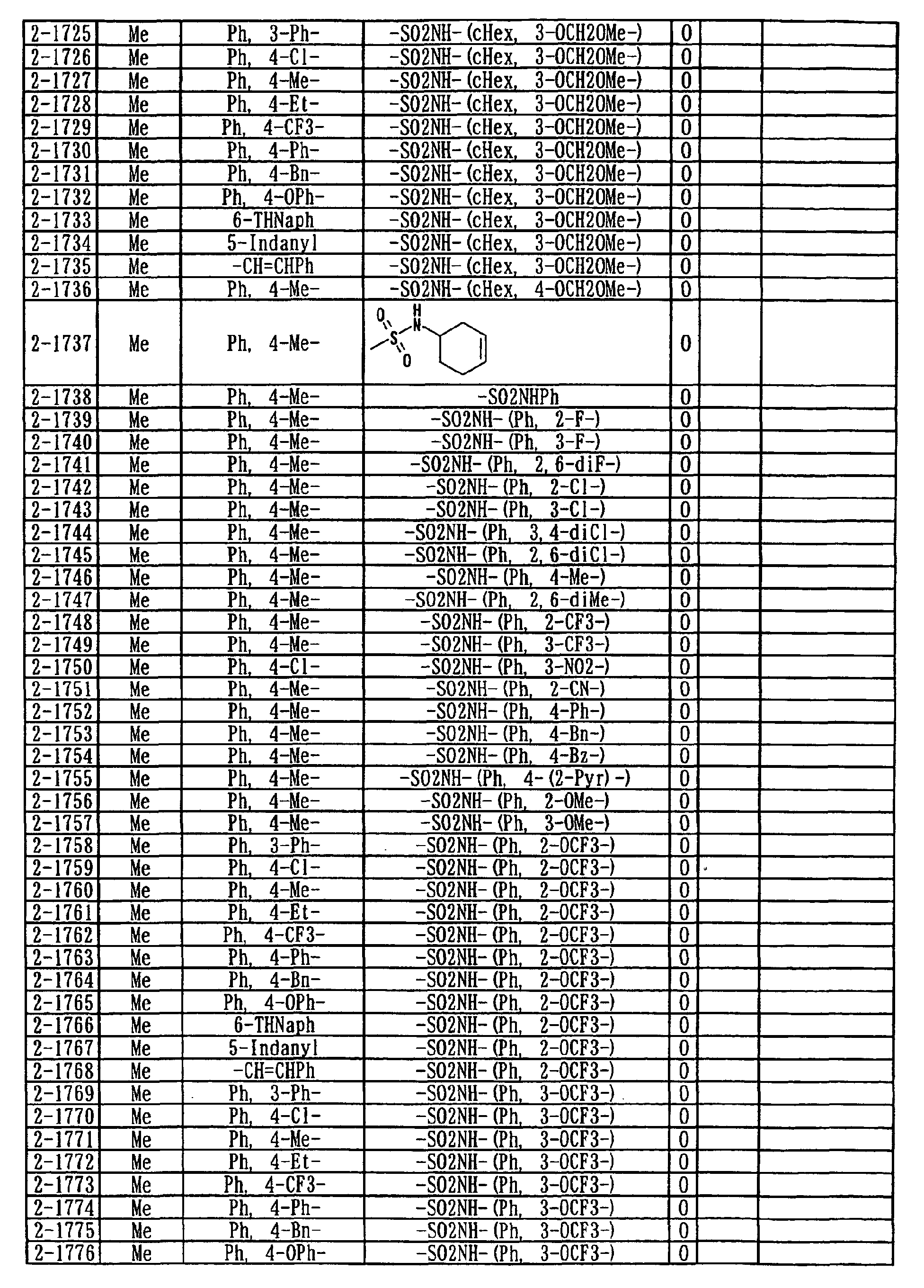

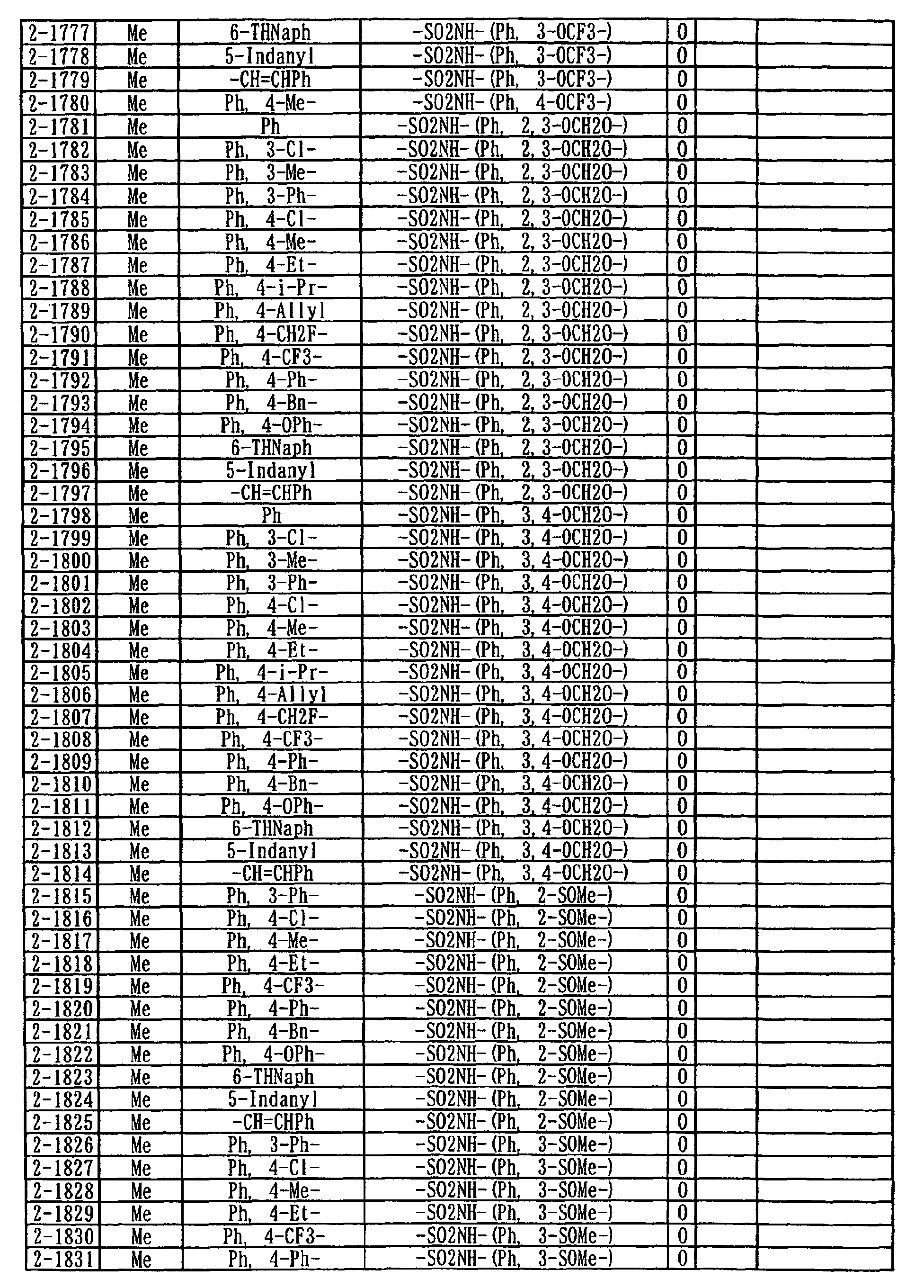

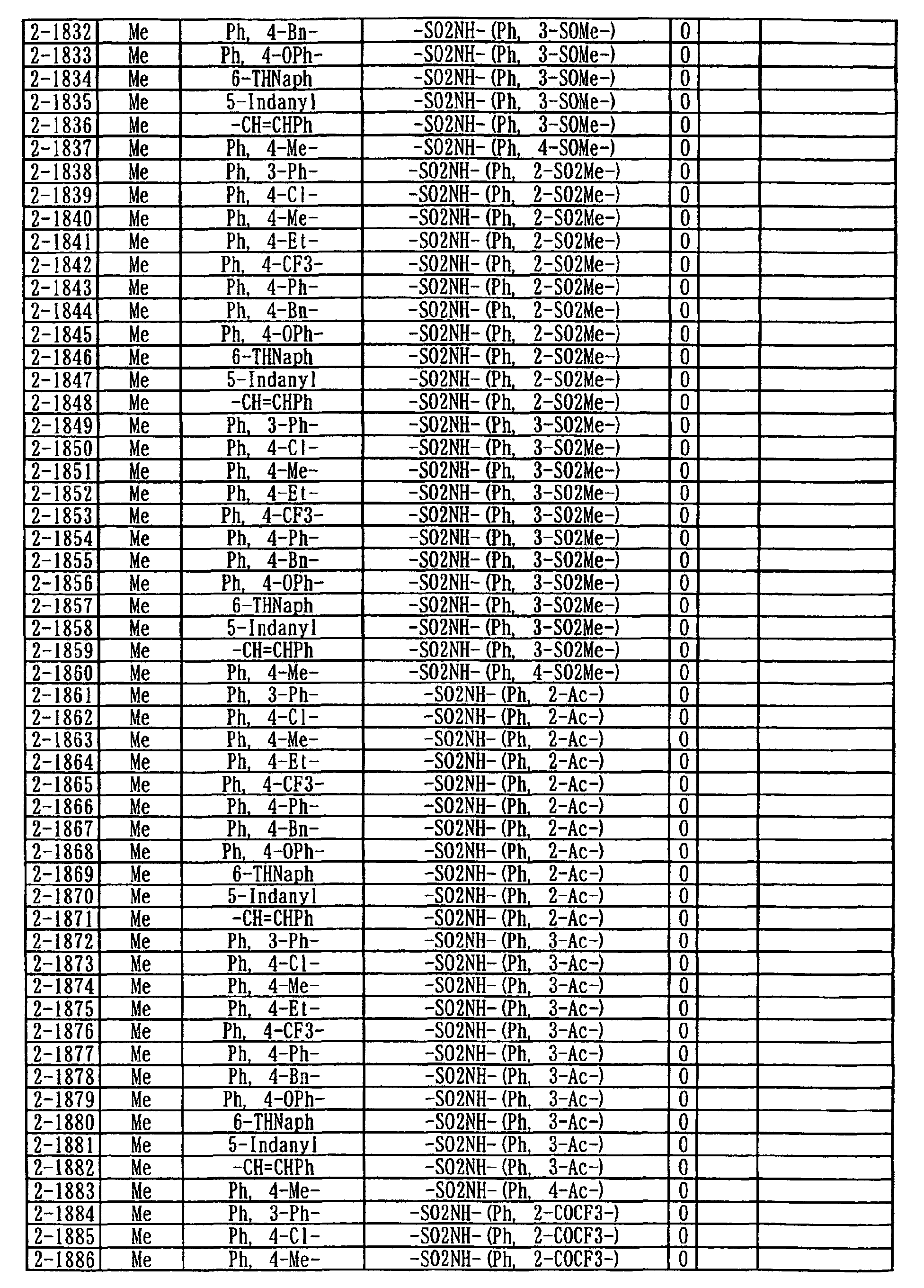

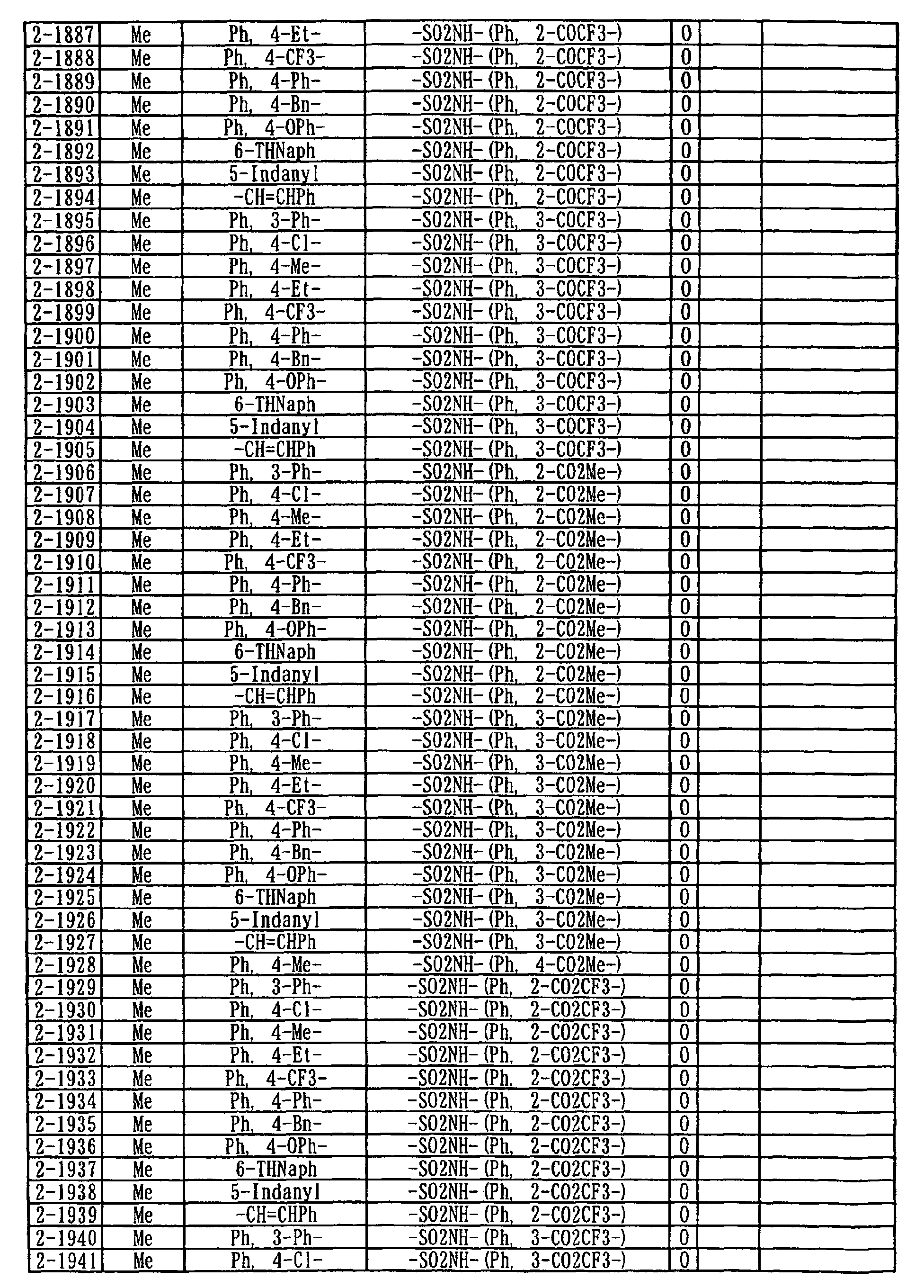

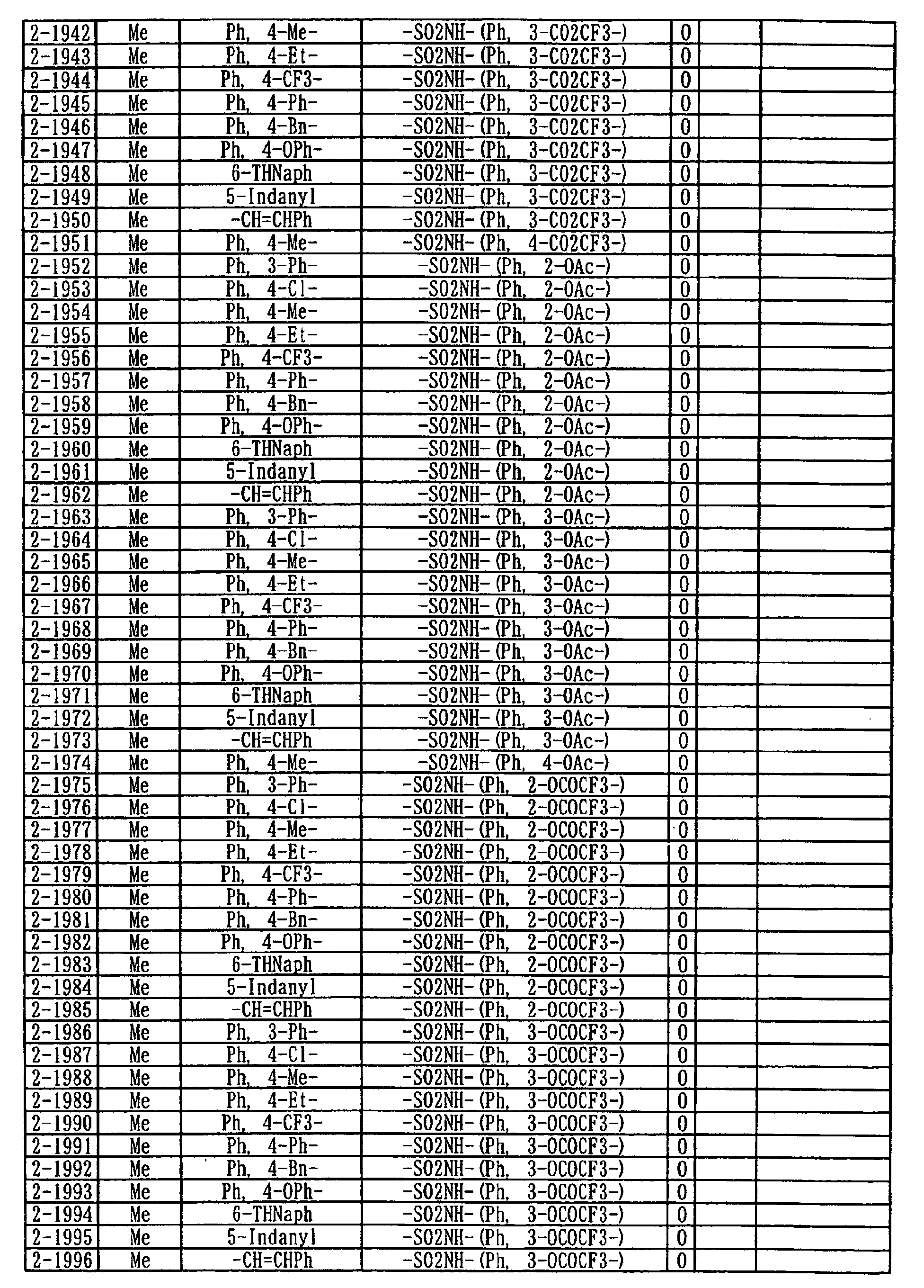

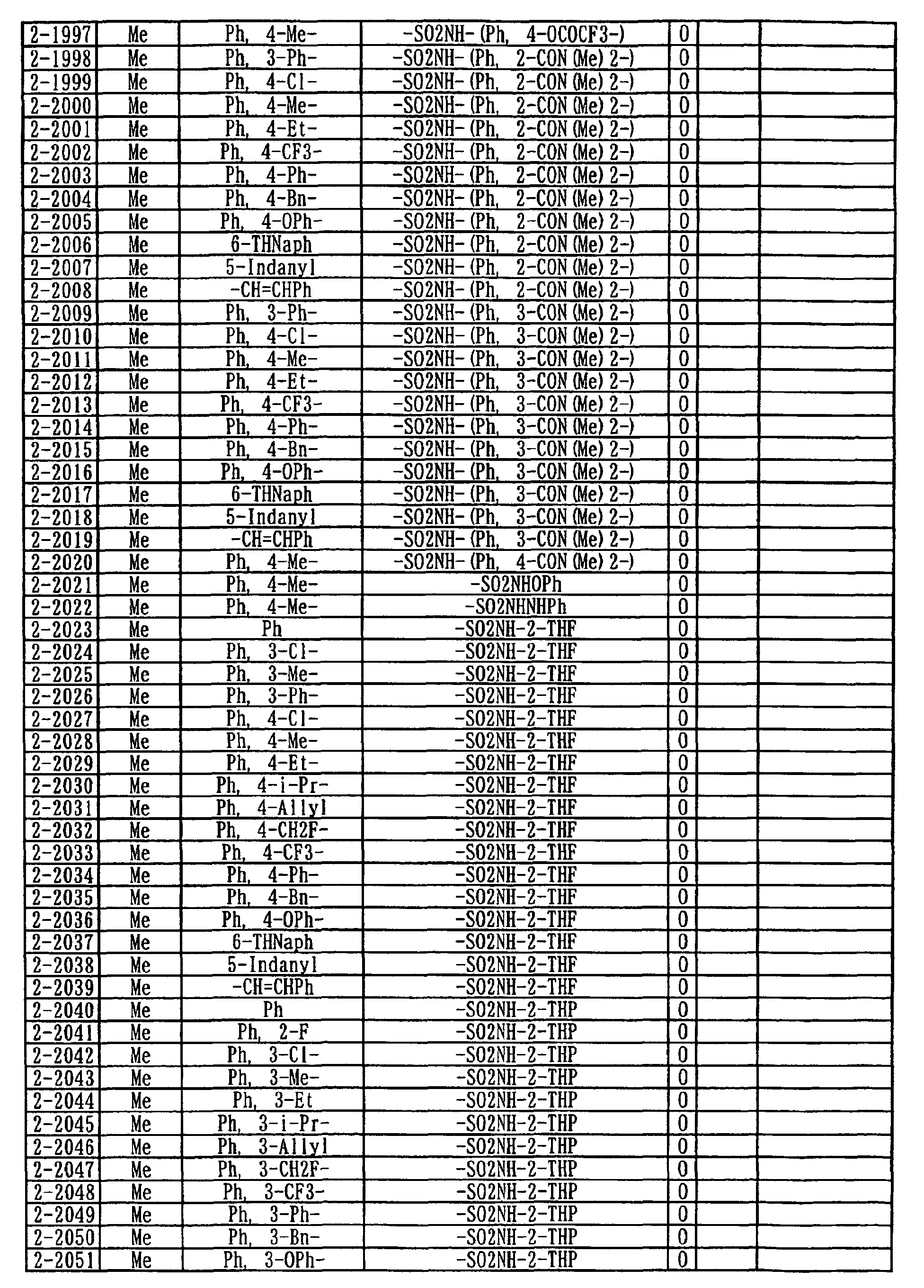

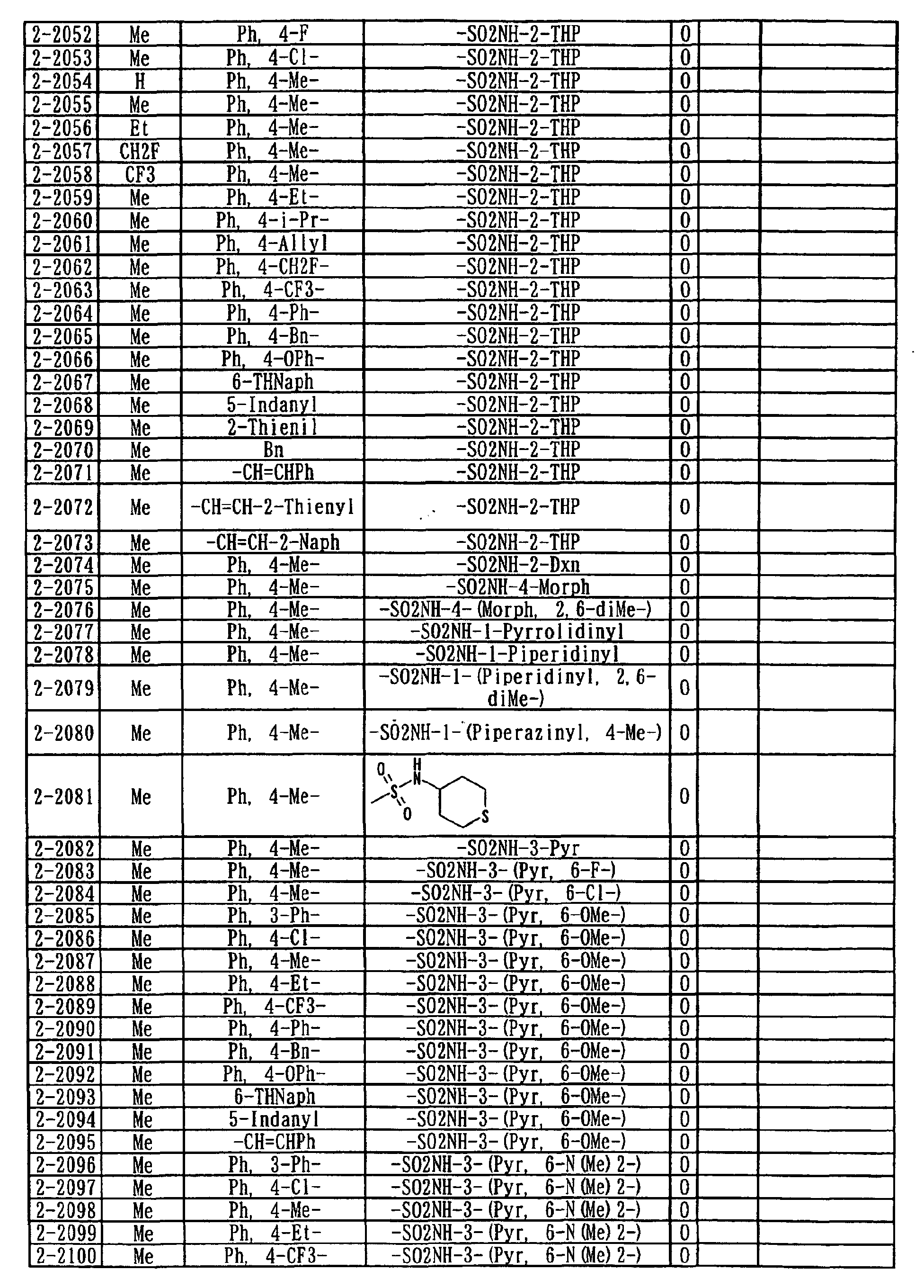

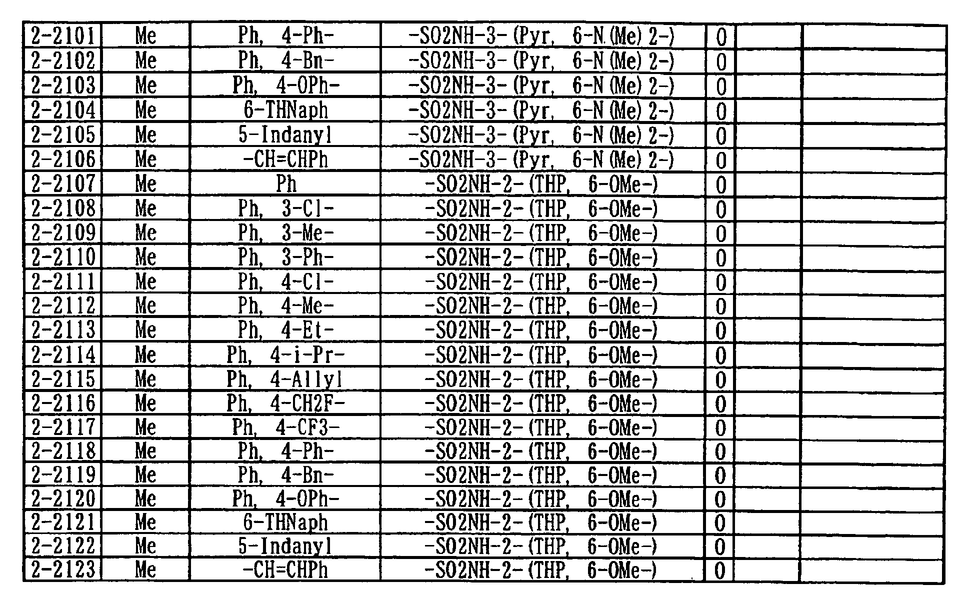

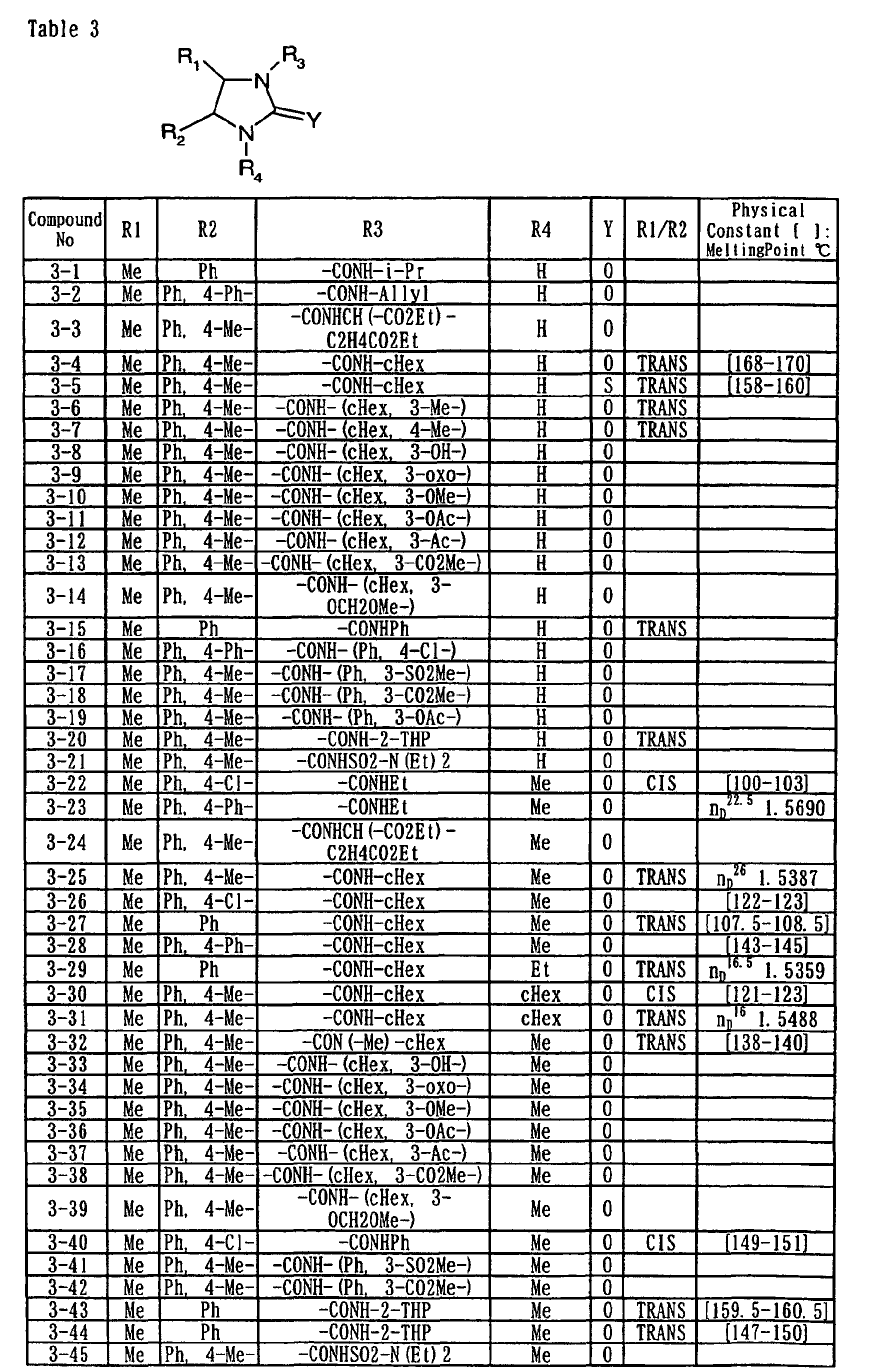

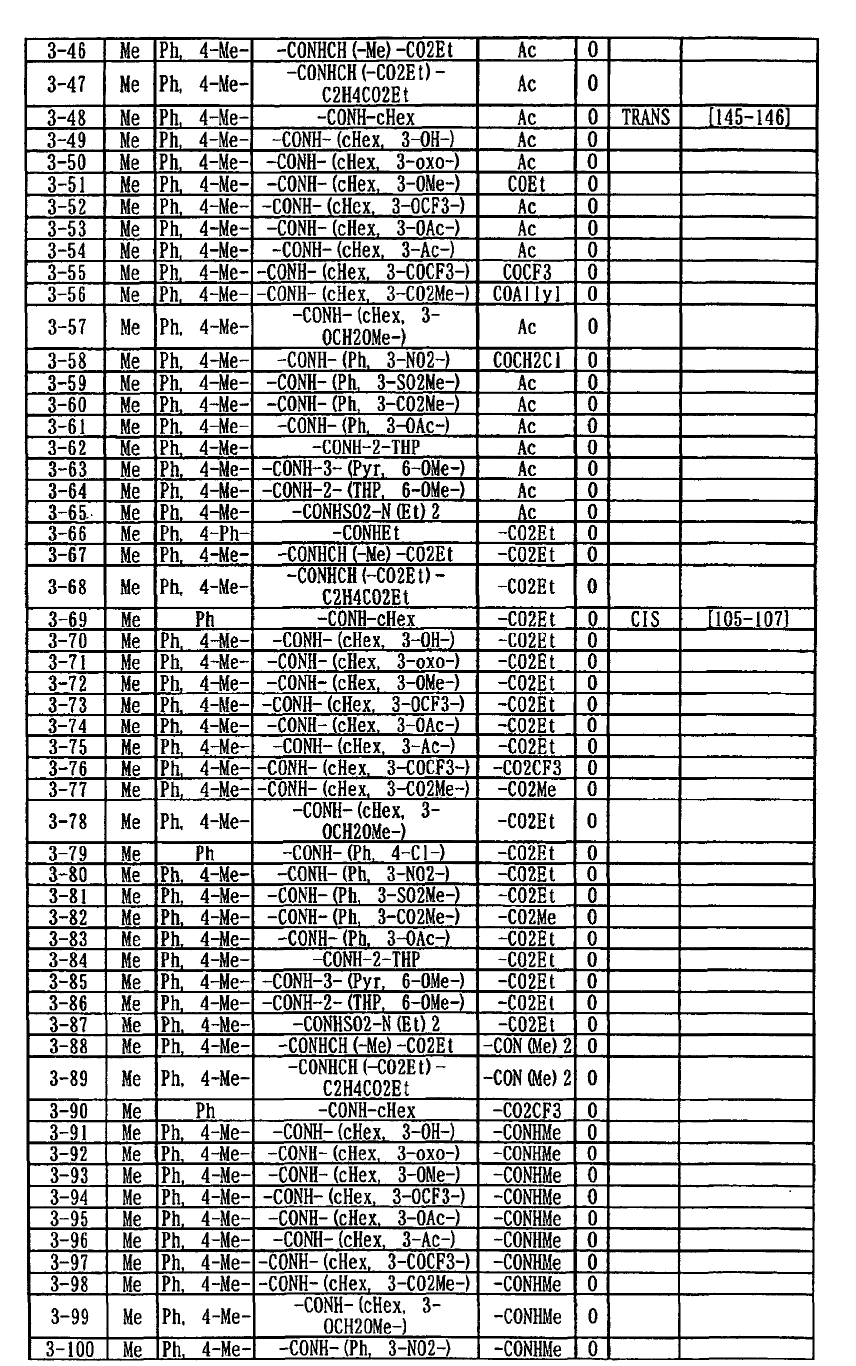

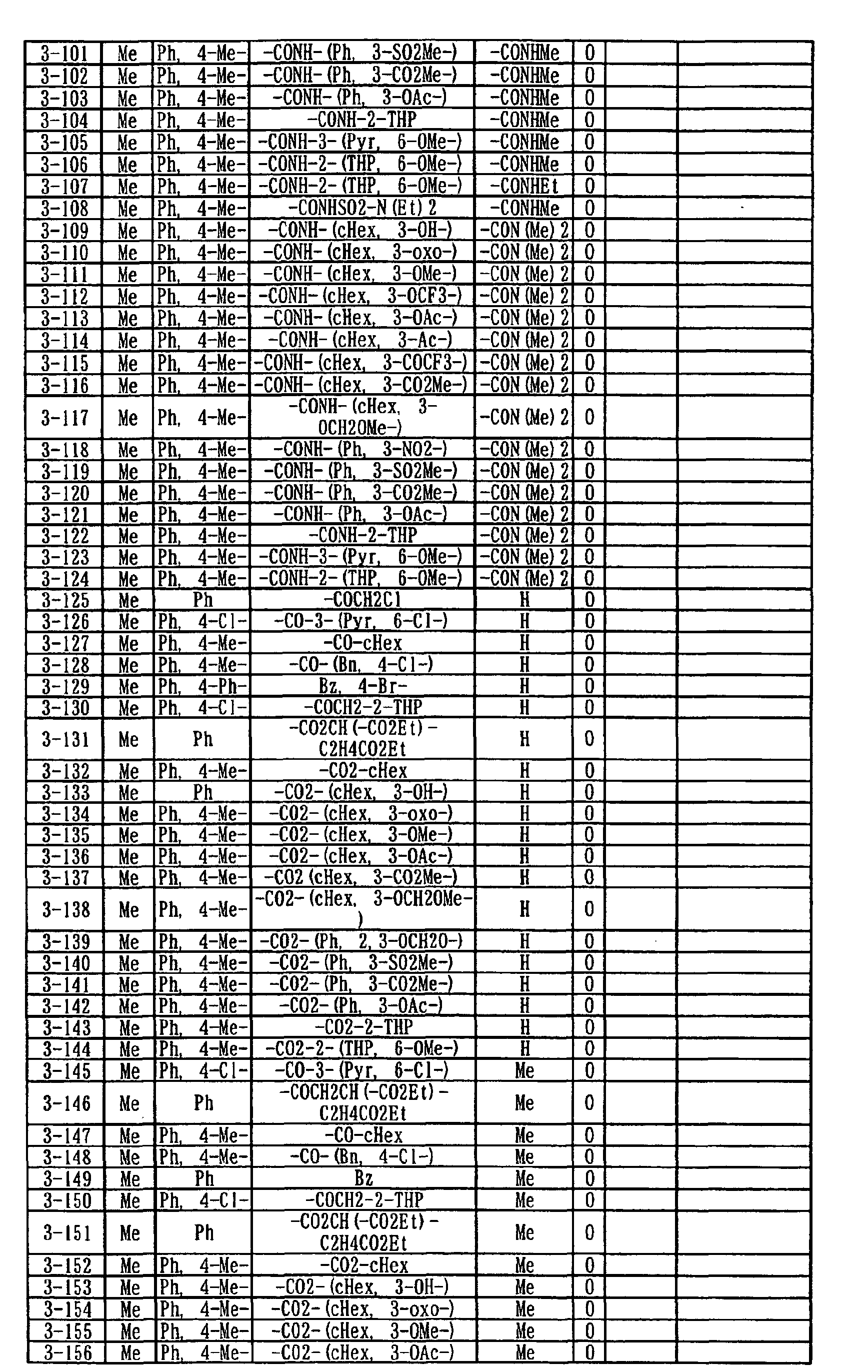

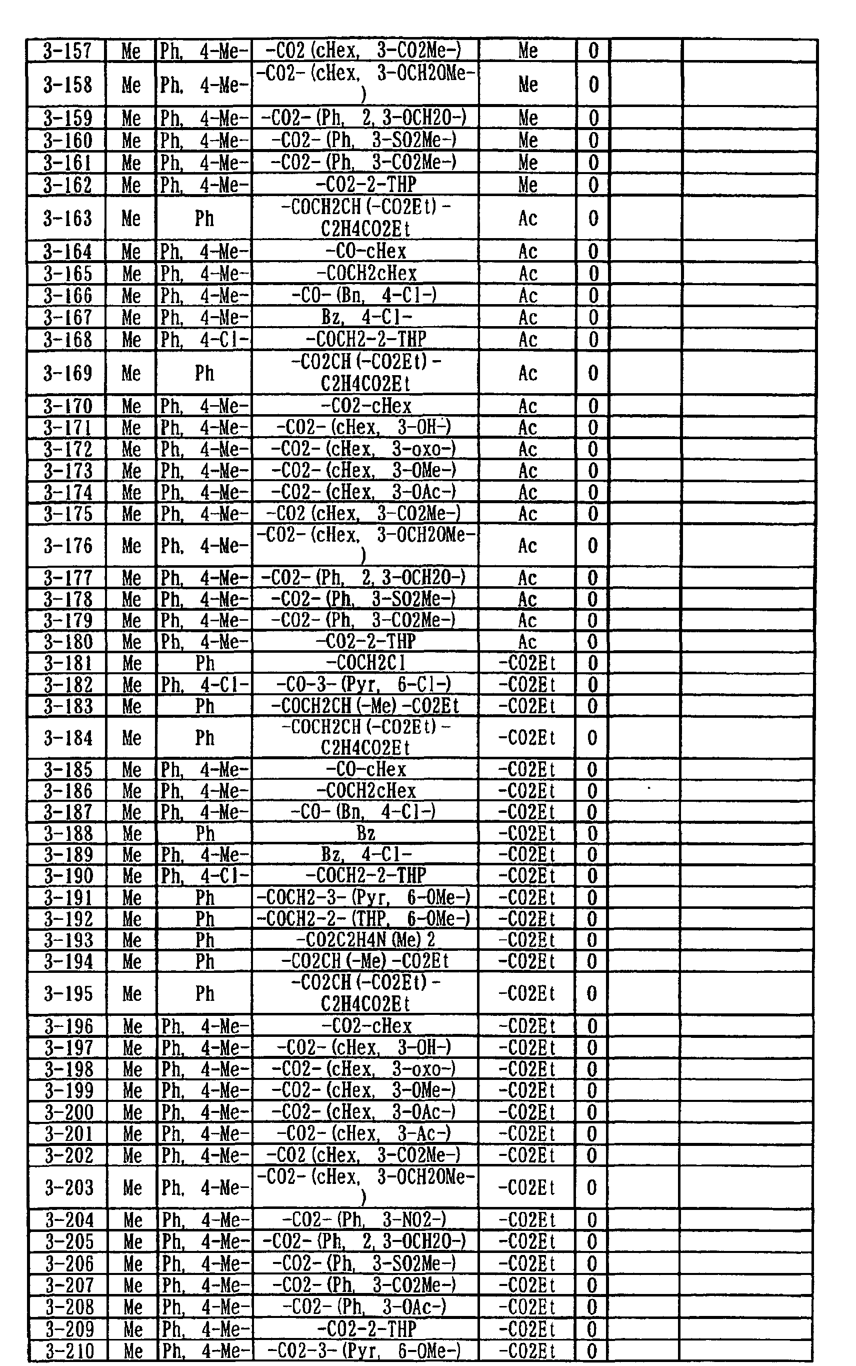

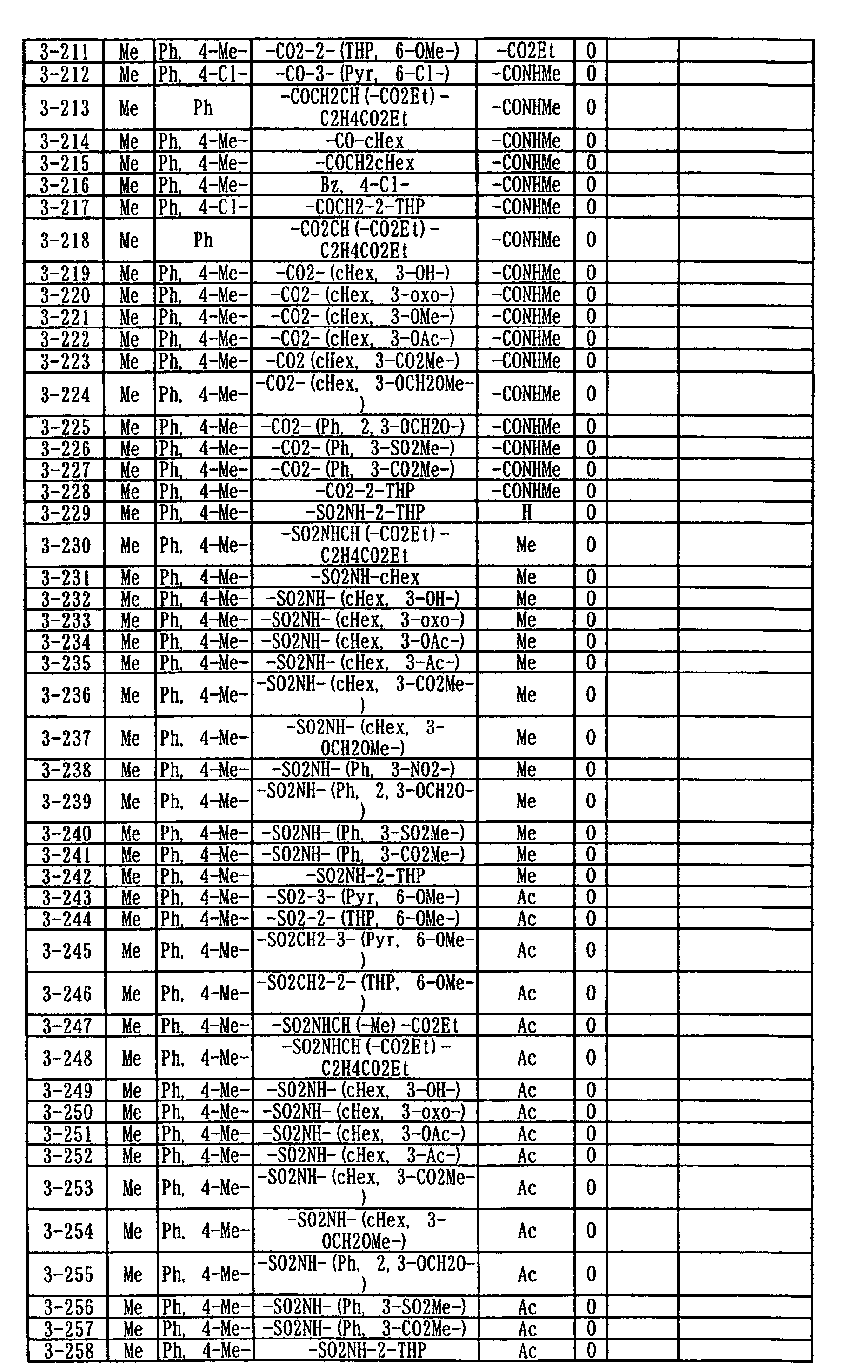

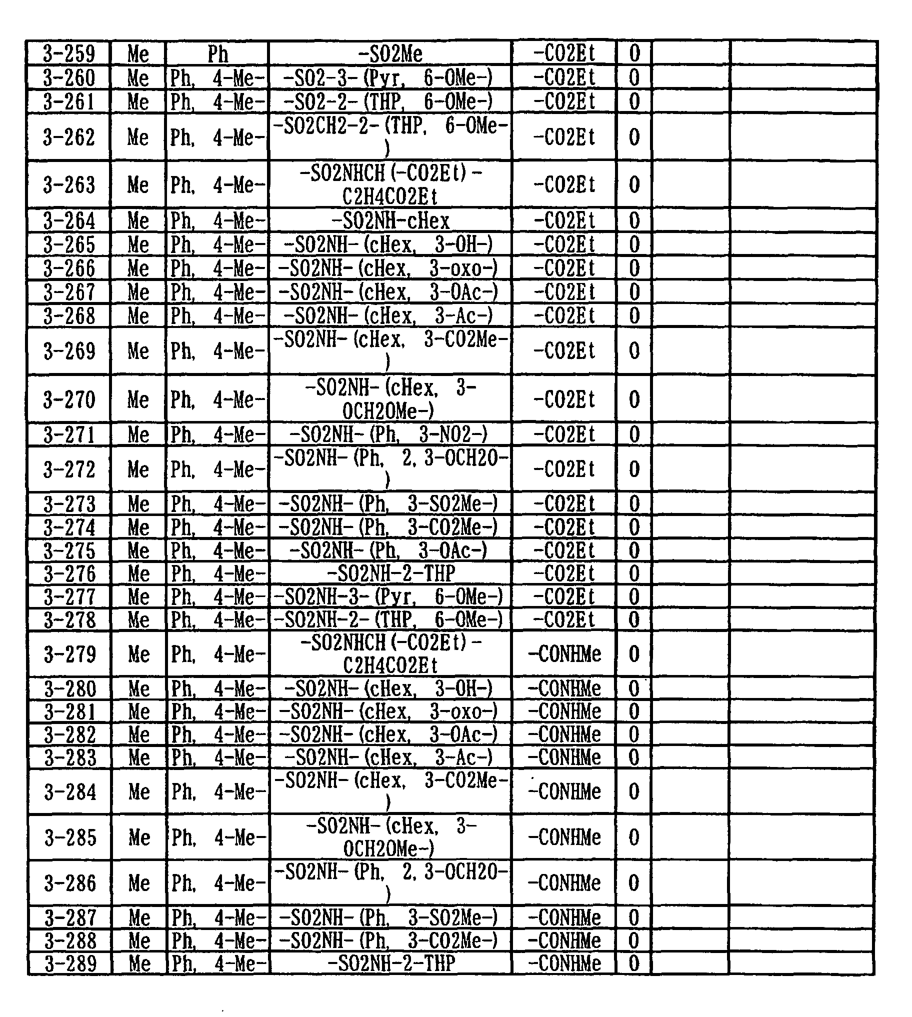

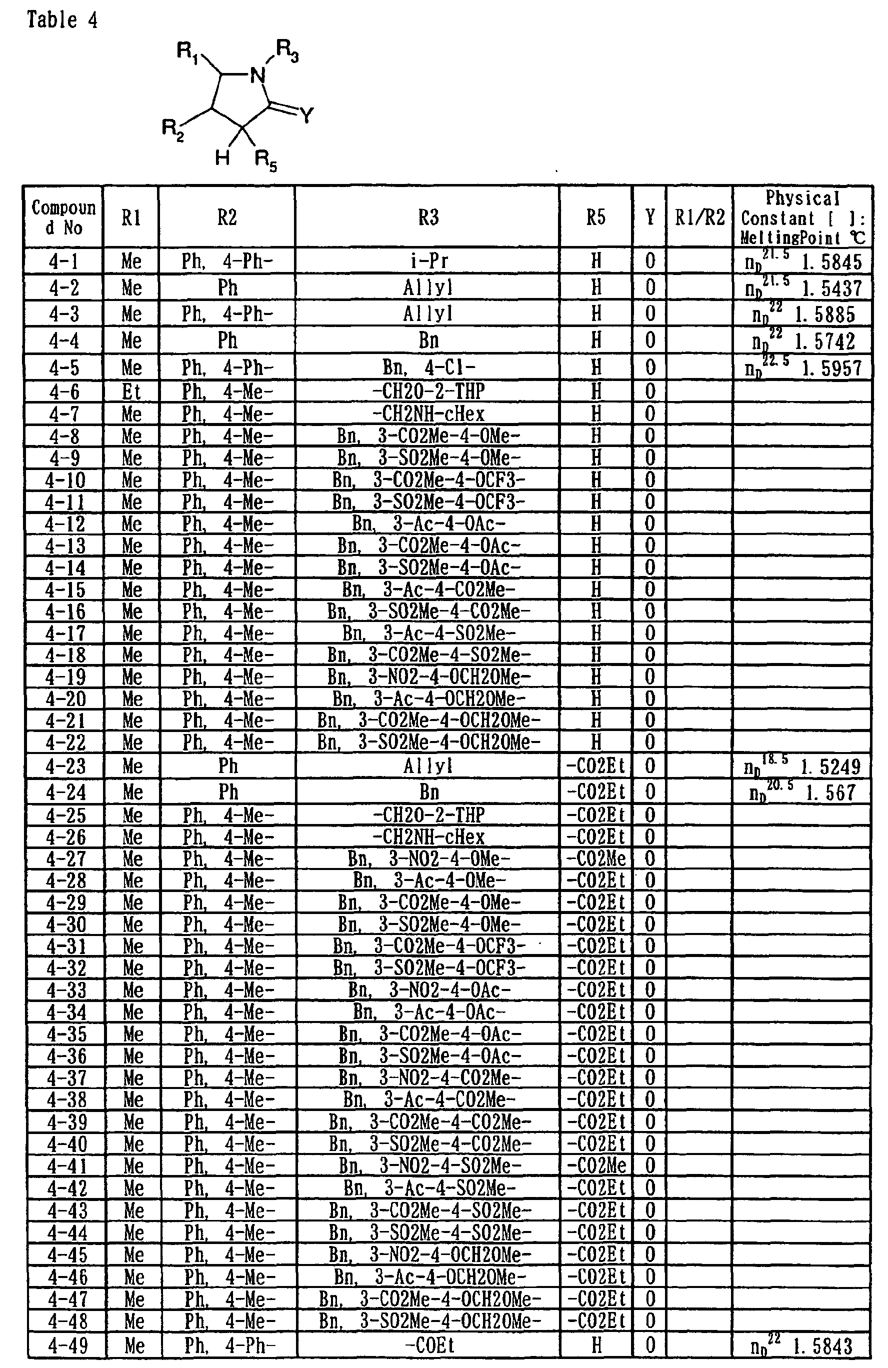

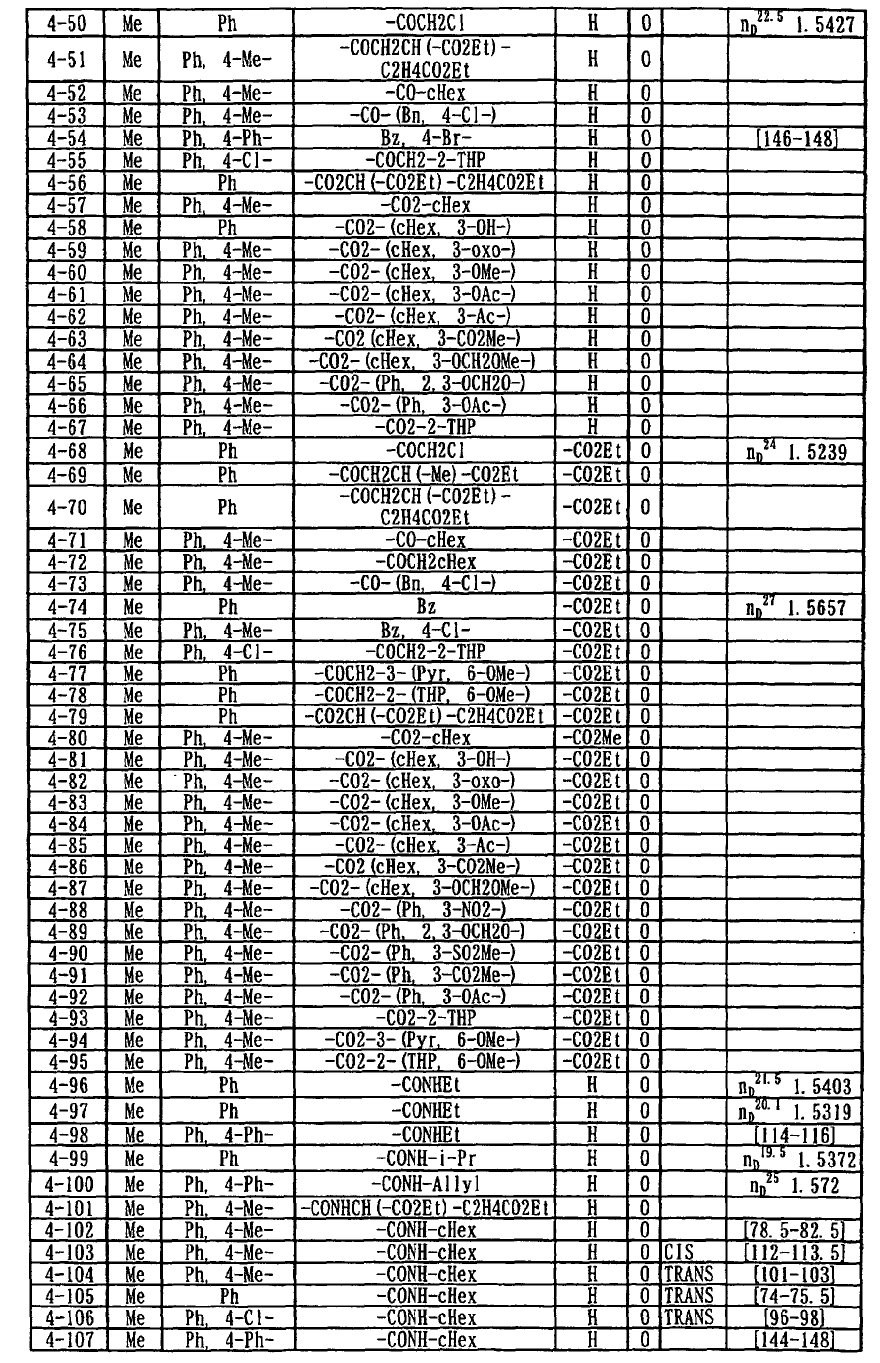

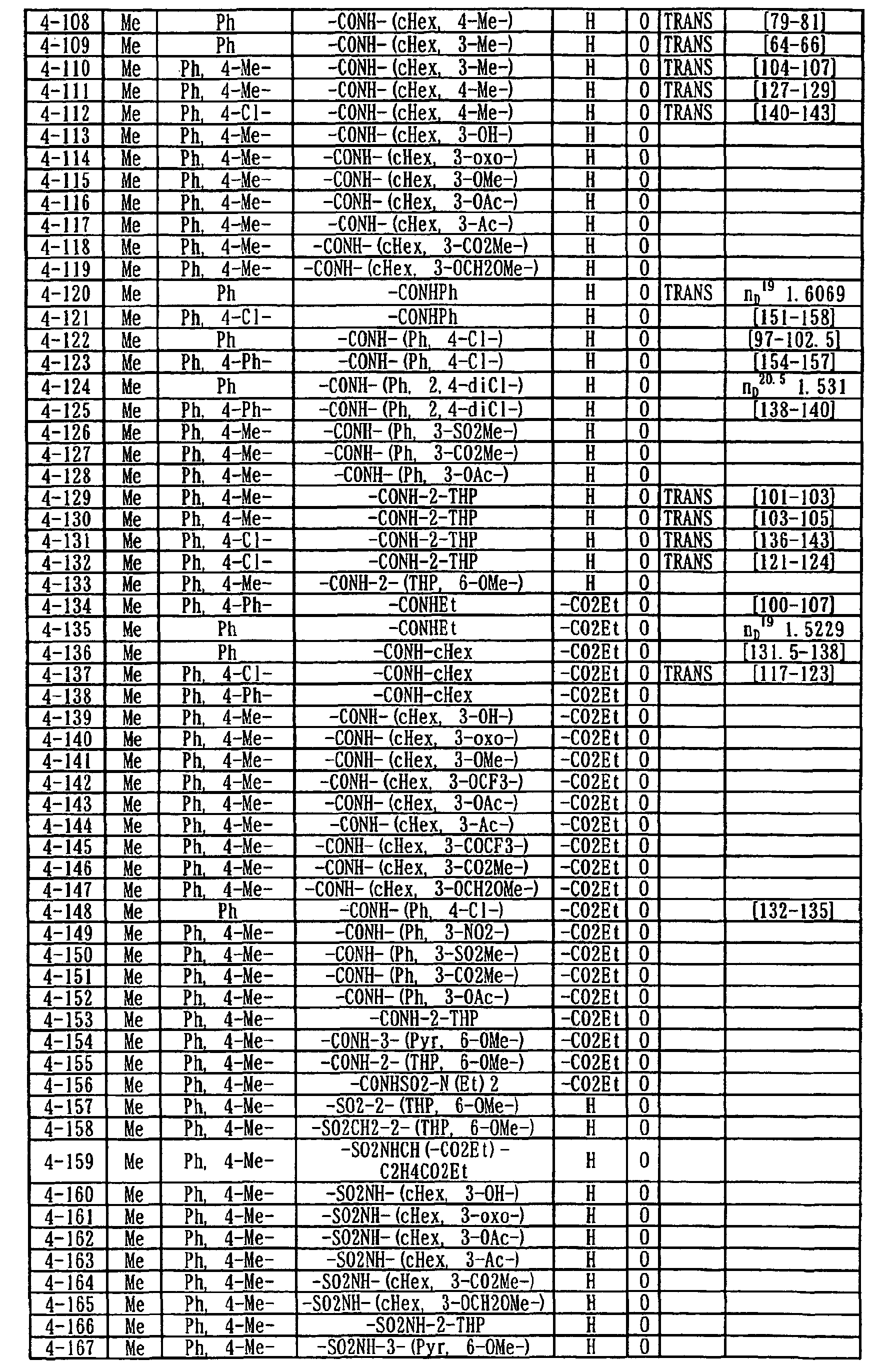

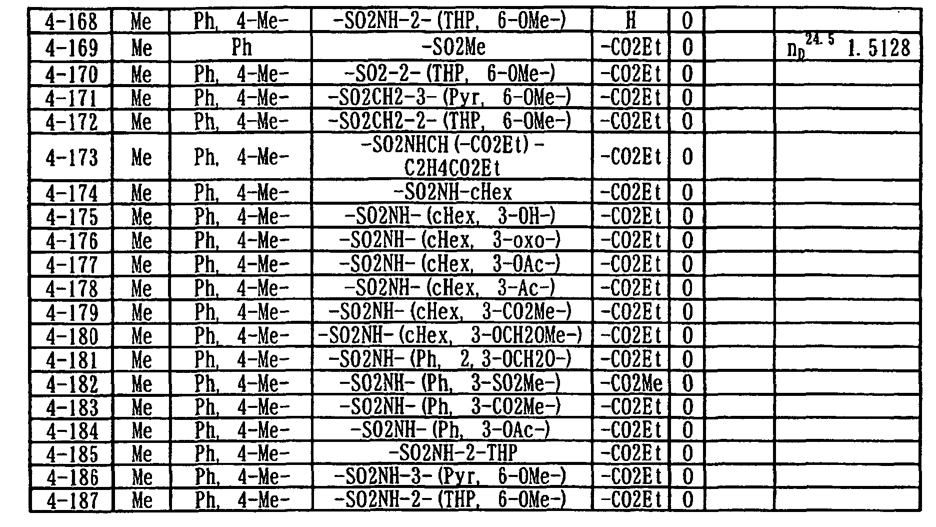

Examples of pharmaceutical formulations for the medicinal compositions of the present invention are described.

### Pharmaceutical Formulation Example 1: Tablet

| Composition | Amount (g) |
|---|---|
| Compound of the present invention | 5 |
| Lactose (The Pharmacopoeia of Japan) | 50 |
| Corn starch (The Pharmacopoeia of Japan) | 25 |
| Crystalline cellulose (The Pharmacopoeia of Japan) | 25 |
| Methyl cellulose (The Pharmacopoeia of Japan) | 1.5 |
| Magnesium stearate (The Pharmacopoeia of Japan) | 1 |

The compound of the present invention, lactose, com starch and crystalline cellulose were sufficiently mixed, and granulated with a 5% aqueous solution of methyl cellulose. The granules were passed through a 200-mesh sieve and carefully dried. The dried granules were mixed with magnesium stearate and made tablets according to a conventional method to give 1,000 tablets.

### Pharmaceutical Formulation Example 2: Capsule

| Composition | Amount (g) |
|---|---|
| Compound of the present invention | 10 |
| Lactose (The Pharmacopoeia of Japan) | 80 |
| Starch (The Pharmacopoeia of Japan) | 30 |
| Talc (The Pharmacopoeia of Japan) | 5 |
| Magnesium stearate (The Pharmacopoeia of Japan) | 1 |

The above ingredients were pulverized finely and sufficiently stirred to make a homogeneous mixture, which was then filled in gelatin capsules of a desired size for oral administration to prepare 1,000 of 2-piece gelatin capsules.

### Pharmaceutical Formulation Example 3: Solution for Injection

| Composition | Amount (g) |
|---|---|
| Compound of the present invention | 1 |
| Polyethylene glycol 4000 (The Pharmacopoeia of Japan) | 0.3 |
| Sodium chloride (The Pharmacopoeia of Japan) | 0.9 |
| Polyoxyethylene sorbitan monooleate (The Pharmacopoeia of Japan) | 0.4 |
| Sodium metabisulfite (The Pharmacopoeia of Japan) | 0.1 |
| Methylparaben (The Pharmacopoeia of Japan) | 0.18 |
| Propylparaben (The Pharmacopoeia of Japan) | 0.02 |
| Distilled water for injection use | Appropriate |
| (Final volume) | 100 (mL) |

The parabens, sodium metabisulfite and sodium chloride were dissolved in distilled water for injection use of an amount about half of the final volume with stirring at 80°C. The obtained solution was cooled down to 40°C. The compound of the present invention, then polyethylene glycol and polyoxyethylene sorbitan monooleate were dissolved in the solution. The remaining distilled water was added to the solution to make the final volume. The resulting solution was filtrated and sterilized through an appropriate filter to give an aqueous solution of a pharmaceutical formulation suitable for parenteral use.

### Pharmaceutical Formulation Example 4: Ointment

| Composition | Amount (g) |
|---|---|
| Compound of the present invention | 0.1 |
| White soft paraffin | 10 |

The compound of the present invention was blended in the base material so as to be homogeneous.

### Pharmaceutical Formulation Example 5: Aerosol

| Composition | Amount (g) |
|---|---|
| Compound of the present invention | 0.25 |
| Ethanol | 29.75 |
| Propellant 22 (chlorodifluoromethane) | 70 |

The compound of the present invention was mixed with ethanol, and further a part of Propellant 22 was added to blend. The resulting mixture was cooled down to -30°C and filled into a filling machine. An amount of the mixture requited for an administration was filled in a stainless steel container and diluted with the remaining Propellant 22. A valve unit was mounted on the container to be ready for the administration.

### Pharmaceutical Formulation Example 6: Dry powder for inhalation

| Composition | Amount (g) |
|---|---|
| Compound of the present invention | 5 |
| Lactose | 95 |

The compound of the present invention was mixed with lactose homogeneously. The mixture was filled in a dry powder inhaler.

### Pharmaceutical Formulation Example 7: Suppository

| Composition | Amount (g) |
|---|---|
| Compound of the present invention | 0.225 |
| Saturated fatty acid glyceride | 2.000 |

The compound of the present invention was passed through a No. 60 mesh U.S. sieve, and suspended into the saturated fatty acid glyceride that was melted beforehand with minimum required heat. The mixture was poured into a suppository mold with an indication capacity of 2g and then cooled.

### Industrial Use:

### Pharmacological Test Example 1: PLA (2) Activity

The PLA (2) activity was measured by a quantitative analysis of the fluorescent product produced by hydrolysis of 1,2-bis-(4,4-difluoro-5,7-dimethyl-4-bora-3a,4a-diaza-*s*-indecene-3-undecanoyl)-*sn*-glycero-3-phosphocholine (hereinafter referred to as bis-BODIPY®FL C₁₁-PC; Molecular Probes Inc., B-7701) using U937 (human monoblastic lymphoma cell line) activated by inflammatory stimuli [Meshulam, T., et al., *The Journal of Biological Chemistry* **267** (30): 21465-21470 (1992) and Solito, E., et al., *British Journal of Pharmacology* **124**: 1675-1683 (1998)]. The bis-BODIPY®FL C₁₁-PC, used as a substrate, is taken into the cell membrane, and the BODIPY®FL fluorescent probe is present in the state of self-quenching. A fluorescent fatty acid chain dissipates strong fluorescence when it is hydrolyzed and liberated by PLA (1) or PLA (2). It has been demonstrated that, in U937 activated by inflammatory stimuli, the substrate is enzymatically cleaved mainly by IV-cPLA (2) based on the feature of its behavior including sensitivity profiles against inhibitors.

The human cell line U937, purchased from Dai-Nippon Pharmaceuticals Co., Ltd., was subcultured by transferring every 3 to 4 days into an RPMI 1640 medium (Sigma Chemical Co., Ltd., R 6504) supplemented with inactivated 10% fetal bovine serum (Fetal Bovine Serum, Sigma Chemical Co., Ltd., F 4135) in a humidified atmosphere of 5% CO₂ at 37°C. Cells to provide for an assay were transferred into the aforementioned subculture medium supplemented with 1.2% (v/v) dimethyl sulfoxide (hereinafter referred to as DMSO, Nacalai Tesque Co., Ltd., D 134-45), and cultured for 96 to 120 hours. The cells were differentiated into macrophage-like cells, which were then collected and washed by centrifugation with an assay medium (Dulbecco's phosphate buffered saline (hereinafter referred to as PBS)-2.2 mM glucose-2.5 µM albumin). Phorbol 12-myristate 13-acetate (hereinafter referred to as TPA, Sigma Chemical Co., Ltd., P 8139) was added to the assay medium to make the final concentration 1x10⁻⁸M. The macrophage-like cells were further cultured for an hour for activation [Rzigalinski, B.A. and Rosenthal, M.D., *Biochemica et Biophysica Acta* **1223**: 219-225 (1994) and Gonchar, M.V., et al., *Biochemical and Biophysical Research Communication* **249**: 829-832 (1998)].

A substrate liposome suspension was prepared in a way that bis-BODIPY®FL C₁₁-PC was blended with phosphatidylserine (Sigma Chemical Co., Ltd., P 7769) in chloroform at a molar ratio of 1:9; the organic solvent was distilled off to dry the mixture under nitrogen flow; the assay medium was added to the mixture to make 100 µg/mL; and the resulting suspension was sonicated for an hour with light shielded while cooling with ice.

A test compound was dissolved in DMSO to make 30 mM, then diluted with DMSO or the assay medium, and added to a reaction system. The DMSO concentration in the reaction system was adjusted to be not more than 0.1%. A solution of the compound of a concentration 30 times higher than the final concentration was put at a concentration of 2.5 µL/well into each well of a 96-wells microplate (Falcon, 3072). The suspension of 6 x 10⁶ cells/mL of the activated U937 was added at a concentration of 25 µL/well to each of the wells. The cells were pre-cultured for 10 minutes in a humidified atmosphere of 5% CO₂ at 37°C. A substrate liposome suspension, 1.5x10⁻⁶M A23187, (Sigma Chemical Co., Ltd., C 7522) was added at a concentration of 47.5 µL/well to each of the wells to mix so that the total of 75 µL/well of a reaction solution was prepared. After light was shielded, the cells were cultured for 30 minutes under the same conditions. The reaction was terminated by adding 100 µL/well of a 0.1% methanol solution of GEDTA (Dojindo Laboratories Co., Ltd., 348-01311) to each well to mix with the reaction solution. The fluorescent intensity of the enzymatic hydrolysis product was measured at 37°C by a microplate reader, SPECTRA FLUOR PLUS (TECAN Austria GmbH) at the top reading with the excitation at 485 nm and emission at 538 nm. The measurements of the lots of the same test were carried out by setting the optimum gain for the first microplate as the common sensitivity. Each test was performed in triplicate. A reaction mixture plot without cells was used as the blank and arachidonyl trifluoromethyl ketone (hereinafter referred to as AACOCF₃, Calbiochem Co., Ltd., 100109) as the positive control. The activity of PLA (2) in each test plot was determined by subtracting the mean value of the fluorescent intensities of the blank plot from the fluorescent intensity of each well. There was no significant statistic difference between the plots with or without 0.1% DMSO. In the pre-examination, the fluorescent intensity based on the substrate hydrolysis was increased linearly with time progression up to 90 minutes. An enzymatic activity was measured as the basic metabolic activity, using subcultured cells that were not differentiated nor activated, without A23187. In this case, the hydrolysis activity was observed about one seventh that of the activated cells. Thus, the difference obtained by subtracting the basic metabolic activity from each enzymatic activity was determined as the inflammation activated PLA (2) activity. Each compound was evaluated for its activity by an inhibition rate on the inflammation activated PLA (2) activity, using the mean value of the non-treated and DMSO-treated plots. Some of the inhibitory activities measured for the test compounds of the present invention are shown in Table 5.

**Table 5**

| Compound No. | Concentration (µM) | Inhibition (%) |
|---|---|---|
| 1-24 | 1 | 91 |
| 1-84 | 1 | 74 |
| 1-91 | 1 | 82 |
| 1-525 | 0.1 | 100 |
| 1-643 | 0.1 | 74 |
| 1-702 | 1 | 81 |
| 1-711 | 1 | 70 |
| 1-1021 | 0.1 | 87 |
| 1-1248 | 1 | 97 |
| 1-1470 | 1 | 87 |
| 2-9 | 0.1 | 75 |
| 2-10 | 0.1 | 78 |
| 2-14 | 0.1 | 63 |
| 2-17 | 0.1 | 88 |
| 2-87 | 1 | 92 |
| 2-91 | 0.1 | 68 |
| 2-98 | 0.1 | 83 |
| 2-134 | 1 | 72 |
| 2-303 | 0.1 | 72 |
| 2-488 | 1 | 69 |
| 2-494 | 1 | 68 |
| 2-647 | 1 | 73 |
| 3-26 | 0.1 | 89 |
| 3-43 | 1 | 84 |
| 3-69 | 0.1 | 76 |
| 4-104 | 0.1 | 68 |
| 4-130 | 0.1 | 100 |
| 4-137 | 0.1 | 95 |
| AACOCF₃ | 3 | 65 |

### Pharmacological Test Example 2: Mouse Ear Edema Induced by TPA

This test was carried out referring to the methods of Carlson, R.P., et al. [*Agents and Actions* **17** (2): 197-204 (1985)] and of Chang, J., et al. [*European Journal of Pharmacology* **142**: 197-205 (1987)]: 5µg/20µL of TPA (Sigma Chemical Co., Ltd.) dissolved in ethanol was applied on the anterior and posterior surfaces of the right ear of an ICR-strain male mouse (6 to 7 weeks old). The thickness of a specific area of each of the ears was measured 3 times by a digimatic micrometer 6 hours after the application. The mean values were calculated. Ear edema was determined by subtracting the mean thickness of the non-treated left ear from that of the TPA-applied right ear. A topical application activity was evaluated in a way that an acetone solution or a 0.1% Tween 80/acetone solution of a compound of the present invention was applied on the anterior and posterior surfaces of the right ear 30 minutes before and 15 minutes after the application of TPA. As the positive control, an acetone solution of dexamethasone-21-acetate (hereinafter referred to as DEX-Ac, Sigma Chemical Co., Ltd., D 1881) or indomethacin (Nacalai Tesque Co., Ltd., 192-33) was applied in the same manner as that for the compound of the present invention. An oral administration activity was evaluated by forced oral administration of a suspension of a compound of the present invention in 2% Tween 80/distilled water an hour before applying TPA. As the positive control, a suspension of hydrocortisone (Sigma Chemical Co., Ltd., H 4001) was administered in the same manner as that for the compound of the present invention. Some of the compounds of the present invention had anti-inflammatory activities shown in Table 6. It was observed 24 hours after TPA was applied that the groups of mice administered with DEX-Ac or indomethacin were in bad shape with significant body weight loss. On the contrary, the mice treated with compounds of the present invention were fit and no significant changes in their body weights were observed.

**Table 6**

| Compound No. | Dose (mg/µL/ear x 2) | Ear edema inhibition (%) |
|---|---|---|
| 1-91 | 1 mg/40 µL | 41.8 |
| 1-649 | 1 mg/40 µL | 48.1 |
| 1-1021 | 1 mg/40 µL | 63.8 |
| 2-17 | 1 mg/40 µL | 30.3 |
| 2-91 | 1 mg/40 µL | 46.9 |
| 2-98 | 1 mg/40 µL | 31.3 |
| 2-134 | 1 mg/40 µL | 57.7 |
| 2-303 | 1 mg/40 µL | 73.3 |
| 3-69 | 1 mg/40 µL | 76.4 |
| DEX-Ac | 1 mg/20 µL | 79.6 |
| Indomethacin | 3 mg/40 µL | 63.5 |

### Pharmacological Test Example 3: Acetic Acid Writhing

This test was carried out referring to the methods of Inoue, K, Motonaga, A. and Nishimura, T. [*Arzneimittel Forshung*/*Drug Research* **41** (1): 235-239 (1991)] and of Murata, T., et al. [*Nature* **388**: 678-682 (1997)]: 7.5 mL/kg of a 0.9% aqueous solution of acetic acid was injected intraperitoneally into an ICR-strain male mouse (6 to 8 weeks old). The number of induced writhes (characteristic behavior of contracting the abdomen, twisting the body and/or extending the hind legs in agony) was counted in a period between 10 minutes and 20 minutes after the injection. A compound of the present invention was homogeneously suspended in 2% Tween 80/saline for injection use and injected intraperitoneally 30 minutes before the induction of stimulation by the acetic acid injection. Alternatively the compound was homogeneously suspended in 2% Tween 80/distilled water and orally administered an hour before the stimulation induction by the acetic acid injection. The analgesic activity of the compound of the present invention was evaluated on the basis of how many writhes were suppressed by the administration. Indomethacin or aspirin was administered as the positive control. Some of the compounds of the present invention had the analgesic activities shown in Table 7.

**Table 7**

| Compound No. | Application route | Dose (mg/kg) | Application time (minutes before) | No. of writhes (mean ± S.E.) |
|---|---|---|---|---|
| Vehicle control | i.p. | - | 30 | 25.2 ± 2.2 |
| 1-1021 | i.p. | 1 | 30 | 5.3 ± 2.5 |
| 2-303 | i.p. | 1 | 30 | 6.5 ± 2.7 |
| 3-69 | i.p. | 1 | 30 | 10.0 ± 2.8 |
| Indomethacin | i.p. | 10 | 30 | 4.8 ± 0.9 |
| Indomethacin | i.p. | 3 | 30 | 13.8 ± 3.0 |
| Aspirin | i.p. | 30 | 30 | 9.5 ± 2.0 |

The above test results obviously show that the compounds of the present invention are excellent inhibitors of the PLA (2) activity, are less toxic, and have strong anti-inflammatory and/or analgesic activities. Thus, a composition containing a compound of the present invention as an active ingredient relieves symptoms of diseases accompanied with the enhanced PLA (2) activity and is very efficient to treat relevant diseases so as to be useful as a therapeutic or preventive drug of a new type that has not been known yet.

## Claims

1. A compound represented by Formula [I] 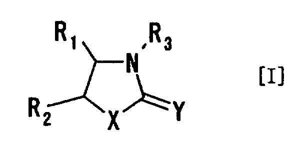 [wherein, X represents oxygen, sulfur, NR₄ or CHR₅;
Y represents oxygen or sulfur;
R₁ represents C₁₋₆ alkyl or C₁₋₆ haloalkyl;
R₂ represents phenyl, naphthyl, indanyl, tetrahydronaphthyl, thienyl, phenyl C₁₋₆ alkyl, phenyl C₂₋₆ alkenyl thienyl C₂₋₆ alkenyl or naphthyl C₂₋₆ alkenyl, and the rings of these groups are optionally substituted with one or more, same or different, groups represented by A,;
R₃ represents C₁₋₆ alkyl optionally substituted with A₂, C₂₋₆ alkenyl optionally substituted with A₂ or one of the groups represented by the following formulae
CZR₆, S(O)mR₇, 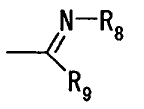
R₄ represents hydrogen, C₁₋₆ alkyl, C₃₋₇ cycloalkyl, C₁₋₆ alkylcarbonyl, C₁₋₆ haloalkylcarbonyl, C₁₋₆ alkenylcarbonyl, C₁₋₆ alkoxycarbonyl, C₁₋₆ haloalkoxycarbonyl, or mono- or di-C₁₋₆ alkylcarbamoyl;
R₅ represents hydrogen or C₁₋₆ alkoxycarbonyl;
R₆ represents C₁₋₆ alkyl optionally substituted with A₂, C₁₋₆ alkoxy optionally substituted with A₂, C₂₋₆ alkenyl optionally substituted with A₂, C₃₋₇ cycloalkyl optionally substituted with A₃, C₃₋₇ cycloalkoxy optionally substituted with A₃, C₃₋₇ cycloalkenyl optionally substituted with A₃, C₃₋₇ cycloalkenyloxy optionally substituted with A₃, phenyl optionally substituted with A₄, a saturated or unsaturated 5- or 6-membered heterocyclic group having at least a hetero atom selected from oxygen, sulfur and nitrogen and optionally substituted with A₃, or a group represented by Formula NR₁₀R₁₁;
R₇ represents C₁₋₆ alkyl optionally substituted with A₂, C₂₋₆ alkenyl optionally substituted with A₂, C₃₋₇ cycloalkyl optionally substituted with A₃, a saturated or unsaturated 5- or 6-membered heterocyclic group having at least a hetero atom selected from oxygen, sulfur and nitrogen and optionally substituted with A₃, or a group represented by Formula NR₁₂R₁₃;
R₈ represents cyano, nitro, C₁₋₆ alkyl or C₃₋₇ cycloalkyl;
R₉ represents C₁₋₆ alkyl, C₁₋₆ alkylthio, C₁₋₆ alkylamino, C₃₋₇ cycloalkylamino or morpholino;
R₁₀ and R₁₂ represent hydrogen or C₁₋₆ alkyl;
R₁₁ and R₁₃ represent C₁₋₆ alkyl optionally substituted with A₂, C₂₋₆ alkenyl optionally substituted with A₂, C₂₋₆ alkynyl optionally substituted with A₂, C₁₋₆ alkoxy optionally substituted with A₂, C₂₋₆ alkenyloxy optionally substituted with A₂, C₂₋₆ alkynyloxy optionally substituted with A₂, C₁₋₆ alkylcarbonyl, phenylcarbonyl optionally substituted with A₄, C₁₋₆ alkylsulfonyl, phenylsulfonyl optionally substituted with A₄, mono- or di-C₁₋₆ alkylamino optionally substituted with A₂, C₃₋₇ cycloalkyl optionally substituted with A₃, C₅₋₇ cycloalkenyl optionally substituted with A₃, phenyl optionally substituted with A₄, phenoxy optionally substituted with A₄, anilino optionally substituted with A₄, or a saturated or unsaturated 5- or 6-membered heterocyclic group having at least a hetero atom selected from oxygen, sulfur and nitrogen and optionally substituted with A₃;
Z represents oxygen or sulfur; m is 0, 1 or 2;
A₁ represents halogen, nitro, cyano, C₁₋₆ alkyl, C₂₋₆ alkenyl, C₁₋₆ haloalkyl, C₃₋₇ cycloalkyl, phenyl optionally substituted with halogen, C₁₋₆ alkyl or C₁₋₆ haloalkyl; pyridyl, thienyl, C₁₋₆ alkoxy, methylenedioxy, C₁₋₆ alkylthio, C₁₋₆ alkylsulfenyl, C₁₋₆ alkylsulfonyl, C₁₋₆ haloalkoxy, optionally substituted benzyl, optionally substituted phenethyl, optionally substituted phenoxy, optionally substituted phenylthio, optionally substituted benzoyl, C₁₋₆ alkylcarbonyl, C₁₋₆ alkoxycarbonyl, C₁₋₆ haloalkylcarbonyl, C₁₋₆ haloalkoxycarbonyl or C₁₋₆ alkylcarbonyloxy;
A₂ represents halogen, cyano, phenyl optionally substituted with A₄, a saturated or unsaturated 5- or 6-membered heterocyclic group having at least a hetero atom selected from oxygen, sulfur and nitrogen and optionally substituted with A₃, amino substituted with one or two groups of 'C₁₋₆ alkyl, C₃₋₇ cycloalkyl or C₁₋₆ alkylcarbonyl', C₁₋₆ alkylcarbonyl, C₁₋₆ alkoxycarbonyl, or one of the groups represented by the following formulae
OR₁₄, S(O)mR₁₅
(wherein, R₁₄ represents hydrogen, C₁₋₆ alkyl, C₂₋₆ alkenyl, C₂₋₆ alkynyl, C₁₋₆ haloalkyl, C₁₋₆ alkylcarbonyl, mono- or di-C₁₋₆ alkylcarbamoyl, C₃₋₇ cycloalkyl, phenyl optionally substituted with A₄, or a saturated or unsaturated 5- or 6-membered heterocyclic group having at least a hetero atom selected from oxygen, sulfur and nitrogen and optionally substituted with A₃; and
R₁₅ represents as defined for R₇);
A₃ represents halogen, hydroxyl, oxo, C₁₋₆ alkyl, C₁₋₆ alkoxy, C₁₋₆ alkoxy C₁₋₆ alkoxy, amino, mono- or di-C₁₋₆ alkylamino, C₁₋₆ alkylcarbonyloxy, C₁₋₆ alkoxycarbonyl, mono- or di-C₁₋₆ alkylcarbamoyl, or mono- or di-C₁₋₆ alkylcarbonylamino;
A₄ represents halogen, nitro, cyano, hydroxyl, C₁₋₆ alkyl, C₁₋₆ haloalkyl, C₁₋₆ alkoxy, C₁₋₆ alkylthio, C₁₋₆ alkylsulfenyl, C₁₋₆ alkylsulfonyl, C₁₋₆ alkoxy C₁₋₆ alkoxy, C₁₋₆ haloalkoxy, C₁₋₆ alkylcarbonyl, C₁₋₆ alkoxycarbonyl, or C₁₋₆ alkylcarbonyloxy;
If a group is substituted with two or more substituents, such as A₁, A₂, A₃ and A₄, the substituents may be the same or different;
R₃ represents not a group represented by Formula CZNR₁₀R₁₁, when X is oxygen or sulfur and R₂ is phenyl optionally substituted with A₁, naphthyl optionally substituted with A₁, indanyl optionally substituted with A₁, tetrahydronaphthyl optionally substituted with A₁, or thienyl optionally substituted with A₁; and
R₃ represents not alkylcarbonyl or alkenylcarbonyl, when X is NR₄], or a pharmaceutically acceptable composite thereof.

2. A compound represented by Formula [I-1] 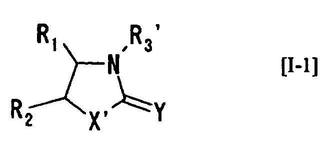 (wherein, X' represents oxygen or sulfur;
R₁, R₂ and Y represent as defined above;
R₃' represents C₁₋₆ alkyl substituted with A₂, C₂₋₆ alkenyl substituted with A₂, or one of the groups represented by the following formulae
CZR₆', S(O)mR₇, 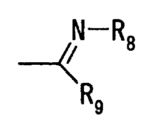
R₆' represents C₁₋₆ alkyl optionally substituted with A₂, C₁₋₆ alkoxy optionally substituted with A₂, C₂₋₆ alkenyl optionally substituted with A₂, C₃₋₇ cycloalkyl optionally substituted with A₃, C₃₋₇ cycloalkoxy optionally substituted with A₃, C₃₋₇ cycloalkenyl optionally substituted with A₃, C₃₋₇ cycloalkenyloxy optionally substituted with A₃, phenyl optionally substituted with A₄, or a saturated or unsaturated 5- or 6-membered heterocyclic group having at least a hetero atom selected from oxygen, sulfur and nitrogen and optionally substituted with A₃; and R₇, R₈ and R₉ are as defined above], or a pharmaceutically acceptable composite thereof.

3. A compound represented by Formula [I-2] 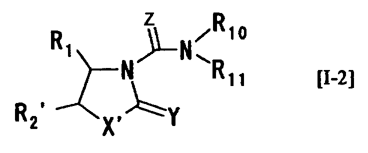 (wherein, X' represents oxygen or sulfur;
R₁, Y, Z, R₁₀ and R₁₁ represent as defined above; and
R₂' represents phenyl C₁₋₆ alkyl optionally substituted with A₁, phenyl C₂₋₆ alkenyl optionally substituted with A₁, thienyl C₂₋₆ alkenyl optionally substituted with A₁ or naphthyl C₂₋₆ alkenyl optionally substituted with A₁), or a pharmaceutically acceptable composite thereof.

4. A compound represented by Formula [I-3] 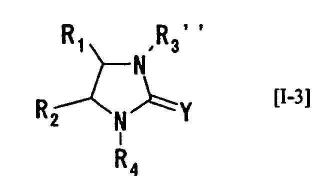 (wherein, R₁, R₂, R₄ and Y represent as defined above;
R₃'' is C₁₋₆ alkyl optionally substituted with A₂, C₂₋₆ alkenyl optionally substituted with A₂, or one of the groups represented by the following formulae
CZR₆'', S(O)mR₇, 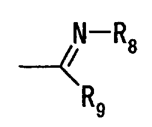
R₆'' represents C₁₋₆ alkyl substituted with A₂, C₁₋₆ alkoxy optionally substituted with A₂, C₂₋₆ alkenyl substituted with A₂, C₃₋₇ cycloalkyl optionally substituted with A₃, C₃₋₇ cycloalkoxy optionally substituted with A₃, C₃₋₇ cycloalkenyl optionally substituted with A₃, C₃₋₇ cycloalkenyloxy optionally substituted with A₃, phenyl optionally substituted with A₄, a saturated or unsaturated 5- or 6-membered heterocyclic group having at least a hetero atom selected from oxygen, sulfur and nitrogen and optionally substituted with A₃; or a group represented by Formula NR₁₀R₁₁; and
R₇, R₈, R₉, R₁₀, R₁₁, A₂, A₃ and A₄ represent as defined above), or a pharmaceutically acceptable composite thereof.

5. A compound represented by Formula [I-4] 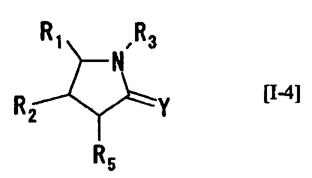 (wherein, R₁, R₂, R₃, R₅ and Y are as defined above), or a pharmaceutically acceptable composite thereof.

6. A medicinal composition **characterized by** containing at least a component selected from the group consisting of the heterocyclic compounds of Formula [I] or pharmaceutically acceptable composites thereof, as an active ingredient.

7. A medicinal composition according to Claim 6 in which the composition is an inhibitor of phospholipase A (2) activity and contains at least a component selected from the group consisting of the heterocyclic compounds of Formula [I] or pharmaceutically acceptable composites thereof as an active ingredient.

8. A use of a medicinal composition **characterized by** containing at least a component selected from the group consisting of the heterocyclic compounds of Formula [I] or pharmaceutically acceptable composites thereof as an ingredient, for a mammal that needs a treatment of inflammatory disease or disorder.

9. A method for treating or relieving an inflammatory disease or disorder by means of controlling and/or preventing the progress of the symptoms of the disease, in which an effective dose of a composition containing at least a component selected from the group consisting of the heterocyclic compounds of Formula [I] or pharmaceutically acceptable composites thereof as an active ingredient is administered to a mammal that needs a treatment of an inflammatory disease or disorder.

10. A method according to Claim 9, in which the inflammatory disease or disorder is anaphylaxis, allergic inflammation, asthma, rhinitis, bronchitis, pneumonia, adult respiratory distress syndrome, inflammatory intestine disorder, Crohn's disease, ulcerative colitis, ischemia/reperfusion injuries, vasculitis, arteriosclerosis, hepatitis, nephritis, nerve degenerative diseases, arthritis, dermatitis, solar keratosis, psoriasis, septic shock or fever.

11. A method according to Claim 9, in which the inflammatory disease or disorder is that the symptom progresses with the enhanced phospholipase A (2) activity.

12. A method according to Claim 9, in which the inflammatory disease or disorder is mediated by arachidonic acid and its metabolites, lysophosphatidylcholine and/or the platelet activating factor (PAF), which are inflammatory lipid mediators.

13. A method according to Claim 9, in which the inflammatory lipid mediators are suppressed by inhibitors of phospholipase A (2) activity.

14. A use of the heterocyclic compounds of Formula [I] for producing drugs to use for easing inflammatory and allergic conditions and conditions associated with immunity, and/or treating such diseases

15. A use, as a medicine, of a composition containing at least a component selected from the group consisting of the heterocyclic compounds of Formula [I] or pharmaceutically acceptable composites thereof as an active ingredient.

16. A use of the composition according to Claim 15 in which the medicine is an anti-inflammatory drug, anti-allergic drug and/or immune controlling agent.
